# EUROPEAN PATENT APPLICATION

(11) **EP 4 148 042 A1**
(43) Date of publication of application: **15.03.2023**
(21) Application number: 21306242.5
(22) Date of filing: 10.09.2021
(51) Int. Cl.: C07C 327/42, C07C 335/16, C07D 207/20, C07D 207/22, A61P 31/12

(54) **PHENOXY-ACETYL-THIOUREIDO-BENZENESULFONAMIDE DERIVATIVES, AND THEIR USES**

(71) Applicant: Centre National de la Recherche Scientifique, 75016 Paris (FR); Université de Montpellier, 34090 Montpellier (FR)
(72) Inventor: GAUDIN, Raphaël, 34090 Montpellier (FR)
(74) Representative: LLR

(57) **Abstract**

The invention relates to a compound of formula I: 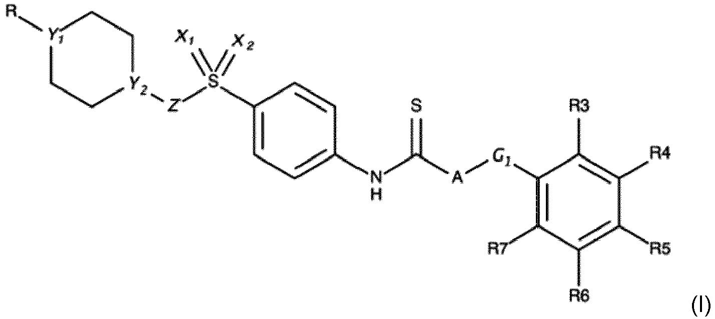 wherein
A is NH or CH2; G1 is a linear or a cyclic alkyl; Y1 is CH or N; Y2 is O, N or CH, W is O, N or CH; Z is NH or O; X1 and X2 and R, and R1 to R8 are functional groups.

## Description

The invention relates to phenoxy-acetyl-thioureido-benzenesulfonamide derivatives, and their use, in particular for treating viral infections.

The Coronaviridae family is subdivided into four genera based on phylogenetic clustering: Alpha-, Beta-, Gamma- and Delta-coronavirus. SARS-CoV, SARS-CoV-2 (CoV2), and MERS-CoV cluster into the beta genus, while the human Coronavirus 229E (HCoV-229E) and NL63, and the feline coronavirus (FCoV) are from the alpha genus. The main outcome of CoV-2 infection is acute respiratory distress syndrome (85%), but other clinical disorders exist, including acute cardiac and kidney injuries (31% and 23% respectively), secondary infection (31%), and shock (23%).

CoV-2 is an enveloped positive-strand RNA virus exhibiting a ≈ 30 kb genome enwrapped into a particle composed of a cellular lipid bilayer and four structural proteins: Spike (S), Nucleocapsid (N), Envelope (E), and Membrane (M).

CoV2 entry is highly dependent on the angiotensin-converting enzyme 2 (ACE2), similarly to its close relative SARS-CoV. However, CoV2 contains a furin cleavage site between S1/S2, allowing S to be processed before egress that is absent from SARS-CoV, which explains its partial dependency for TMPRSS2. It is likely that CoV-2 Spike can be cleaved and activated directly at the cell surface, although it is still possible that the virus is internalized into endosomes before fusion. The virus is internalized in a clathrin-dependent manner and endosomal acidification is required if the target cells do not express TMPRSS2. Upon fusion with cellular membranes, the RNA genome of CoV-2, protected by N protein, is targeted to ER membranes where translation immediately of replication proteins occurs, initiating the formation of viral replication organelles consisting of characteristic perinuclear double-membrane vesicles (DMVs).

Since the beginning of the COVID-19 pandemic, thousands of clinical trials of molecules and experimental therapies have been started worldwide. As of today, several vaccines based on the induction of anti-Spike antibody generation have been approved, giving some hope to shut-down this pandemic. However, preparedness is key, and because the inventor is observing the emergence of variants that escape vaccination, complementary tools, such as antiviral development, remain highly needed.

Drug repurposing has been started at the onset of the pandemic, but this approach has not been successful to date. One strong candidate for antiviral COVID-19 treatment is Remdesivir, a nucleoside analog inhibiting SARS-CoV-2 *in vitro* and in preliminary clinical reports. However, it does not improve the condition of severe COVID-19 patients, exhibits notable side-effects, and is not accessible to non-hospitalized patients because of the intravenous route of administration. Although further clinical investigations are still needed, one can anticipate that a direct-acting agent (DAA) targeting the viral polymerase, may not be sufficient to fight the disease on in the long run, as RNA viruses tend to rapidly evolve powerful escape strategies through punctual mutations. As such, host-targeting agents (HTA) should also be developed.

Specifically, the need for novel antiviral strategies is still highly required because 1) drug repurposing may fail or need chemical optimization to increase efficiency against SARS-CoV-2, 2) side effects may be too important to treat vulnerable populations, 3) viral mutation/adaptation could render potent antivirals less efficient, and 4) multitherapy is an interesting strategy to prevent/limit viral escape.

The aim of the invention is to obviate the drawbacks of the prior art, and to propose a specific and efficient medicament for curing SARS-CoV-2 infection, or preventing its side effects.

The invention relates to a compound of formula I: 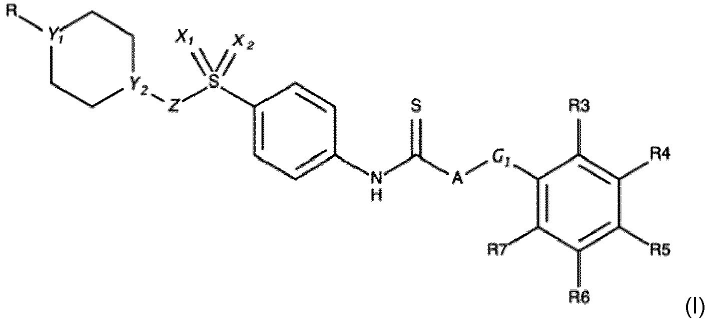 wherein
- R is H or a C1-C4 alkyl;
- A is NH or CH₂;
- Y₁ is CH or N;
- Y₂ is O, N or CH;
- Z is NH or O;
- G₁ is one of the following: 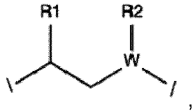 wherein W is CH, N or O, and wherein R1 and R2, are independently from each other, H, a C1-C2 alkyl, linear or branched, saturated of not, and wherein R2 is missing when W is O; 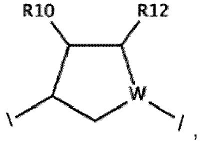 wherein W is C or N, R10 is a group chosen from an hydroxyl, an methoxy, and ethoxy, a C1-C2 alkyl, an halogen or a -CF₃ group, and R12 is H or =O or OH; 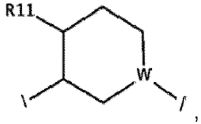 wherein W is C or N, and R11 is an amine or a C1-C2 alkyl,
- X₁ and X₂ are, independently from each other, NH, N-R9, wherein R9 is a C1-C3 alkyl, linear or branched, a -CN group or a -CF3 group;
- R3 to R7 are, independently from each other, H or a functional group from the list consisting of fluoro, chloro, bromo, nitro, cyano, amino, amino alkyl such as amino methyl and amino ethyl, methyl, hydroxylmethyl, trifluoromethyl, methoxy, trifluoromethyloxy, ethyl, trifluoroethyl, ethoxy and trifluoroethyloxy;
or a salt or a solvate thereof.

Advantageously, the invention relates to the above defined compounds, the compound having one of the following formulas: 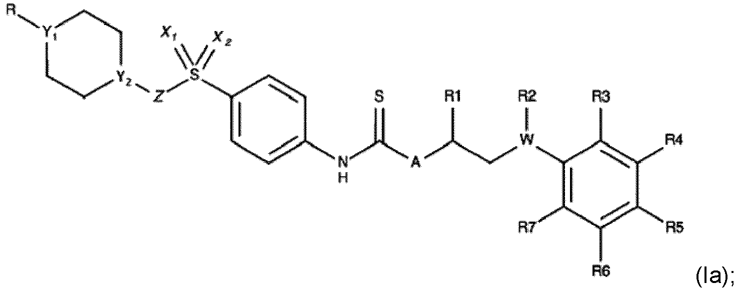 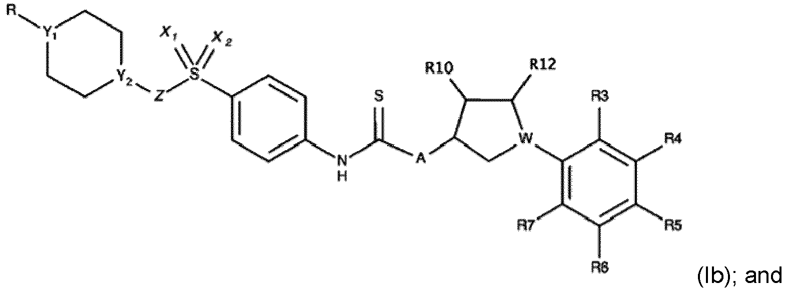 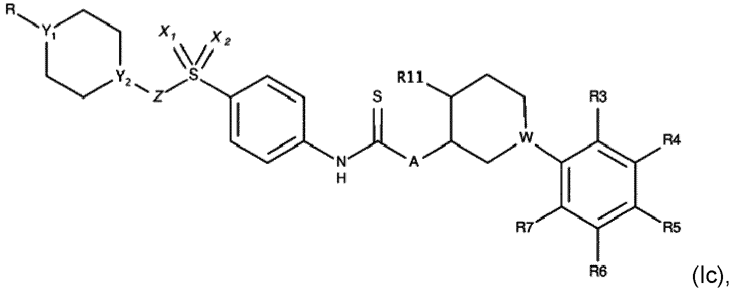 wherein A, Y1, Y2, W, Z, X1 and X2 and R and R1 to R7 are as defined above.

The inventor unexpectedly identified that specific phenoxy-acetyl-thioureido-benzenesulfonamide derivatives, are potent and efficient antiviral compounds, but are also efficient against coronaviruses, in particular against SARS-CoV-2 virus.

All the compounds covered by formula I are novel, and R groups may increase either stability or solubility in an aqueous solvent, or both.

Advantageously, the invention relates to the compound as defined above, having one of the following formulas: 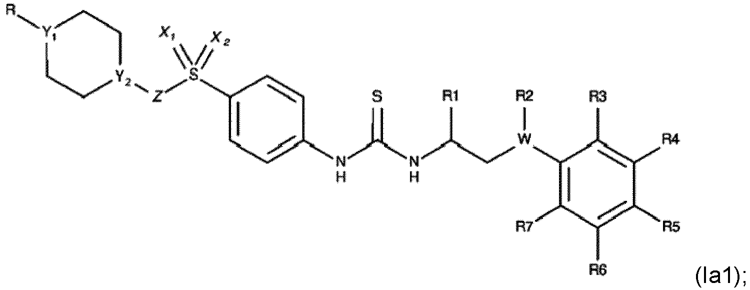 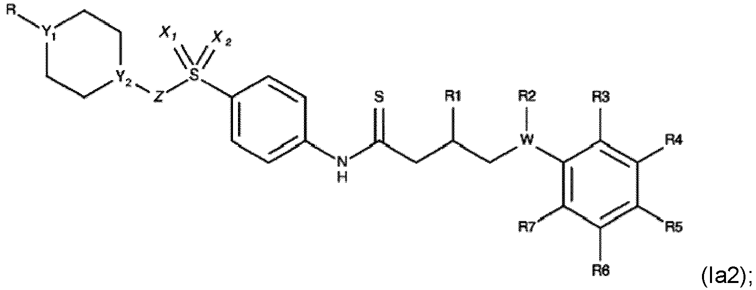 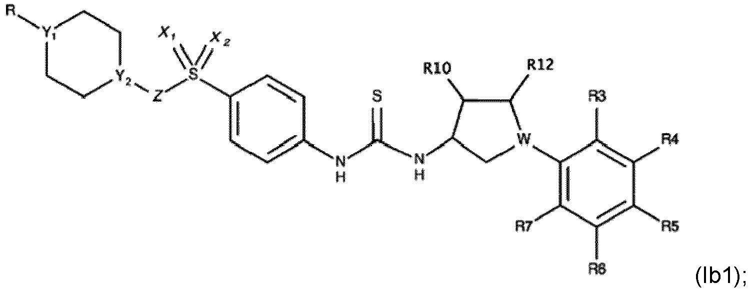 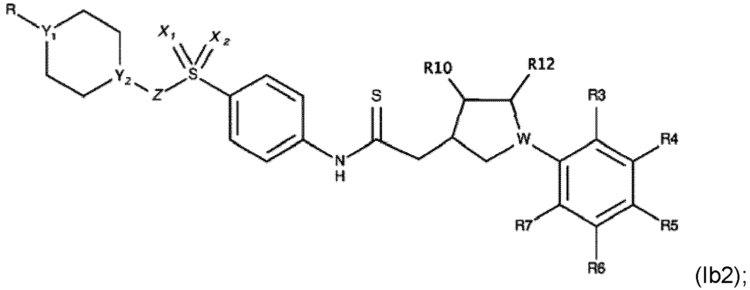 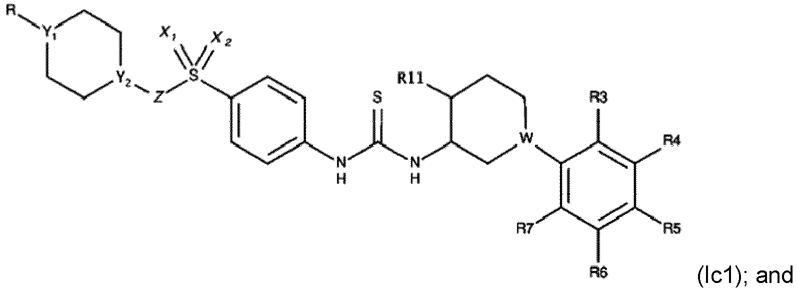 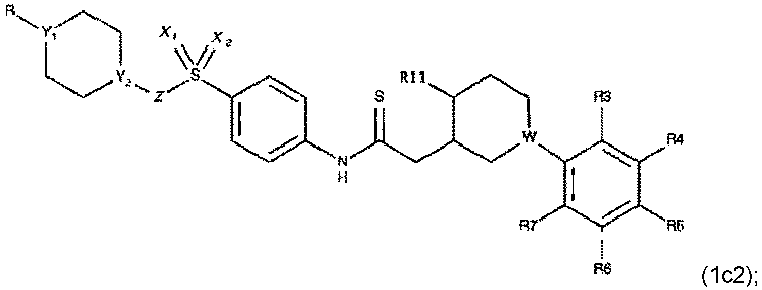 wherein Y1, Y2, W, Z, X1, X2, R and R1 to R7 are as defined above.

Advantageously, the invention relates to the compound as defined above, having one of the following formulas: 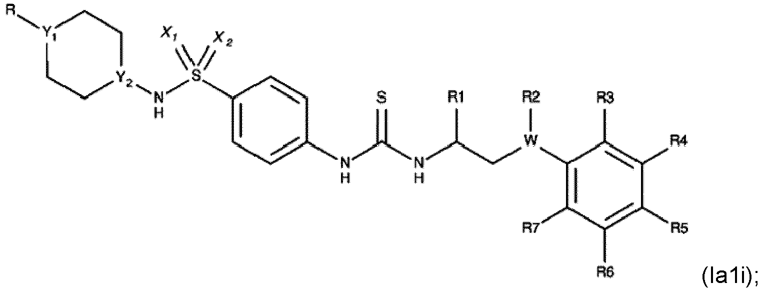 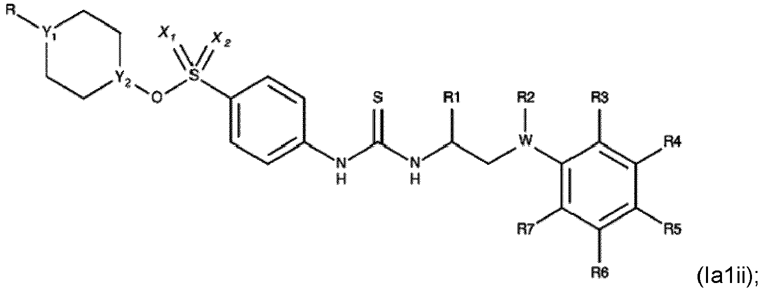 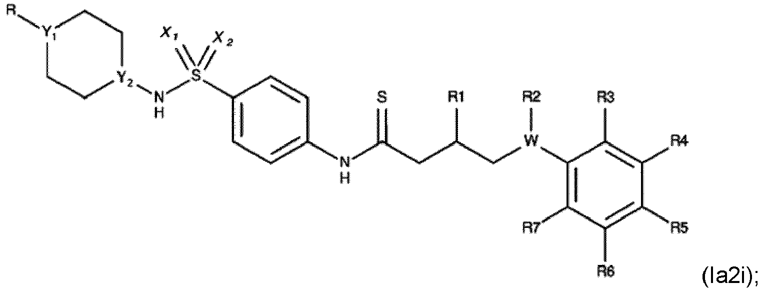 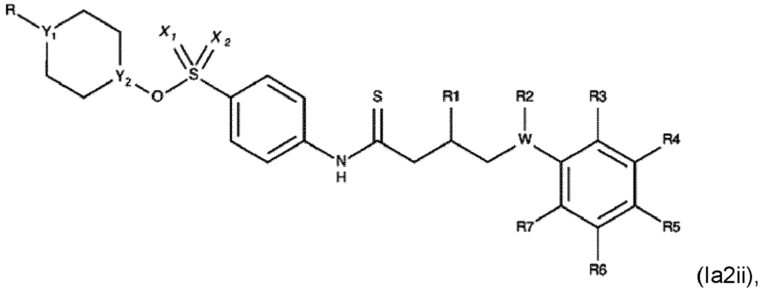 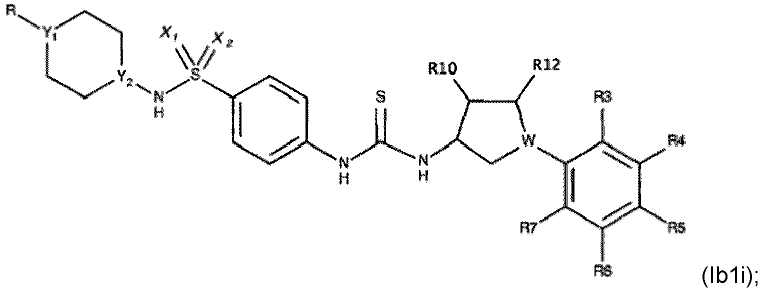 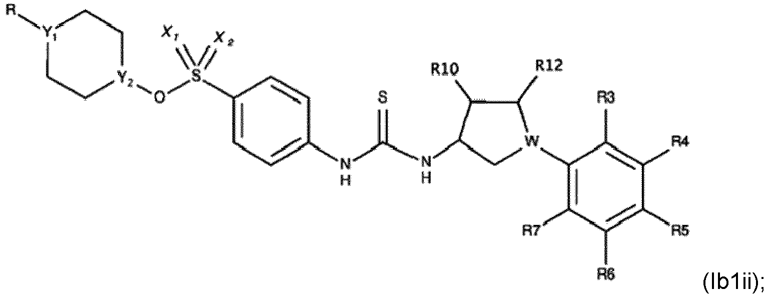 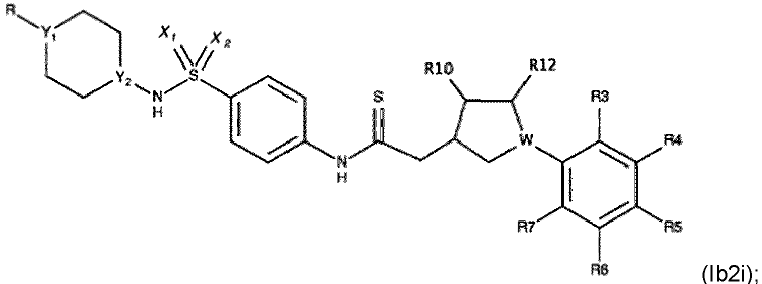 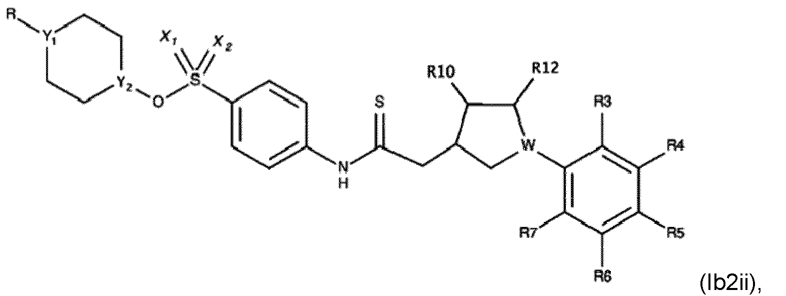 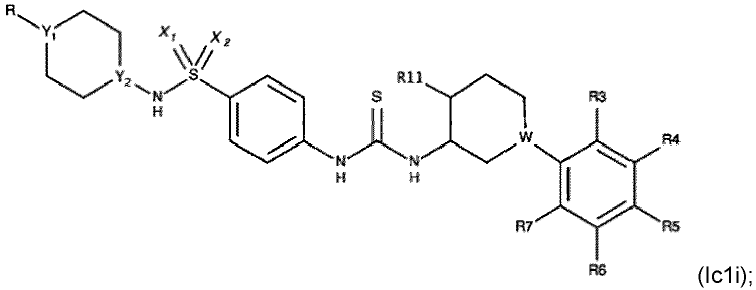 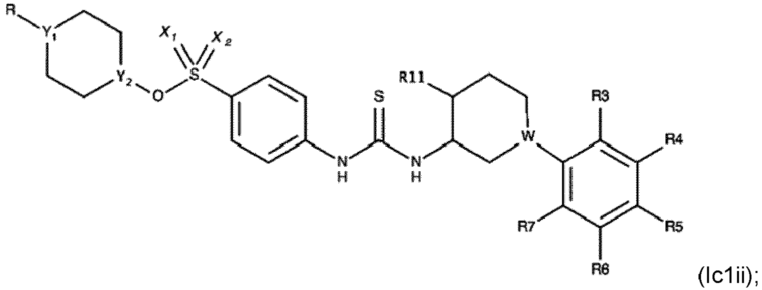 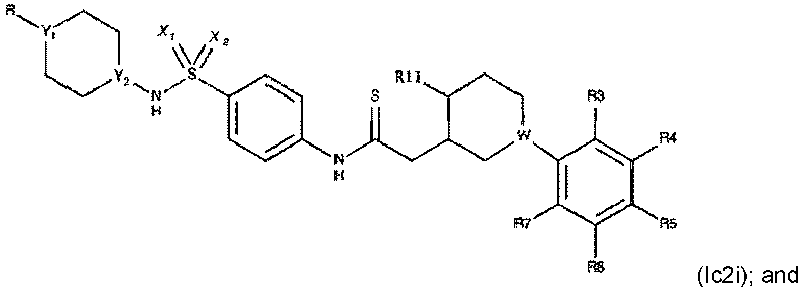 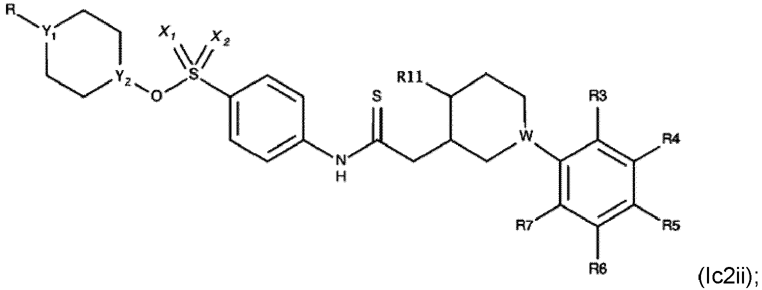 wherein Y1, Y2, W, X1, X2, R and R1 to R7 are as defined above.

More advantageously, the invention relates to the compound as defined above, having one of the following formulas: 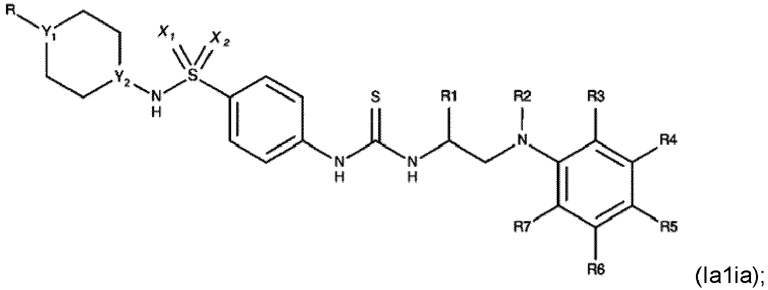 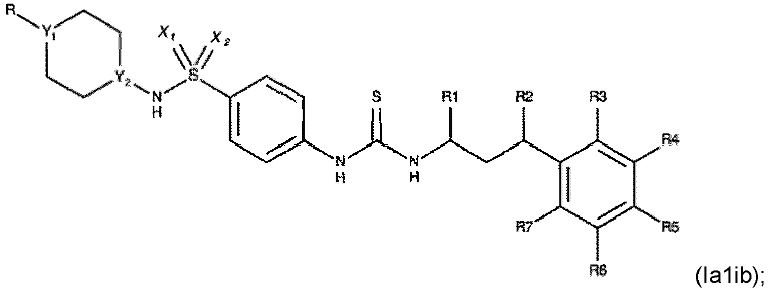 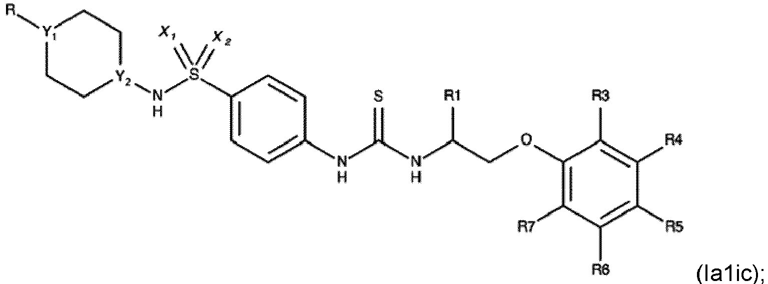 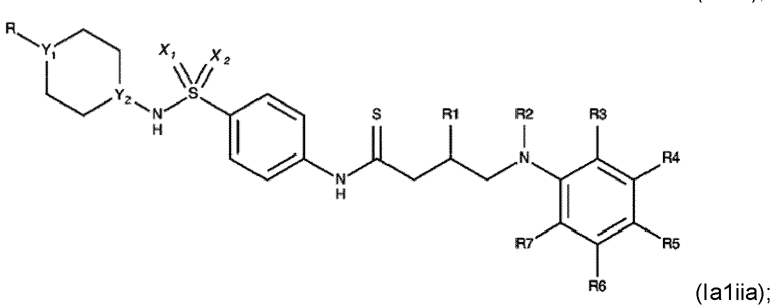 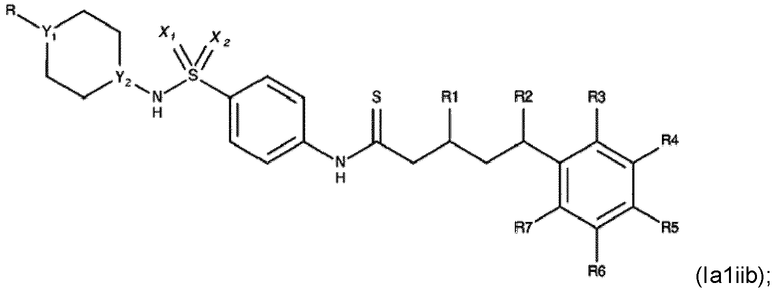 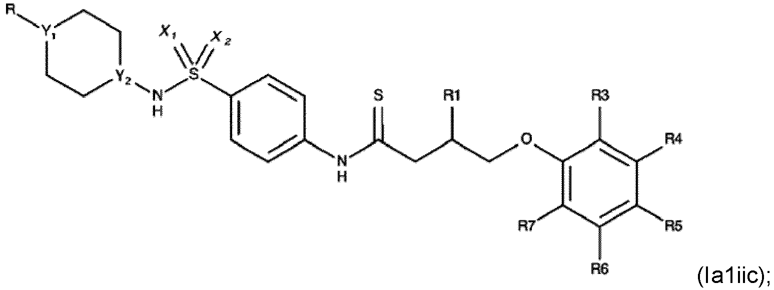 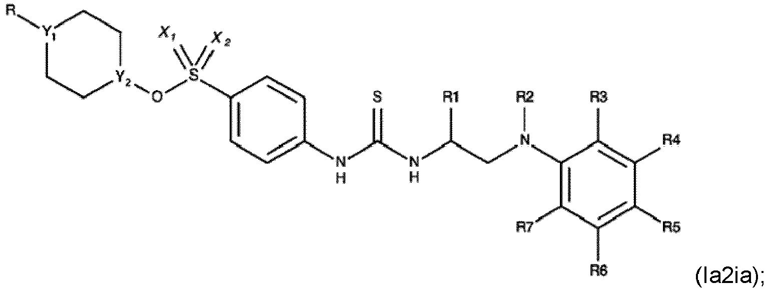 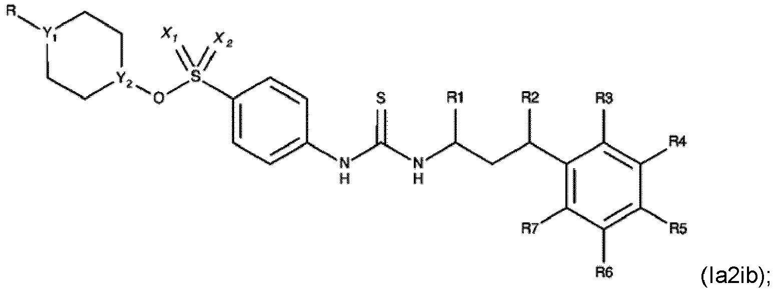 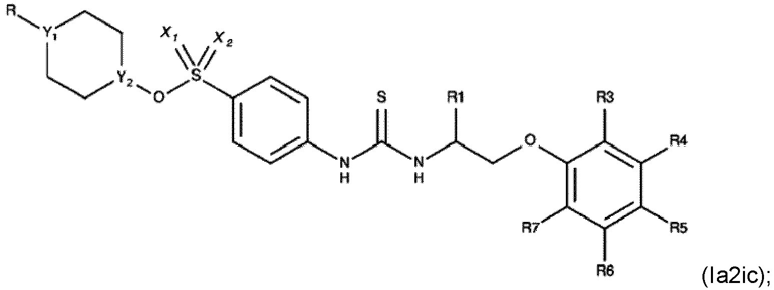 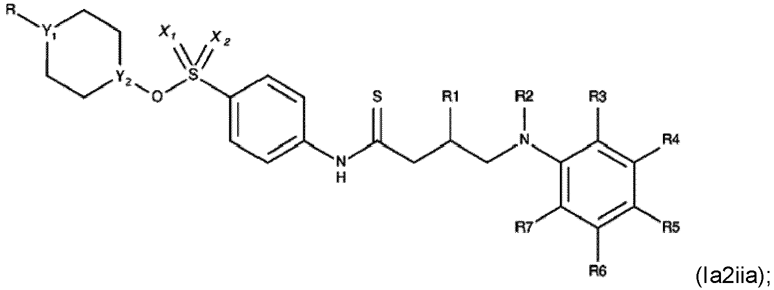 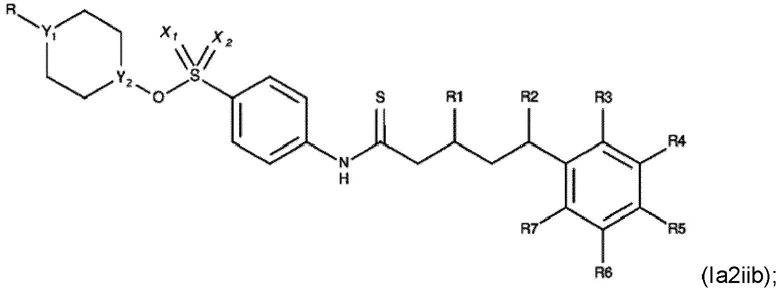 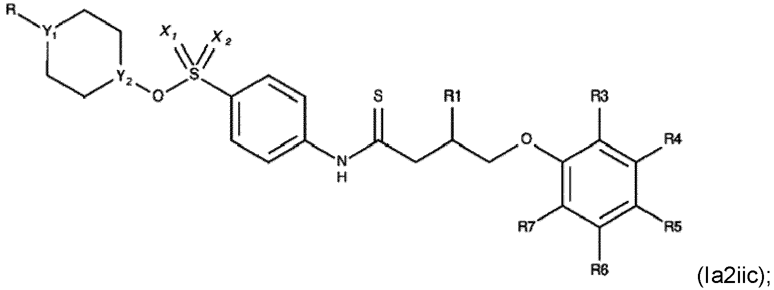 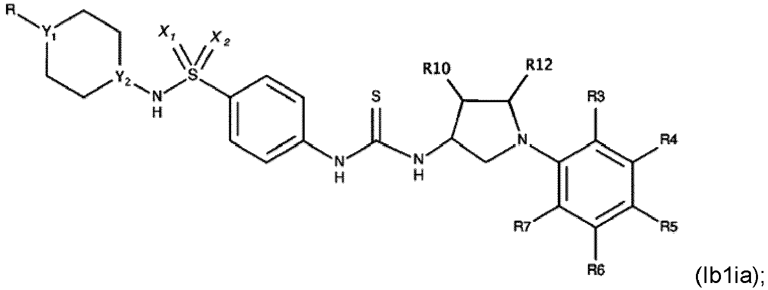 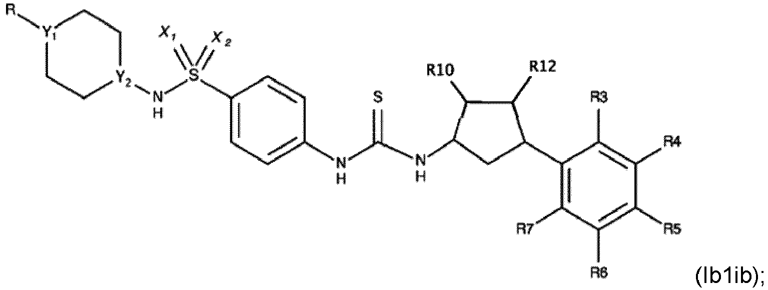 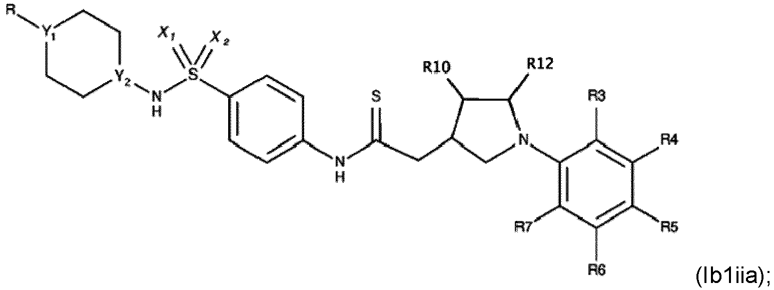 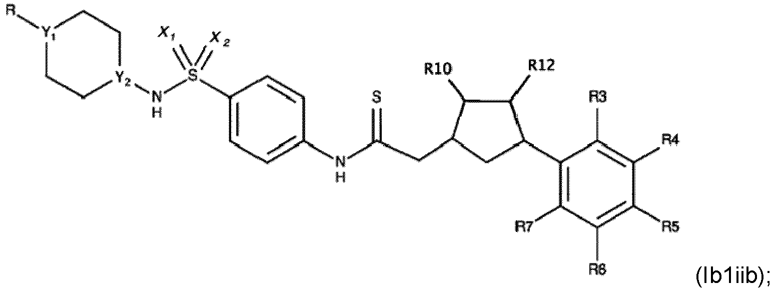 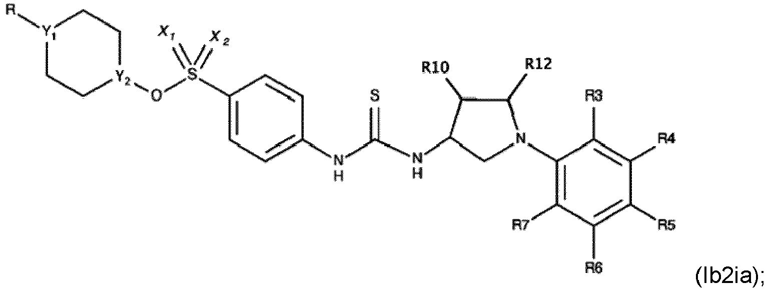 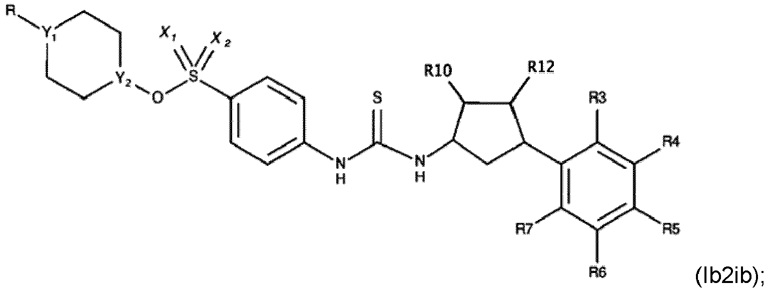 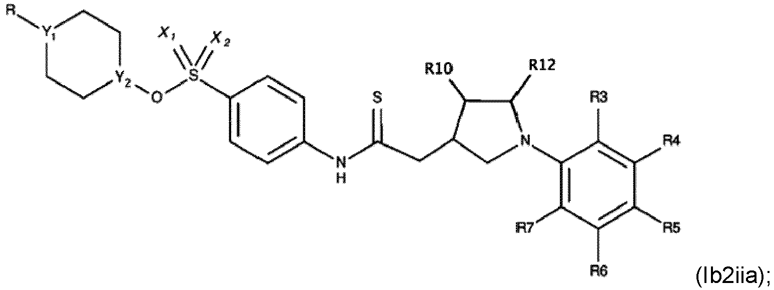 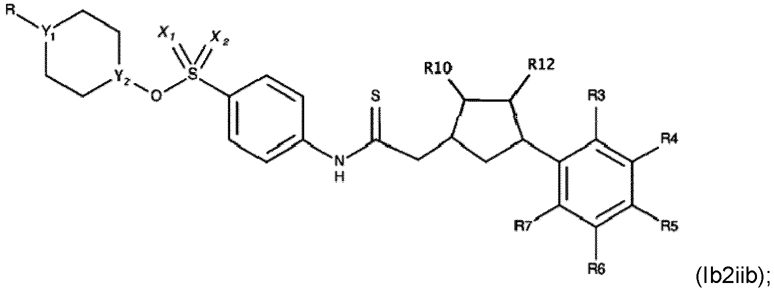 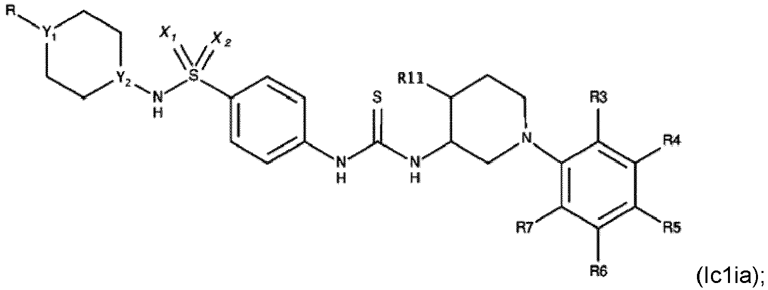 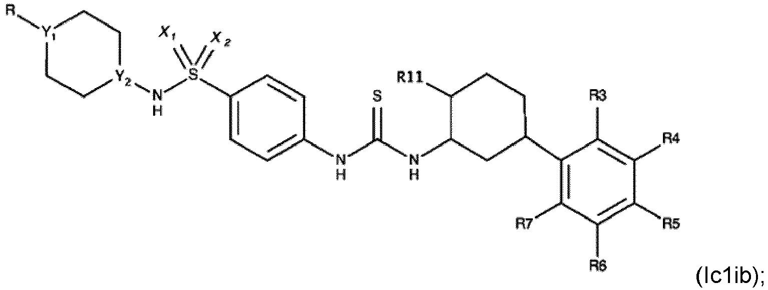 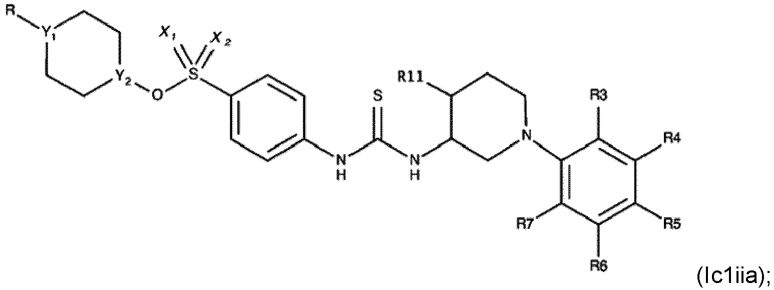 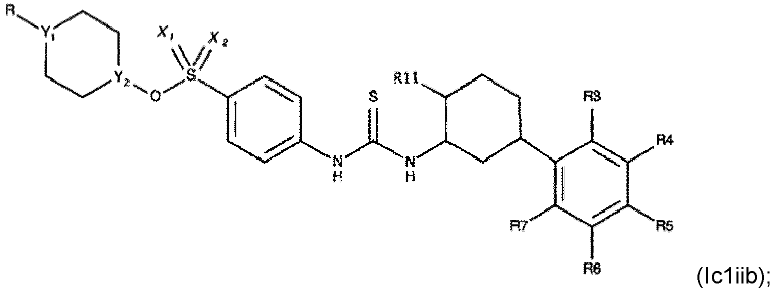 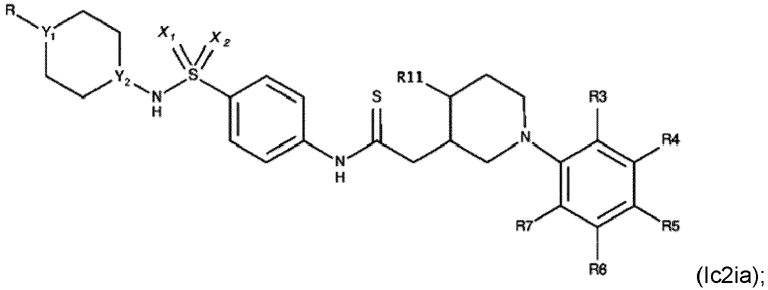 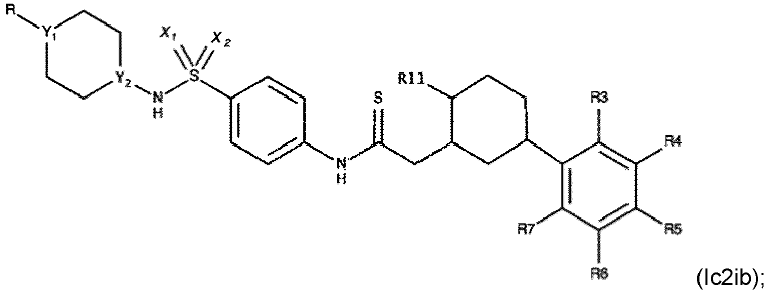 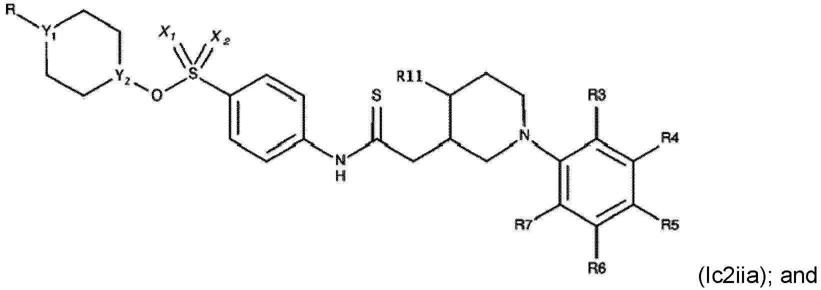 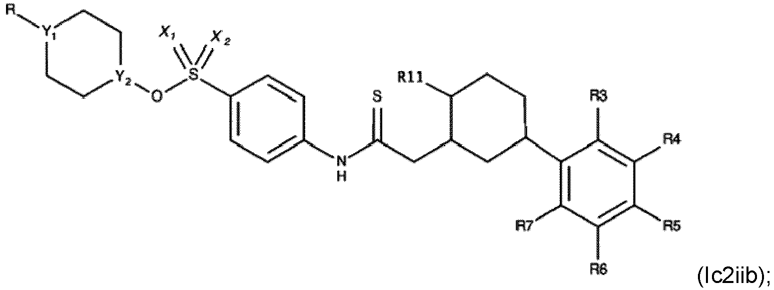 wherein Y1, Y2, X1 and X2, R and R1 to R7 are as defined above.

More advantageously, the invention relates to the compound as defined above, wherein X1 and X2 are O.

More advantageously, the invention relates to the compound as defined above, wherein R3, R4, R6 and R7 are H.

More advantageously, the invention relates to the compound as defined above, wherein R5 is -O-CH₃.

Advantageously, the invention relates to the compound as defined above, the compound having one of the following formulas:

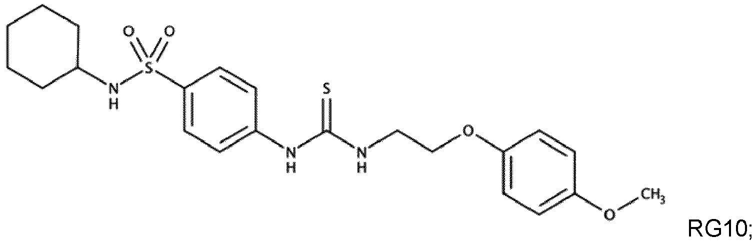

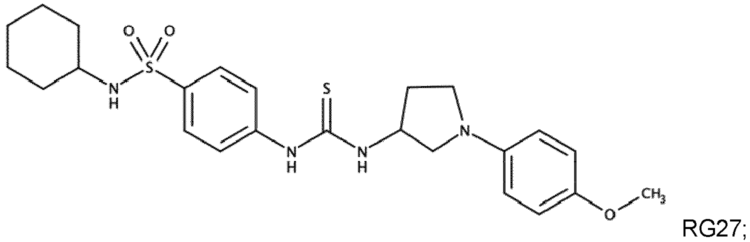

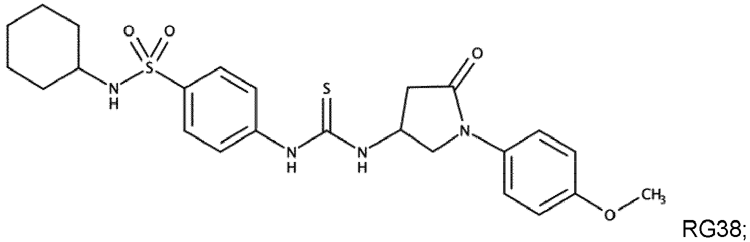

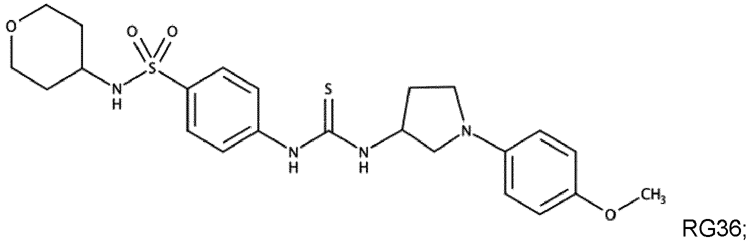

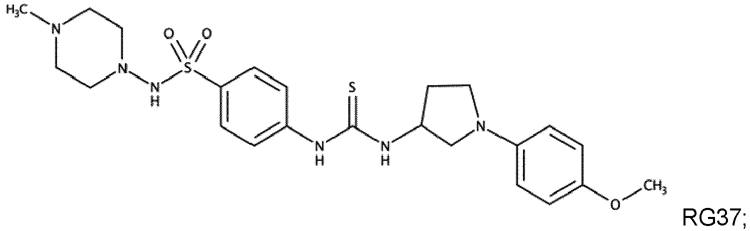

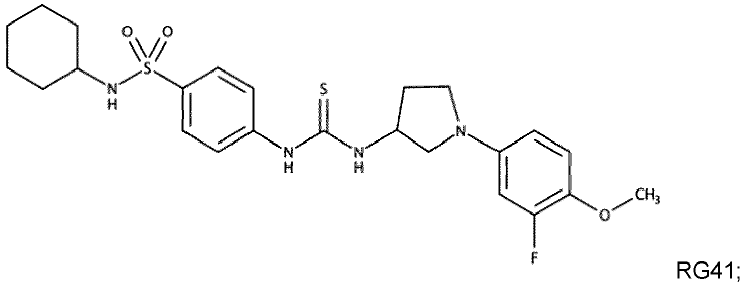

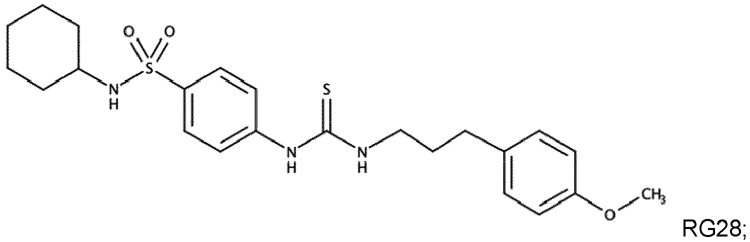

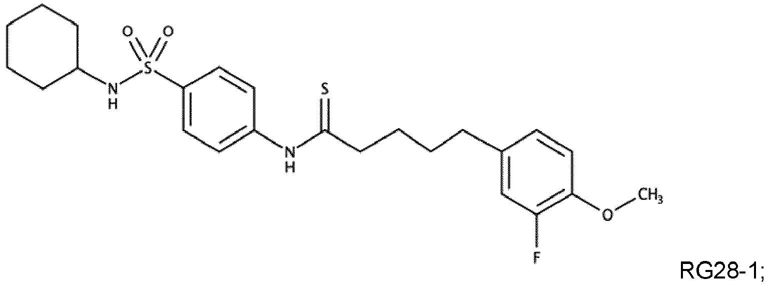

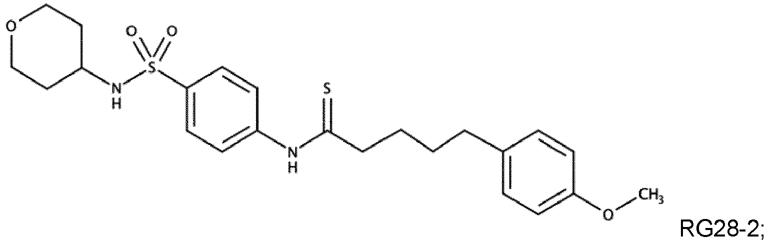

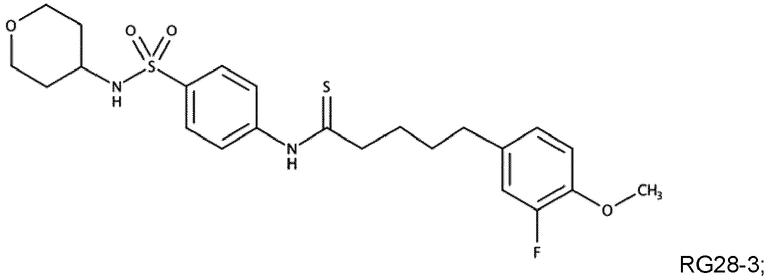

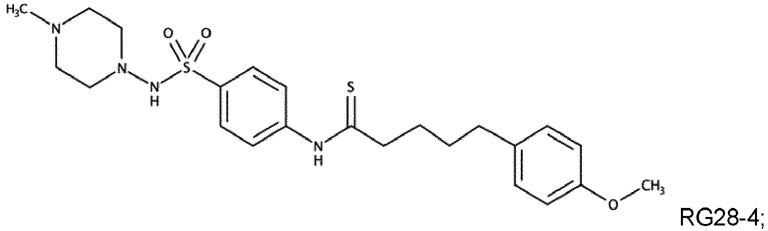

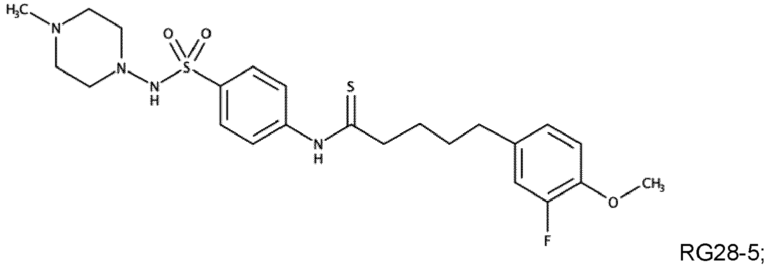

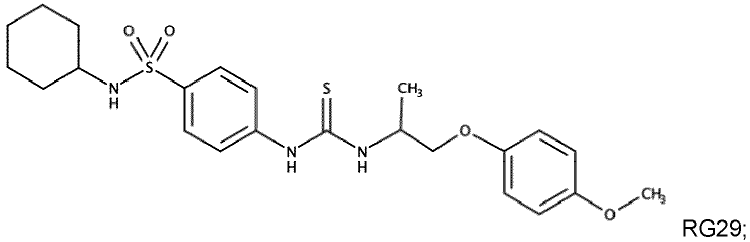

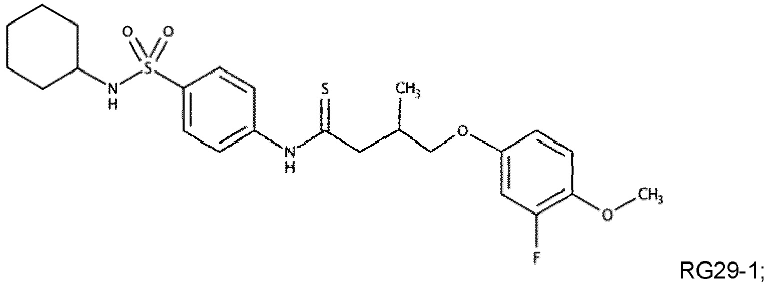

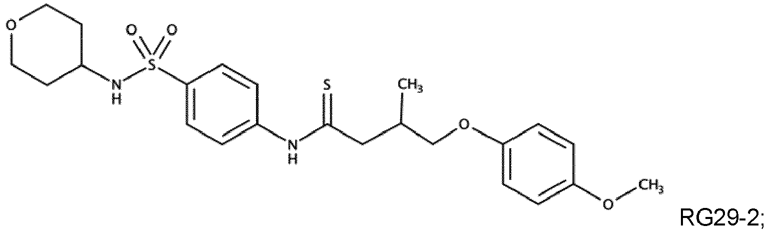

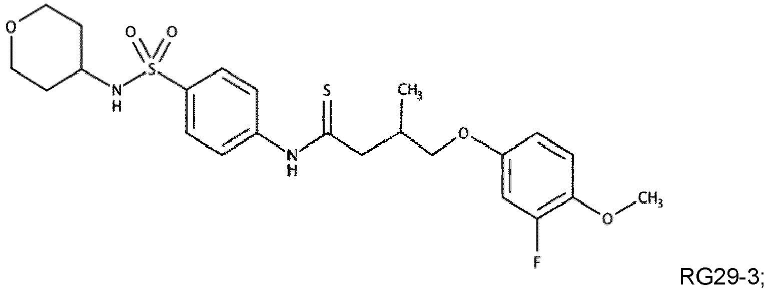

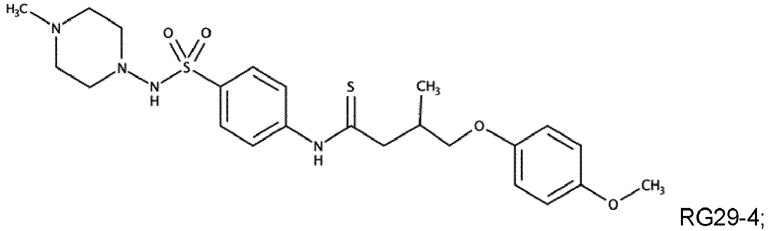

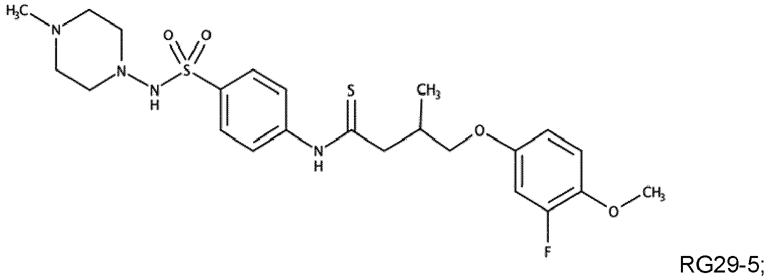

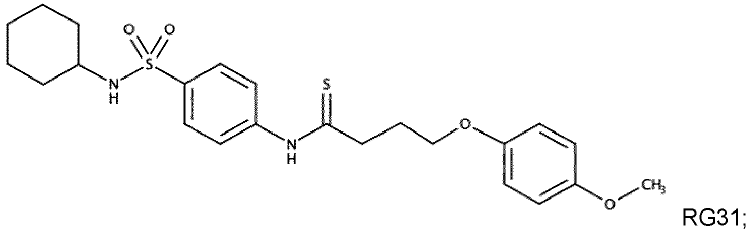

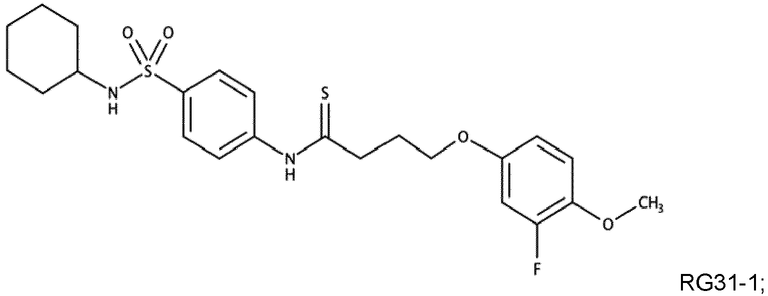

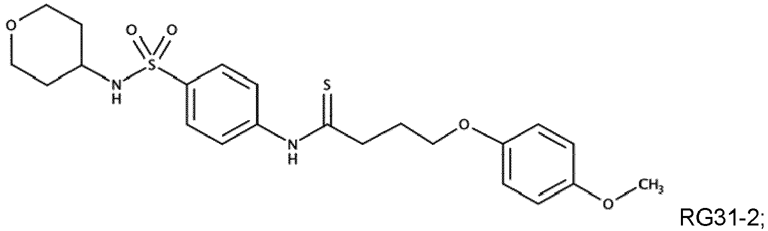

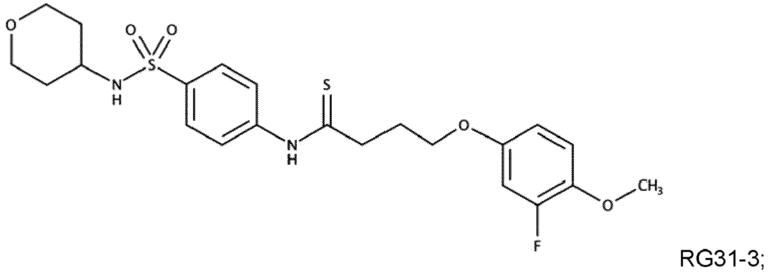

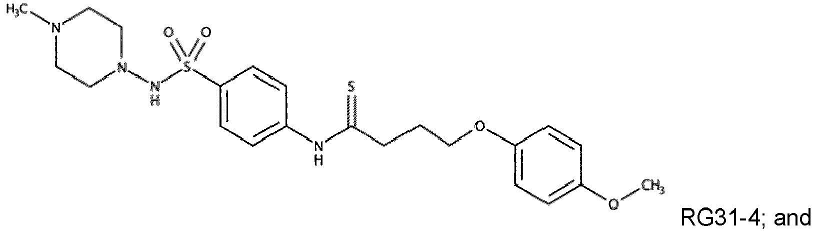

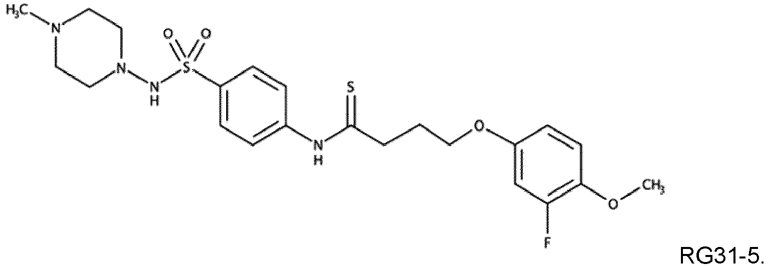

The invention also relates to a pharmaceutical composition comprising, as active ingredient, the compound as defined above, in association with a pharmaceutically acceptable carrier.

Compositions within the scope of the invention include all compositions wherein the compounds of the present invention are contained in an amount which is effective to achieve its intended purpose. While individual needs vary, determination of optimal ranges of effective amounts of each component is within the skill of the art. Typically, the compounds may be administered to mammals, e.g. humans, orally at a dose of 0.0025 to 50 mg/kg, or an equivalent amount of the pharmaceutically acceptable salt thereof, per day of the body weight of the mammal being treated for disorders responsive to induction of apoptosis. In one embodiment, about 0.01 to about 25 mg/kg is orally administered to treat, ameliorate, or prevent such disorders. For intramuscular injection, the dose is generally about one-half of the oral dose. For example, a suitable intramuscular dose would be about 0.0025 to about 25 mg/kg, or from about 0.01 to about 5 mg/kg.

The unit oral dose may comprise from about 0.01 to about 1000 mg, for example, about 0.1 to about 100 mg of the compound. The unit dose may be administered one or more times daily as one or more tablets or capsules each containing from about 0.1 to about 10 mg, conveniently about 0.25 to 50 mg of the compound or its solvates.

In a topical formulation, the compound may be present at a concentration of about 0.01 to 100 mg per gram of carrier. In a one embodiment, the compound is present at a concentration of about 0.07-1.0 mg/ml, for example, about 0.1-0.5 mg/ml, and in one embodiment, about 0.4 mg/ml.

In addition to administering the compound as a raw chemical, the compounds of the invention may be administered as part of a pharmaceutical preparation containing suitable pharmaceutically acceptable carriers comprising excipients and auxiliaries which facilitate processing of the compounds into preparations which can be used pharmaceutically. The preparations, particularly those preparations which can be administered orally or topically and which can be used for one type of administration, such as tablets, dragees, slow release lozenges and capsules, mouth rinses and mouth washes, gels, liquid suspensions, hair rinses, hair gels, shampoos and also preparations which can be administered rectally, such as suppositories, as well as suitable solutions for administration by intravenous infusion, injection, topically or orally, contain from about 0.01 to 99 percent, in one embodiment from about 0.25 to 75 percent of active compound(s), together with the excipient.

The pharmaceutical compositions of the invention may be administered to any patient which may experience the beneficial effects of the compounds of the invention. Foremost among such patients are mammals, e.g., humans, although the invention is not intended to be so limited. Other patients include veterinary animals (cows, sheep, pigs, horses, dogs, cats and the like).

The compounds and pharmaceutical compositions thereof may be administered by any means that achieve their intended purpose. For example, administration may be by parenteral, subcutaneous, intravenous, intramuscular, intraperitoneal, transdermal, buccal, intrathecal, intracranial, intranasal or topical routes. Alternatively, or concurrently, administration may be by the oral route. The dosage administered will be dependent upon the age, health, and weight of the recipient, kind of concurrent treatment, if any, frequency of treatment, and the nature of the effect desired.

The pharmaceutical preparations of the present invention are manufactured in a manner which is itself known, for example, by means of conventional mixing, granulating, dragee making, dissolving, or lyophilizing processes. Thus, pharmaceutical preparations for oral use can be obtained by combining the active compounds with solid excipients, optionally grinding the resulting mixture and processing the mixture of granules, after adding suitable auxiliaries, if desired or necessary, to obtain tablets or dragee cores.

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone. If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries are, above all, flow-regulating agents and lubricants, for example, silica, talc, stearic acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethyl-cellulose phthalate, are used. Dye stuffs or pigments may be added to the tablets or dragee coatings, for example, for identification or in order to characterize combinations of active compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are in one embodiment dissolved or suspended in suitable liquids, such as fatty oils, or liquid paraffin. In addition, stabilizers may be added.

Possible pharmaceutical preparations which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400. Aqueous injection suspensions may contain substances which increase the viscosity of the suspension include, for example, sodium carboxymethyl cellulose, sorbitol, and/or dextran. Optionally, the suspension may also contain stabilizers.

The topical compositions of this invention are formulated in one embodiment as oils, creams, lotions, ointments and the like by choice of appropriate carriers. Suitable carriers include vegetable or mineral oils, white petrolatum (white soft paraffin), branched chain fats or oils, animal fats and high molecular weight alcohol (greater than C12). The carriers may be those in which the active ingredient is soluble. Emulsifiers, stabilizers, humectants and antioxidants may also be included as well as agents imparting color or fragrance, if desired. Additionally, transdermal penetration enhancers can be employed in these topical formulations. Examples of such enhancers can be found in U.S. Pat. Nos. 3,989,816 and 4,444,762.

Ointments may be formulated by mixing a solution of the active ingredient in a vegetable oil such as almond oil with warm soft paraffin and allowing the mixture to cool. A typical example of such an ointment is one which includes about 30% almond oil and about 70% white soft paraffin by weight. Lotions may be conveniently prepared by dissolving the active ingredient, in a suitable high molecular weight alcohol such as propylene glycol or polyethylene glycol.

The skilled person will readily recognize that the foregoing represents merely a detailed description of certain preferred embodiments of the present invention. Various modifications and alterations of the compositions and methods described above can readily be achieved using expertise available in the art and are within the scope of the invention.

The invention also relates to a compound as defined above, for its use as a medicament, medicine or drug

The invention also relates to a compound as defined above, for its use for the treatment of viral infections or a pathology linked to the viral infection.

Advantageously, the invention relates to the above compounds, wherein the viral infection is a coronavirus infection.

Advantageously, the invention relates to the above compounds, for its use as defined above, wherein the viral infection is a Sars-CoV-2 infection, and the pathology liked to the viral infection is COVID-19.

Severe acute respiratory syndrome coronavirus 2 (SARS-CoV-2) is an enveloped, positive-sense, single-stranded RNA beta-coronavirus that emerged in Wuhan in November 2019 and rapidly developed into a global pandemic. The associated disease, COVID-19, has an array of symptoms, ranging from flu-like illness and gastrointestinal distress to acute respiratory distress syndrome, heart arrhythmias, strokes, and death. Drug repurposing has played an important role in the search for COVID-19 therapies. Recently, the FDA issued emergency approval of remdesivir (GS-5734), a monophosphoramidate prodrug of a nucleoside inhibitor developed for Ebola virus treatment, and hydroxychloroquine, an aminoquinoline derivative first developed in the 1940s for the treatment of malaria, for severely ill patients with COVID-19. However, there are no established prophylactic strategies or direct antiviral treatments available to limit SARS-CoV-2 infections and to prevent/cure the associated disease COVID-19.

The invention also relates to the use of the above defined compound for the preparation of a drug for the treatment, or the prevention of viral infection, in particular for the treatment of or the prevention of SARS-CoV-2 infections, or the treatment or the prevention of COVID-19.

In one other aspect, the invention relates to a method for preventing or treating viral infection in a subject in a need thereof, comprising administering to the subject an effective amount of the compound as defined or an effective amount of the pharmaceutical composition as defined above.

The dosages and the galenic form of the compound use in this use or method are as defined above.

The invention also relates to a kit comprising
- a compound as defined above; and
- a vaccine against a viral infection, or an antiviral compound, or a mixture thereof.

Vaccine against a viral infection can be either an attenuated virus, i.e. a virus deficient in its replication process but liable to infect target cells, or a nucleic acid molecule of the virus liable to produce viral protein to elicit an immune response.

Antiviral compound can be one of the followings: interferon, remdesivir, azithromycin, hydroxychloroquine, chloroquine tocilizumab and sarilumab.

It is also possible to use the above combination, wherein both the compound as defined above and the vaccine against a viral infection, or the antiviral compound are used simultaneously, separately, or at a different time.

In the above kit, when remdesivir is present, it is present in an amount between about 1 mg and about l00mg, preferably from 10mg to 50 mg, more preferably from 20 mg to 30 mg.

The invention will be better understood in light of the following examples and the following drawings.

### Brief description of the drawings

**[****Fig. 1****]** Vero E6 cells were treated with the indicated RG derivatives or Remdesivir at indicated concentrations and subsequently infected with SARS-CoV-2 at MOI 0.01 for 48 h in the presence of the compounds. The graph shows relative SARS-CoV-2 RNA (vRNA) levels measured by RT-qPCR and normalized by Rpl27 RNA expression. The bars are means +/- SD from duplicates representative of two independent experiments. Student's t-test ** p value < 0.01; *** p value < 0.005; **** p value < 0.001.
**[****Fig. 2****]** Cytotoxicity measured by LDH release assay showed no significant differences between the compounds.
**[****Fig. 3****]** Vero E6 cells were treated with RG10 at various concentrations and subsequently infected and processed as in [Fig. 1]. The IC₅₀ was 1.55 µM +/- 0.11 as measured from three independent experiments.
**[****Fig. 4****]** Vero E6 (monkey kidney cells), Caco2 (human intestinal cells) or Huh7.5.1 cells (human liver cells), were treated with 10 µM RG10 and subsequently infected with CoV2 at MOI 0.01 for 48 h in the presence of the compounds. The graph shows relative CoV2 RNA (vRNA) levels measured by RT-qPCR and normalized by Rpl27 RNA expression. The bars are means +/- SD from duplicates representative of two independent experiments. Student's t-test ** p value < 0.01; *** p value < 0.005; **** p value < 0.001.
**[****Fig. 5****]** Huh7.5.1 cells were treated with 10 µM RG10 or 10 µM Remdesivir and subsequently infected with CoV2 at indicated MOI for 48 h. The graph shows relative vRNA levels measured by RT-qPCR and normalized by Rpl27 RNA expression. The bars are means +/- SD from duplicates representative of two independent experiments. Student's t-test * p value < 0.05 and **** p value < 0.001.
**[****Fig. 6****]** Huh7.5.1 cells were treated with 10 µM RG10 or 10 µM Remdesivir and subsequently infected with SARS-CoV-2 at MOI 0.5 for 48 h. Cells were then lysed 48 h post infection. The lysates were processed for western blotting using an anti SARS-CoV-2 Spike antibody and an anti-GAPDH antibody as loading control. Both immunostainings were revealed using secondary antibodies coupled to HRP.
**[****Fig. 7****]** Cells were infected as in [Fig. 6] then fixed and stained using an anti SARS-CoV-2 Nucleocapsid antibody and a J2 antibody recognizing double stranded RNA (dsRNA) representative of viral replication complexes. The micrographs show the individual channels that have been acquired using a confocal microscope and processed using ImageJ.
**[****Fig. 8****]** Samples were treated as in [Fig. 8] and cells were detached and fixed 48 h post infection. Samples were stained and analyzed by flow cytometry. Student's t-test * p value < 0.05.
**[****Fig. 9****]** A scheme representing the time-of-addition experiment is shown.
**[****Fig. 10****]** Vero E6 cells were non-infected (NI), or infected with CoV2 at MOI 0.1 (SARS-CoV-2). Cells were treated with 10 µM RG10 during the first 4 h of infection and washed-out (RG10 Pre), starting at 4 h post-infection (RG10 Post), or during the whole infection (RG10 all).
**[****Fig. 11****]** Cells were lysed after 24 h and processed for flow cytometry, staining for dsRNA.
**[****Fig. 12****]** Cells were lysed after 24 h and processed for flow cytometry, staining for the N protein of CoV2.
**[****Fig. 13****]** Schematic representation of RG10 molecule coupled to fluorescent Rhodamine (RG10-Rhod) consisting of covalently linked RG10 and Rhodamine moieties.
**[****Fig. 14****]** Vero E6 cells were treated with indicated concentrations of RG10 or RG10-Rhod and subsequently infected with SARS-CoV-2 at MOI 0.01 for 48 h in the presence of the compounds. The graph shows relative vRNA levels measured by RT-qPCR and normalized by Rpl27 RNA expression. The bars are means +/- SD from duplicates representative of two independent experiments. Student's t-test *** p value < 0.005; **** p value < 0.001.
**[****Fig. 15****]** Huh7.5.1 cells were treated with 25 µM of RG10-Rhod or free Rhodamine for 3 h. Cells were fixed and stained using an anti-Calnexin antibody as a marker for ER membranes and DAPI for nuclear staining. One channel corresponds to rhodamine fluorescence. The micrographs show the individual channels and merge that have been acquired using confocal microscopy and processed using ImageJ. Scale bar = 10 µm.
**[****Fig. 16****]** Vero E6 cells were treated with 1 or 5 µM of RG10-Rhod and then were infected with CoV2 for 24 h in the presence of the inhibitors. Samples were fixed and dsRNA staining was performed to localize viral replication complexes.
**[****Fig. 17****]** Huh.7.5.1 cells were treated with 50 µM of RG10 for 6 h and then were processed for confocal imaging.
**[****Fig. 18****]** Huh7.5.1 cells were treated with 50 µM RG7 (an inactive derivative of RG10), 50 µM RG10 or 1 mM DTT for 6 h. Cells were then fixed and stained using an anti-Calnexin antibody as a marker for ER membranes and DAPI for nuclear staining. The micrographs show the individual channels and merge that have been acquired using confocal microscopy and processed using ImageJ. Scale bar = 10 µm.
**[****Fig. 19****]** Huh7.5.1 cells were treated as in A. The graph shows relative RNA levels measured by RT-qPCR and normalized by GAPDH RNA expression. The bars are means +/- SD from duplicates representative of two independent experiments. Student's t-test ** p value < 0.01 and **** p value < 0.001.
**[****Fig. 20****]** Huh.7.5.1 cells were treated with either 10 µM or 50 µM of RG10 or RG7 for 6 or 24 h. Exposure for 6 h to 1 mM of DTT was used as a positive control of ER stress. The graph shows relative spliced sXBP-1 levels measured by RT-qPCR and normalized by GAPDH RNA expression.
**[****Fig. 21****]** Vero E6 cells were infected with WT or N-mRuby3 CoV2 for 3 h at MOI 1. Cells were fixed, permeabilized and stained with DAPI (blue) and an anti-spike antibody (green). Images are from a single z plan acquired by spinning disk confocal microscopy. Scale bar = 10 µm. Magnified images from the dashed square highlight the co-distribution of N-mRuby3-containing virus particles (magenta) and Spike. The zoomed region is represented as single and merge images. Scale bar = 2 µm.
**[****Fig. 22****]** Vero E6 or Vero E6 N-mRuby3 expressing cells were infected with CoV2 for 3 days. The graph shows relative supernatant vRNA levels measured by RT-qPCR and normalized by Rpl27 RNA expression.
**[****Fig. 23****]** Huh.7.5.1 cells were labeled for the endoplasmic reticulum using the ER painter reagent and then infected with N-mRuby3 CoV2 at an MOI 1. Cells were either incubated with DMSO or 10 µM of RG10 during infection. The micrographs represent snapshots from Movies (not shown). The white arrows highlight a single particle over time for each condition. Scale bar = 10 µm.
**[****Fig. 24****]** CoV2 particles were individually segmented using the same intensity threshold for all conditions and particles were tracked using the Imaris software. The figure represents tracking trails in DMSO or RG10-treated cells associate to Supplemental Movies S3 and S4. The RG10 condition shows viral particles with barely any visible track because the viruses were extremely steady.
**[****Fig. 25****]** Quantification of the mean speed are shown for n = 8 movies per conditions with > 500 virus per condition. Student's t-test *** p value < 0.005.
**[****Fig. 26****]** Tracking duration are shown for n = 8 movies per conditions with > 500 virus per condition. Student's t-test *** p value < 0.005.
**[****Fig. 27****]** Vero E6 WT cells and Vero E6 cells stably expressing N-mRuby3 were infected for 48 h at MOI 0.01 with CoV2. Cells were fixed, permeabilized and stained with Dapi and an anti-Spike antibody. N-mRuby3 is shown in magenta. Merged immunofluorescence images are from a single z plan acquired by spinning disk confocal microscopy. Scale bar = 10 µm.
**[****Fig. 28****]** Vero E6 WT cells and Vero E6 cells stably expressing N-mRuby3 were infected for 48 h at MOI 0.01 with CoV2. Cells were fixed, permeabilized and stained with Dapi and an anti-dsRNA antibody. N-mRuby3 is shown in magenta. Merged immunofluorescence images are from a single z plan acquired by spinning disk confocal microscopy. Scale bar = 10 µm.
**[****Fig. 29****]** Vero E6 or Vero E6 N-mRuby3 expressing cells were infected with CoV2 for 3 days. Supernatants were then used to perform plaque assays on Vero E6 cells.
**[****Fig. 30****]** Vero E6 cells were treated with 10 µM RG10 or 1 µM Tunicamycin (Tm) and infected with CoV2 at MOI 0.01 for 24 h in the presence of the compounds. The graph shows relative vRNA levels measured by RT-qPCR and normalized by GAPDH RNA expression. The bars are means +/- SD from duplicates representative of two independent experiments. Student's t-test *** p value < 0.005; **** p value < 0.001.
**[****Fig. 31****]** Vero E6 cells were treated with 10 µM RG10 or 1 µM Tunicamycin (Tm) and infected with ZIKV at MOI 0.3 for 24 h in the presence of the compounds. The graph shows relative vRNA levels measured by RT-qPCR and normalized by GAPDH RNA expression. The bars are means +/- SD from duplicates representative of two independent experiments. Student's t-test *** p value < 0.005; **** p value < 0.001.
**[****Fig. 32****]** HuH7.5.1 cells were treated with 10 µM RG10 and infected with HCoV-229E-Renilla at MOI 2 for 24 h in the presence of the compound.
**[****Fig. 33****]** HAE cells were either untreated (Mock) or treated with 10 µM of RG10 and then infected with SARS-CoV-2 mNeonGreen virus at MOI 0.1. After 4 days, cells were fixed and stained for CoV2 spike protein and actin filaments (phalloidin staining). Z-stack images were acquired using a confocal microscope and processed using ImageJ to produce an average intensity image for each individual channel. Scale bar = 20 µm.
**[****Fig. 34****]** HAE cells were either untreated (Mock) or treated with 10 µM of RG10 and then infected with SARS-CoV-2 mNeonGreen virus at MOI 0.1. After 4 days, cells were fixed and stained for CoV2 spike protein and actin filaments (phalloidin staining). Z-stack images were acquired using a confocal microscope and processed using ImageJ to produce an average intensity image for each individual channel. Scale bar = 20 µm.
**[****Fig. 35****]** 3D representation of the images from [Fig. 33] and [Fig. 34] using the Imaris software. Scale bar = 20 µm.
**[****Fig. 36****]** HAE cells were treated with 10 µM of RG10 and Remdesivir and then infected with wild type CoV2 at MOI 0.01 for 4 days. Graphs show relative sXBP1 RNA levels normalized with GAPDH RNA expression.
**[****Fig. 37****]** Same samples from [Fig. 36] were processed to measure by RT-qPCR, relative vRNA levels normalized by Rpl27 RNA expression. The bars are means +/- SD from two pooled independent experiments. Student's t-test ** p value < 0.01; *** p value < 0.005; **** p value < 0.001.
**[****Fig. 38****]** Vero E6 cells were treated with the indicated concentrations of RG10 or RG10b and then infected with CoV2 for 48 h. Graphs show relative vRNA levels normalized with Rpl27 RNA expression.
**[****Fig. 39****]** Vero E6 cells were treated with RG10b at different concentrations and subsequently infected and processed as in [Fig. 38]. The IC₅₀ of RG10b was ≈ 0.99 µM.
**[****Fig. 40****]** Table shows T_{1/2} of RG10 and RG10b as determined by pharmacokinetics experiments in mice.
**[****Fig. 41****]** Graph shows weight of mice after treatment with various concentrations of RG10b. Two mice were used per condition.
**[****Fig. 42****]** HAE cells were treated with 10 µM of RG10, RG10b, and Remdesivir and then infected for 4 days with CoV2 at MOI 0.01. Cells were then processed for RNA extraction and RT-qPCR experiments. Graph shows relative vRNA levels normalized with Rpl27 RNA expression. Student's t-test * p value < 0.05 ** p value < 0.01; *** p value < 0.005.
**[****Fig. 43****]** The graph shows relative SARS-CoV-2 RNA (vRNA) levels measured by RT-qPCR and normalized by Rpl27 RNA expression further to treatment with the indicated drug at a concentration of 10µM. The bars are means +/- SD from quadruplicates.
**[****Fig. 44****]** The graph shows relative SARS-CoV-2 RNA (vRNA) levels measured by RT-qPCR and normalized by Rpl27 RNA expression further to treatment with the indicated drug at a concentration varying from 2µM to 25µM.

### Examples

### Example 1 - Synthesis of compounds according to the invention

### A- General procedures for the compounds according to the invention

### Flash chromatography

▪ Apparatus: Biotage SP with auto-collector and UV detection (2 wavelengths).
▪ Normal phase columns: 120g or 300g Biotage external dry load cartridge kit, packed with Sigma-Aldrich 40-63µm silica gel.

### Liquid Chromatography:

▪ Apparatus: Waters alliance 2695 HPLC system with autosampler and Waters 2996 diode array detector.
▪ Column: Macherey-Nagel Nucleoshell RP18 plus (5 µm, 4 mm × 100 mm).
▪ Column temperature: 40°C.
▪ Solvents: A (H₂O 99.9%, H₂CO₂ 0.1%); B (CH₃CN 99.9%, H₂CO₂ 0.1%).
▪ Flow rate: 1mL/min.
▪ Gradient (A/B v/v): 90/10 (t=0min), 90/10 (t=1min), 0/100 (t=7min), 0/100 (t=10min).
▪ Detection: 210-400nm range.

### Chiral Chromatography:

▪ Chiral column: Daicel ChiralPak IG (Amylose-based) 20 µm, 4.6 mm × 100 mm
▪ Column temperature: 25°C
▪ Solvents: A (Heptane, 50%, EtOH containing 0.1% Et₃N, 40% and DCM, 10%)
▪ Flow rate: 1mL/min.
▪ Isocratic conditions.

### Mass Spectrometer:

▪ Apparatus: Waters Micromass ZQ (simple quad).
▪ Mass detection method: Electrospray positive mode (ESI+), mass range: 50-800 uma.

### NMR Spectrometer:

▪ Apparatus: Bruker 400 MHz.
▪ Methods: 1H NMR spectra performed in DMSO-d6 using DMSO-d5 as internal reference, chemical shifts expressed in parts per million (ppm), signals expressed as follows: s = singlet, d = doublet, t = triplet, q = quadruplet, sept = septuplet, dd = double doublet, dt = double triplet, m = multiplet or large singlet, br = broad, H = proton.
▪ ³¹P NMR spectra performed in DMSO-d6, chemical shifts expressed in parts per million (ppm), signals expressed as follows: s = singlet.

### General procedure A for alkylation of phenol derivatives

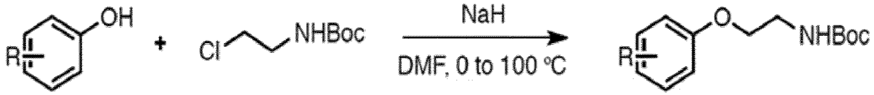

Phenol (1 equiv) was dissolved in dry DMF. The clear solution was cooled to 0 °C and Sodium Hydride (60% in mineral oil, 1.5 equiv) was added portionwise. A strong gaz evolution was observed. Mixture was stirred for 45 min and 1,1-Dimethylethyl *N*-(2-chloroethyl)carbamate (1.1 equiv) was added. Resulting mixture was stirred at room temperature overnight. Reaction mixture was diluted in 50 mL EtOAc then washed with saturated ammonium chloride solution (2 × 25 mL), water (2 × 25 mL) and brine (25 mL). Organic layer was separated and dried over anhydrous sodium sulfate, filtered and concentrated to dryness. The crude mixture was purified by flash chromatography using a DCM-EtOAc or Hexanes-EtOAc gradient mixtures as eluent.

### General Procedure B for boc deprotection

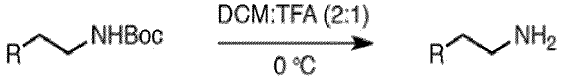

Boc-protected amine was dissolved in DCM and the mixture cooled to 0 °C. TFA was then added dropwise to afford a 1:2 DCM:TFA ratio. Resulting mixture is stirred for 1 h, initially at 0 °C and allowed to reach room temperature. After this time, reaction mixture was concentrated to dryness and the residue was quenched with addition of a saturated sodium bicarbonate solution (30 mL). The organic layer was extracted with butanol (3 × 30 mL) and concentrated to dryness. The crude product was purified by reverse phase chromatography using a mixture of H₂O-MeOH.

### General procedure C for thioamide coupling

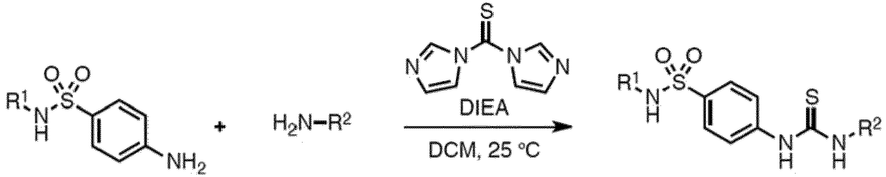

Arylsulfonamide (1equiv) was suspended in dry DCM (0.2 M) under inert atmosphere. Diisopropylethylamine (3 equiv) was added to the mixture, followed by thiocarbonyldiimidazole (1 equiv). The reaction mixture was stirred at room temperature overnight. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes.

### Procedure B for the preparation of sulfonamide intermediates

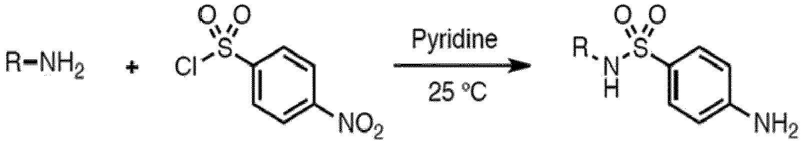

Amine (1 equiv) was suspended in dry pyridine (0.25 M). Sulfonyl chloride (1 equiv) was added in one drop and the orange mixture was stirred at room temperature for 4 h. After this time, reaction mixture was concentrated to dryness. The resulting solid was suspended in EtOAc (100 mL), washed with water (50 mL), saturated ammonium chloride solution (2 × 50 mL) and brine (50 mL). The organic layer was separated and dried over anhydrous sodium sulfate. The yellow crude solid was then purified by flash chromatography using an Hexanes-EtOAc gradient mixture as eluent.

### General procedure for nitro reduction

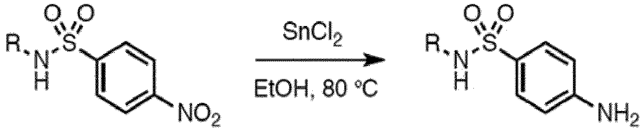

4-nitro-arylsulfonamide (1 equiv) was suspended in absolute ethanol (90 mL). Tin dichloride (5 equiv) was added and the resulting mixture was heated at reflux for 2.5 h. Reaction mixture was cooled to room temperature and poured into ice. A saturated sodium bicarbonate solution (50 mL) was added carefully to reach the pH up to 7-8. Mixture was diluted with EtOAc (150 mL) and DCM (50 mL). Organic layer was separated and dried over anhydrous sodium sulfate, filtered and concentrated to dryness.

### General procedure for aryl coupling

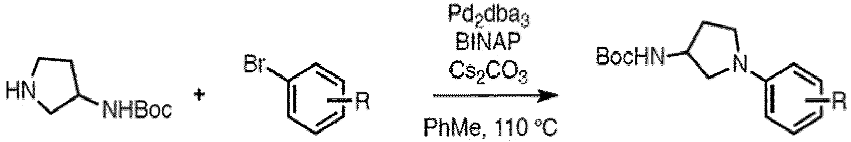

Boc-Amino-pyrrolidine (1 equiv) and cesium carbonate (1.2 equiv) were dissolved in anhydrous Toluene (0.3 M) and arylbromide (1.15 equiv) was added. The resulting mixture was degassed under argon for 5 min and Pd₂(dba)₃ (6 mol%) was added, followed by BINAP (15 mol %). The mixture was then stirred at 110 °C under argon atmosphere for 5 h. After this time, the reaction mixture was diluted in ethyl acetate, washed with water (3 × 20 mL) and brine (20 mL). The organic layer was separated and concentrated to dryness. The crude product was purified by flash chromatography using a mixture of EtOAc in Hexanes.

### B- Synthesis of specific compounds according to the invention 1- RG10

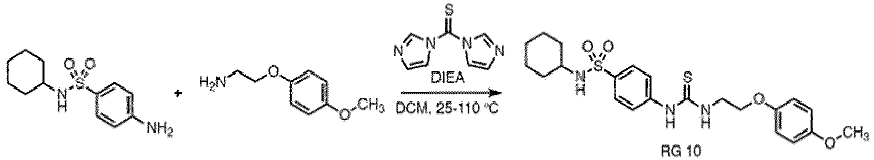

Amine compound (152 mg, 0.598 mmol) was suspended in dry DCM (2 mL) under inert atmosphere. Diisopropylethylamine (229 µL) was added to the mixture, followed by thiocarbonyldiimidazole (0.598 mmol, 107 mg). The reaction mixture was stirred at room temperature for 1 h then arylsulfonamide (100 mg, 0.598 mmol) was added and the reaction mixture was stirred at room temperature overnight. After this time, reaction mixture was heated under microwave irradiation for 1 h at 120 °C. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of MeOH in H₂O. Compound **RG10** was obtained as a white solid (49 mg, 18 % yield).
**LCMS:** Rt (6.48 min), 464.8 (M+1), 98% purity
**¹H NMR (400 MHz, DMSO-d6) δ** 9.98 (s, 1H), 8.24 (s, 1H), 7.72 (s, 4H), 7.54 (d, J = 7.3 Hz, 1H), 6.98 ― 6.83 (m, 4H), 4.10 (t, J = 5.5 Hz, 2H), 3.85 (d, J = 5.6 Hz, 2H), 3.70 (s, 3H), 2.91 (s, 1H), 1.58 (d, J = 8.1 Hz, 4H), 1.45 (d, J = 11.9 Hz, 1H), 1.20 ― 0.97 (m, 5H).

### 2- RG11

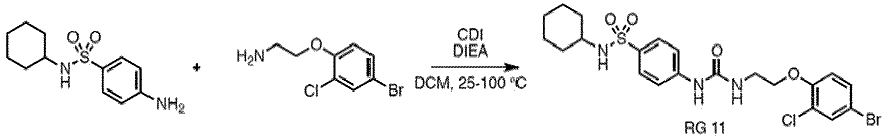

Arylsulfonamide (100 mg, 0.399 mmol) was suspended in dry DCM (2 mL) under inert atmosphere. Diisopropylethylamine (151 µL) was added to the mixture, followed by carbonyldiimidazole (0.399 mmol, 65 mg). The reaction mixture was stirred at room temperature for 45 min then amine compound (101 mg, 0.399 mmol) was added and the reaction mixture was heated under microwave irradiation for 1.5 h at 100 °C. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of MeOH in H₂O. Compound RG11 was obtained as a white solid (22.7 mg, 11 % yield).
**LCMS :** Rt (6.83 min), 530.5; 532.5; 534.5(M; M+2; M+4), 99% purity
**¹H NMR (400 MHz, DMSO-d6)** δ 9.07 (s, 1H), 7.73 ― 7.60 (m, 3H), 7.55 (d, J = 8.6 Hz, 2H), 7.49 (dd, J = 8.9, 2.5 Hz, 1H), 7.40 (d, J = 7.3 Hz, 1H), 7.17 (d, J = 8.9 Hz, 1H), 6.50 (t, J = 5.8 Hz, 1H), 4.12 (t, J = 5.6 Hz, 2H), 3.51 (q, J = 5.6 Hz, 2H), 2.86 (s, 1H), 1.67 ― 1.49 (m, 4H), 1.43 (d, J = 11.6 Hz, 1H), 1.19 ― 0.91 (m, 5H).

### 3- RG12

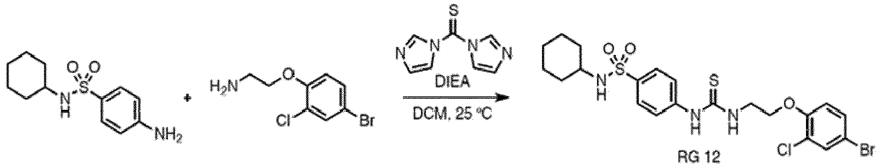

Amine compound (102 mg, 0.400 mmol) was suspended in dry DCM (2 mL) under inert atmosphere. Diisopropylethylamine (152 µL) was added to the mixture, followed by thiocarbonyldiimidazole (0.400 mmol, 71 mg). The reaction mixture was stirred at room temperature for 45 min then arylsulfonamide (100 mg, 0.400 mmol) was added and the reaction mixture was stirred at room temperature for 2 h. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of MeOH in H₂O. Compound RG12 was obtained as a white solid (26 mg, 23 % yield).
**LCMS :** Rt (7.15 min), 546.5; 548.5; 550.5(M; M+2; M+4), 99% purity
**¹H NMR (400 MHz, DMSO-d6)** δ 10.01 (s, 1H), 8.23 (s, 1H), 7.80 ― 7.64 (m, 4H), 7.60 ― 7.41 (m, 2H), 7.19 (d, J = 8.9 Hz, 1H), 4.26 (t, J = 5.6 Hz, 2H), 3.90 (t, J = 5.5 Hz, 2H), 2.89 (s, 1H), 1.56 (d, J = 7.6 Hz, 3H), 1.43 (d, J = 12.1 Hz, 1H), 1.23 ― 0.92 (m, 5H).

### 4- RG17

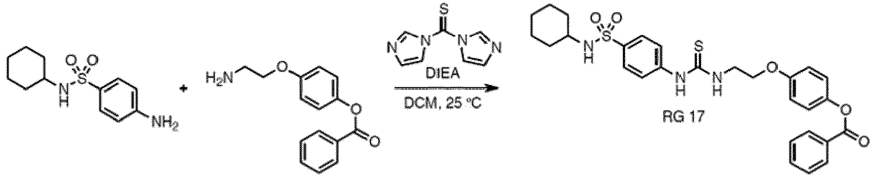

Arylsulfonamide (0.435 mmol, 110 mg) was suspended in dry DCM (2 mL) under inert atmosphere. Diisopropylethylamine (166 µM) was added to the mixture, followed by thiocarbonyldiimidazole (0.435 mmol, 78 mg). The reaction mixture was stirred at room temperature for 2 h then amine compound (112 mg, 0.435 mmol). The reaction mixture was stirred at room temperature overnight. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of MeOH in H₂O. Compound RG17 was obtained as an off-white solid (14.5 mg, 7.4% yield).
LCMS: Rt (5.92 min), 450.8 (M+1), 96% purity
**¹H NMR (400 MHz, DMSO-d6)** δ 10.02 (s, 1H), 8.93 (s, 1H), 8.66 - 8.45 (m, 1H), 8.28 (s, 1H), 7.71 (s, 4H), 7.52 (d, J = 7.2 Hz, 1H), 7.48 ― 7.29 (m, 1H), 6.91 ― 6.75 (m, 2H), 6.75 ― 6.55 (m, 2H), 4.17 ― 3.96 (m, 2H), 3.82 (s, 2H), 2.90 (s, 1H), 1.57 (d, J = 8.1 Hz, 3H), 1.43 (d, J = 12.0 Hz, 2H), 1.27 ― 0.87 (m, 5H).

### 5- RG27

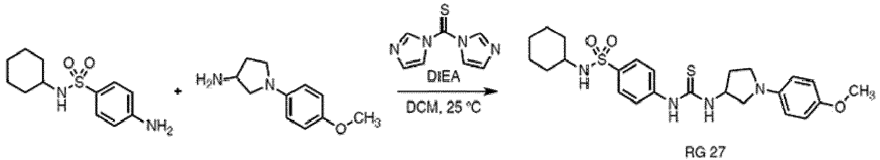

Arylsulfonamide (90 mg, 0.354 mmol) was suspended in dry dichloromethane (5 mL) under inert atmosphere. Diisopropylethylamine (135 µL) was added to the mixture, followed by thiocarbonyldiimidazole (63 mg, 0.354 mmol). The reaction mixture was stirred at room temperature for 2 h. Amide compound (68 mg, 0.354 mmol) was added and the resulting mixture was stirred at room temperature for 1.5 h. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of Acetonitrile in H₂O. Compound RG27 was obtained as a pale yellow solid (72 mg, 41 % yield).
**LCMS:** Rt (6.77 min), 489.7 (M+1), 97% purity
**¹H NMR (400 MHz, DMSO-d6)** δ 9.76 (s, 1H), 8.35 (d, J = 6.8 Hz, 1H), 7.82 ― 7.63 (m, 4H), 7.53 (d, J = 7.3 Hz, 1H), 6.82 (d, J = 8.6 Hz, 2H), 6.53 (d, J = 8.6 Hz, 2H), 4.87 (s, 1H), 3.66 (s, 3H), 3.50 (dd, J = 9.8, 6.2 Hz, 1H), 3.20 (ddt, J = 13.3, 9.4, 4.4 Hz, 2H), 2.89 (s, 1H), 2.28 (q, J = 6.7 Hz, 2H), 2.03 (dq, J = 12.6, 5.9 Hz, 1H), 1.56 (d, J = 7.4 Hz, 4H), 1.44 (d, J = 12.0 Hz, 1H), 1.10 (dq, J = 15.8, 8.0 Hz, 5H).

### 6- RG28

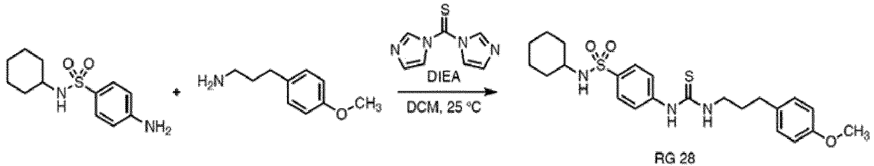

Arylsulfonamide (40 mg, 0.157 mmol) was suspended in dry dichloromethane (2 mL) under inert atmosphere. Diisopropylethylamine (60 µL) was added to the mixture, followed by thiocarbonyldiimidazole (28 mg, 0.157 mmol). The reaction mixture was stirred at room temperature for 3 h. Amide compound (26 mg, 0.157 mmol) was added and the resulting mixture was stirred at room temperature for 3 h. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of Acetonitrile in H₂O. Compound RG28 was obtained as a white solid (29 mg, 40 % yield).
**LCMS:** Rt (6.73 min), 462.5 (M+1), 99% purity
**¹H NMR (400 MHz, DMSO-d6)** δ 9.81 (s, 1H), 8.12 (s, 1H), 7.69 (d, J = 2.2 Hz, 4H), 7.54 (d, J = 7.3 Hz, 1H), 7.14 (d, J = 8.3 Hz, 2H), 6.85 (d, J = 8.3 Hz, 2H), 3.71 (s, 3H), 3.47 (s, 2H), 2.90 (s, 1H), 2.57 (t, J = 7.7 Hz, 2H), 1.83 (p, J = 7.4 Hz, 2H), 1.57 (d, J = 8.0 Hz, 4H), 1.44 (d, J = 11.7 Hz, 1H), 1.21 ― 0.90 (m, 5H).

### 7- RG29

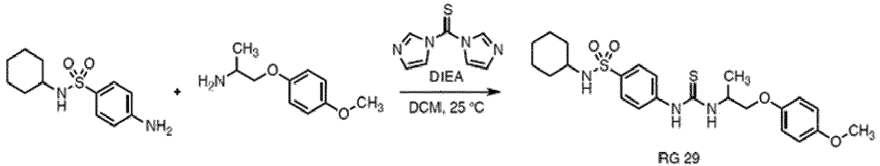

Arylsulfonamide (40 mg, 0.157 mmol) was suspended in dry dichloromethane (2 mL) under inert atmosphere. Diisopropylethylamine (60 µL) was added to the mixture, followed by thiocarbonyldiimidazole (28 mg, 0.157 mmol). The reaction mixture was stirred at room temperature for 3 h. Amine compound (29 mg, 0.157 mmol) was added and the resulting mixture was stirred at room temperature for 3 h. The reaction mixture was diluted in EtOAc (40 mL), water (2 × 20 mL) and brine (20 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of Acetonitrile in H₂O. Compound RG29 was obtained as a white solid (26 mg, 34 % yield).
**LCMS:** Rt (6.73 min), 478.5 (M+1), 99% purity
**¹H NMR (400 MHz, DMSO-d6)** δ 9.87 (s, 1H), 8.15 (d, J = 7.8 Hz, 1H), 7.81 ― 7.61 (m, 4H), 7.54 (d, J = 7.3 Hz, 1H), 7.01 ― 6.73 (m, 4H), 4.68 (s, 1H), 3.99 (ddd, J = 33.8, 9.6, 5.1 Hz, 2H), 3.70 (s, 3H), 2.89 (s, 1H), 1.56 (d, J = 7.8 Hz, 4H), 1.44 (d, J = 11.7 Hz, 1H), 1.27 (d, J = 6.7 Hz, 3H), 1.20 ― 0.81 (m, 5H).

### 8- RG31

### Reaction sequence for the preparation of RG31

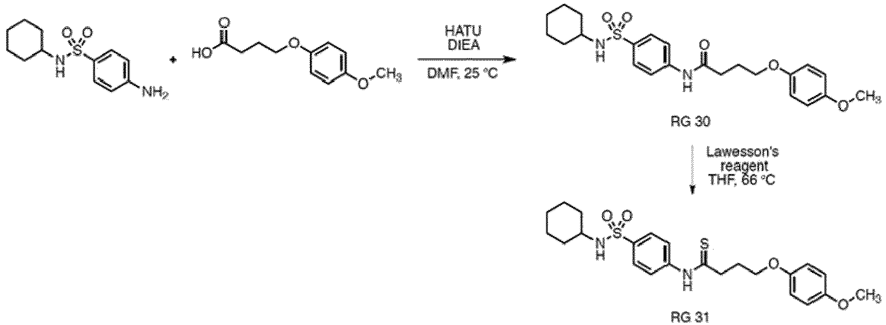

### Procedure for the preparation of RG30

Arylsulfonamide (50 mg, 0.238 mmol) was suspended in dry dimethylformamide (2 mL) under inert atmosphere. HATU (109 mg, 0.285 mmol) was added to the mixture, followed by diisopropylethylamine (123 µL, 0.714 mmol). Carboxylic acid (73 mg, 0.285 mmol) was added and the resulting mixture was stirred at room temperature overnight. After this time, the crude mixture was directly purified by reverse phase chromatography using a mixture of Acetonitrile in H₂O. Compound RG30 was obtained as a white solid (79 mg, 74 % yield).

### Procedure for the preparation of RG31

RG30 (79 mg, 0.177 mmol) was dissolved in dry tetrahydrofuran (3 mL) under inert atmosphere. Lawesson reagent (61 mg, 0.152 mmol) was added to the mixture. The reaction mixture was stirred at 66 °C for 5 h. After this time, reaction mixture was diluted with a saturated sodium bicarbonate solution (20 mL) and organic layer was extracted with DCM (3 × 20 mL) and concentrated to dryness. The crude product was purified by flash chromatography using a mixture of EtOAc in Hexanes, followed by reverse phase chromatography using a mixture of Acetonitrile in H₂O. Compound RG31 was obtained as a white solid (34 mg, 41 % yield).
**LCMS:** Rt (6.96 min), 463.4 (M+1), 98% purity
**¹H NMR (400 MHz, DMSO-d6)** δ 11.82 (s, 1H), 8.12 ― 7.97 (m, 2H), 7.90 ― 7.74 (m, 2H), 7.63 (d, J = 7.3 Hz, 1H), 6.95 ― 6.73 (m, 4H), 3.98 (t, J = 6.2 Hz, 2H), 3.01 ― 2.82 (m, 3H), 2.26 ― 2.09 (m, 2H), 1.55 (d, J = 7.2 Hz, 4H), 1.43 (d, J = 12.0 Hz, 1H), 1.21 ― 0.89 (m, 5H).

### 9- RG36

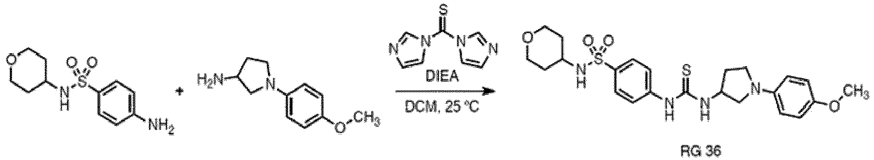

Arylsulfonamide (50 mg, 0.195 mmol) was suspended in dry DCM (3 mL) under inert atmosphere. Diisopropylethylamine (74 µL, 0.429 mmol) was added to the mixture, followed by thiocarbonyldiimidazole (35 mg, 0.195 mmol,). The reaction mixture was stirred at room temperature 3.5 h. Amine compound (38 mg, 0.195 mmol) was added and the resulting mixture was stirred at room temperature for 1 h. The reaction mixture was diluted in EtOAc (50 mL), water (2 × 25 mL) and brine (25 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of MeOH in H₂O. Compound RG36 was obtained as a yellow solid (31 mg, 32 % yield).
**LCMS:** Rt (6.27 min), 491.5 (M+1), 99% purity
**¹H NMR (400 MHz, DMSO-d6)** δ 9.79 (s, 1H), 8.38 (s, 1H), 7.82 ― 7.58 (m, 5H), 6.87 ― 6.72 (m, 2H), 6.61 ― 6.42 (m, 2H), 4.86 (s, 1H), 3.70 (dt, J = 11.7, 3.7 Hz, 2H), 3.65 (s, 3H), 3.49 (dd, J = 9.8, 6.2 Hz, 1H), 3.39 - 3.34 (m, 1H), 3.25 ― 3.07 (m, 5H), 2.36 ― 2.21 (m, 1H), 2.09 ― 1.95 (m, 1H), 1.49 (td, J = 8.5, 3.5 Hz, 2H), 1.32 (dtd, J = 12.9, 10.9, 4.3 Hz, 2H).

### 10- RG37

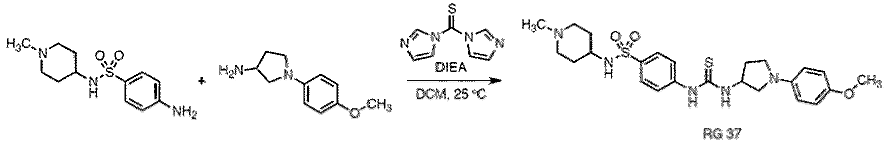

Arylsulfonamide (45 mg, 0.166 mmol) was suspended in dry DCM (3 mL) under inert atmosphere. Diisopropylethylamine (63 µL, 0.366 mmol) was added to the mixture, followed by thiocarbonyldiimidazole (30 mg, 0.166 mmol,). The reaction mixture was stirred at room temperature 3 h. Amine compound (32 mg, 0.166 mmol) was added and the resulting mixture was stirred at room temperature for 1 h. The reaction mixture was diluted in EtOAc (50 mL), water (2 × 25 mL) and brine (25 mL). The organic layer was separated and dried over anhydrous sodium sulfate, filtered and concentrated under reduced pressure. The crude product was purified by flash chromatography using a mixture of EtOAc in Hexanes, followed by reverse phase chromatography using a mixture of MeOH in H₂O. Compound RG37 was obtained as a yellow solid (36 mg, 43 % yield).
**LCMS:** Rt (6.27 min), 491.5 (M+1), 99% purity
**¹H NMR (400 MHz, DMSO-d6)** δ 9.81 (s, 1H), 8.61 (s, 1H), 8.38 (d, J = 6.9 Hz, 1H), 7.78 (d, J = 8.9 Hz, 2H), 7.74 ― 7.65 (m, 2H), 6.85 ― 6.75 (m, 2H), 6.58 ― 6.43 (m, 2H), 4.86 (s, 1H), 3.65 (s, 3H), 3.49 (dd, J = 9.8, 6.1 Hz, 1H), 3.40 ― 3.34 (m, 1H), 3.25 ― 3.12 (m, 3H), 2.45 (d, J = 4.8 Hz, 4H), 2.36 ― 2.24 (m, 2H), 2.18 (s, 3H), 2.05 (s, 4H).

### 11- RG38

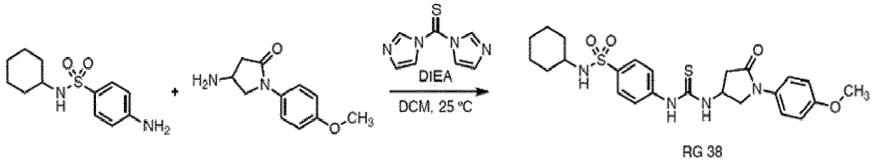

Arylsulfonamide (50 mg, 0.197 mmol) was suspended in dry DCM (3 mL) under inert atmosphere. Diisopropylethylamine (109 µL, 0.629 mmol) was added to the mixture, followed by thiocarbonyldiimidazole (35 mg, 0. 197 mmol,). The reaction mixture was stirred at room temperature 3.5 h. Amine compound (48 mg, 0.197 mmol) was added and the resulting mixture was stirred at room temperature for 1 h. The reaction mixture was diluted in EtOAc (50 mL), water (2 × 25 mL) and brine (25 mL). The organic layer was separated and dried over anhydrous sodium sulfate, filtered and concentrated under reduced pressure. The crude product was purified by flash chromatography using a mixture of MeOH in DCM, followed by reverse phase chromatography using a mixture of Acetonitrile in H₂O. Compound RG38 was obtained as a white solid (52 mg, 53 % yield).
**LCMS:** Rt (6.38 min), 503.6 (M+1), 99% purity
**¹H NMR (400 MHz, DMSO-d6)** δ 9.85 (s, 1H), 8.66 (s, 1H), 7.69 (d, J = 1.2 Hz, 4H), 7.63 ― 7.41 (m, 3H), 7.05 ― 6.80 (m, 2H), 4.92 (s, 1H), 4.19 (dd, J = 10.3, 6.7 Hz, 1H), 3.73 (s, 4H), 2.98 (dd, J = 17.2, 8.1 Hz, 1H), 2.89 (s, 1H), 2.53 (d, J = 4.0 Hz, 2H), 1.67 ― 1.47 (m, 4H), 1.43 (d, J = 11.8 Hz, 1H), 1.22 ― 0.85 (m, 5H).

### 12- RG41

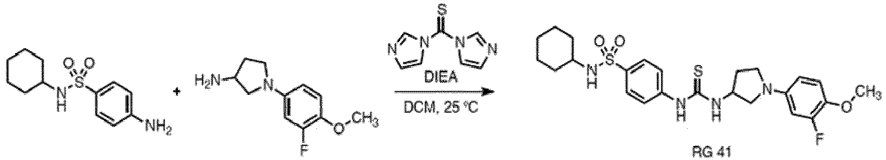

Arylsulfonamide (47 mg, 0.185 mmol) was suspended in dry DCM (3 mL) under inert atmosphere. Diisopropylethylamine (71 µL, 0.408 mmol) was added to the mixture, followed by thiocarbonyldiimidazole (33 mg, 0. 185 mmol,). The reaction mixture was stirred at room temperature 4.5 h. Amine compound (39 mg, 0.185 mmol) was added and the resulting mixture was stirred at room temperature for 30 min. The reaction mixture was diluted in EtOAc (50 mL), water (2 × 25 mL) and brine (25 mL). The organic layer was separated and dried over anhydrous sodium sulfate, filtered and concentrated under reduced pressure. The crude product was purified by flash chromatography using a mixture of MeOH in DCM, followed by reverse phase chromatography using a mixture of MeOH in H₂O. Compound RG41 was obtained as a white solid (42 mg, 45 % yield).
**LCMS:** Rt (7.07 min), 507.5 (M+1), 96% purity
**¹H NMR (400 MHz, DMSO-d6)** δ 9.76 (s, 1H), 8.36 (d, J = 7.0 Hz, 1H), 7.82 ― 7.65 (m, 4H), 7.54 (d, J = 7.3 Hz, 1H), 7.02 (dd, J = 9.9, 8.9 Hz, 1H), 6.47 (dd, J = 14.3, 2.8 Hz, 1H), 6.31 (ddd, J = 8.9, 2.9, 1.2 Hz, 1H), 4.88 (s, 1H), 3.73 (s, 3H), 3.52 (dd, J = 10.0, 6.1 Hz, 1H), 3.30 ― 3.11 (m, 2H), 2.90 (s, 1H), 2.37 ― 2.19 (m, 2H), 2.13 ― 1.97 (m, 1H), 1.71 ― 1.50 (m, 4H), 1.44 (d, J = 11.8 Hz, 1H), 1.22 ― 0.88 (m, 5H).

### Example 2 - Pharmacological perturbation of intracellular dynamics as a SARS-CoV-2 antiviral strategy

SARS-CoV-2 (CoV2) is the viral agent responsible for the pandemic of the coronavirus disease 2019 (COVID-19). Vaccines are being deployed all over the world with good efficacy, but there is no approved antiviral treatment to date. This is particularly needed since the emergence of variants and the potential immune escape may prolong pandemic spreading of the infection for much longer than anticipated. Here, the inventor developed a series of small molecules and identified RG10 as a potent antiviral compound against SARS-CoV-2 in cell lines and in human airway epithelia (HAE). RG10 localizes to endoplasmic reticulum (ER) membranes, perturbing ER morphology and inducing ER stress. Yet, RG10 does not associate with SARS-CoV-2 replication sites although preventing virus replication. To further investigate the antiviral properties of the compound, the inventor developed fluorescent SARS-CoV-2 viral particles allowing them to track virus arrival to ER membranes. Live cell imaging of replication-competent virus infection revealed that RG10 of formula: 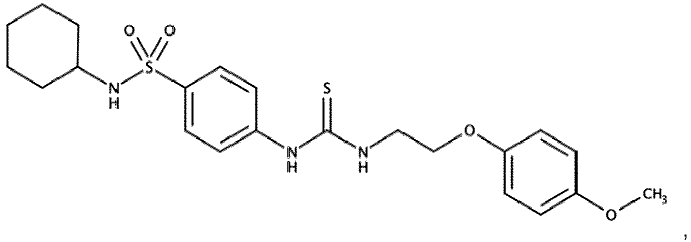 stalls the intracellular dynamics of virus-ER dynamics.

Finally, he synthesized RG10b, a stable version of RG10, of formula: 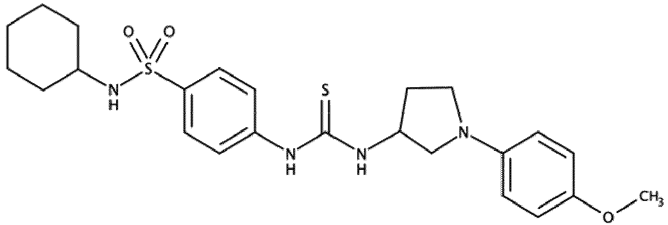 (RG10b or RG27),
that showed increased potency in vitro and in HAE with a pharmacokinetic half-life greater than 2 h. Together, the inventor's work reports on a novel fluorescent virus model and innovative antiviral strategy consisting of the perturbation of ER/virus dynamics, highlighting the promising antiviral properties of RG10 and RG10b.

### Results

### Identification of RG10 as a potent SARS-CoV-2 antiviral molecule

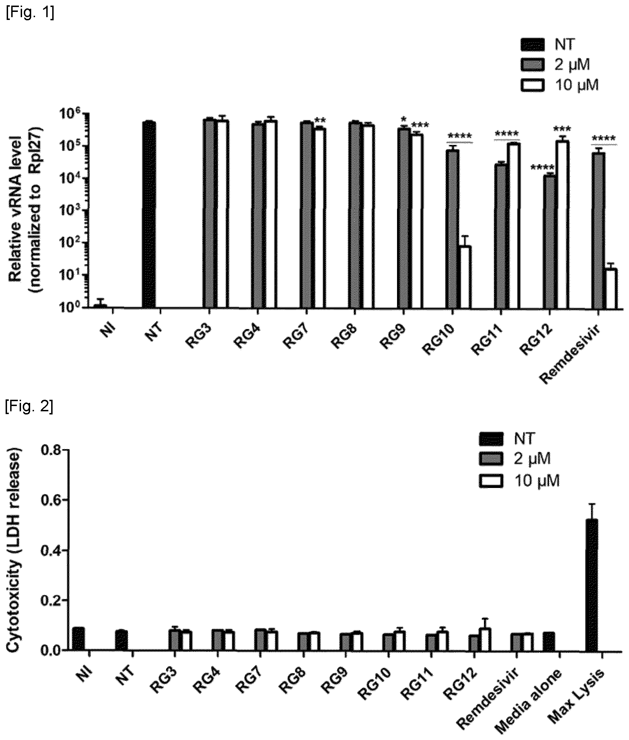

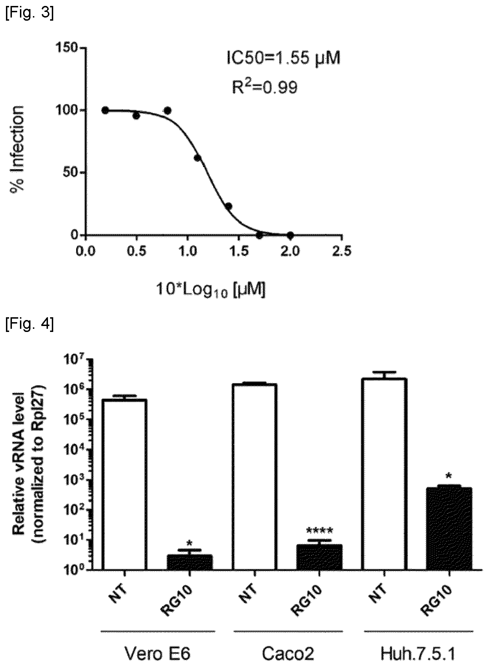

The inventor had synthesized a series of sulfamoyl-phenyl derived compounds designed as potential antiviral agents. While most of them did not prevent CoV2 replication at 2 and 10 µM, they identified the molecule RG10, a novel small molecule of unknown target, as a potent antiviral molecule with similar activity levels as Remdesivir (Rem; **Figure 1**). RG10 did not show cytotoxicity at working concentrations (**Figure 2**) and measurement of its potency in Huh7.5.1 cells returned an EC50 ≈ 1.5 µM (**Figure 3**).

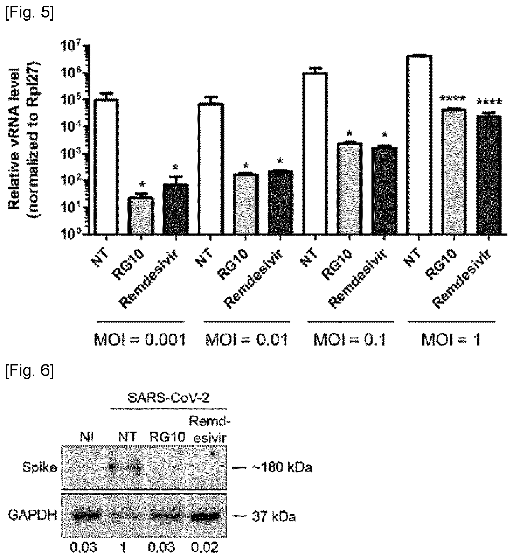

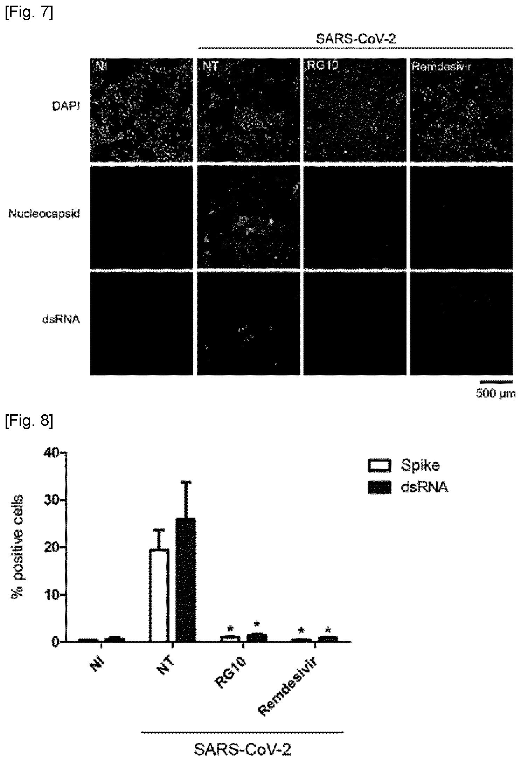

RG10 exhibited high CoV2 antiviral activity in Vero E6 cells but also in human Caco2 and Huh7.5.1 cells **(****Figure 4****)** and retained potency at high multiplicity of infection (MOI) similar to Rem (**Figure 5**). By Western blot, neosynthesized Spike protein (S) could barely be detected upon RG10 or Rem treatment (**Figure 6**). Microscopy and flow cytometry analyses confirmed that both RG10 and Rem prevent viral protein synthesis **(****Figures 7 and 8****).** Moreover, immunolabeling using an antibody recognizing double stranded RNA (dsRNA), specific of viral replication complexes, showed that RG10 prevents viral RNA synthesis **(****Figures 7 and 8****).**

### RG10 inhibits CoV2 replication

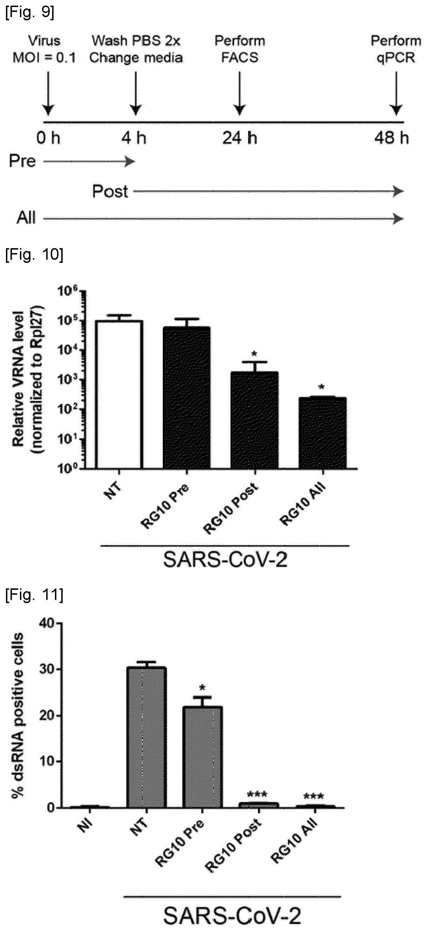

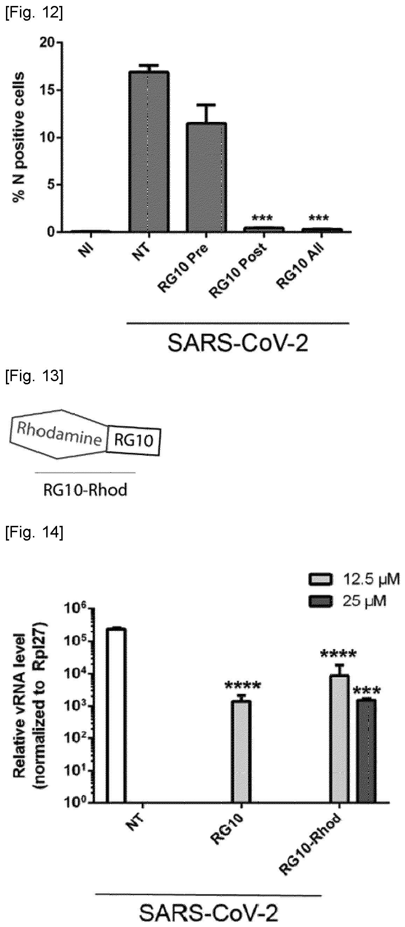

To discriminate whether RG10 inhibits virus entry or post-entry events, the inventor performed time-of-addition experiments where the molecule was added for four hours and then washed-out (Pre), or conversely; no drug was added for the first 4 h to allow early virus entry steps to occur and only then RG10 was added to the cells (Post; **Figure 9**). The inventor found that RG10 had no antiviral activity when incubated only during virus entry while adding RG10 post-entry significantly decreased vRNA expression levels (**Figure 10**). In these conditions, comparing the amount of viral N protein and dsRNA levels by flow cytometry confirmed that RG10 exerts strong antiviral activity post entry, although some inhibition was also observed at the entry steps **(****Figures 11** **and** **12****).** These results indicate that RG10 inhibits CoV2 replication, while inhibition of virus entry is likely minimal.

### RG10 localizes to the endoplasmic reticulum

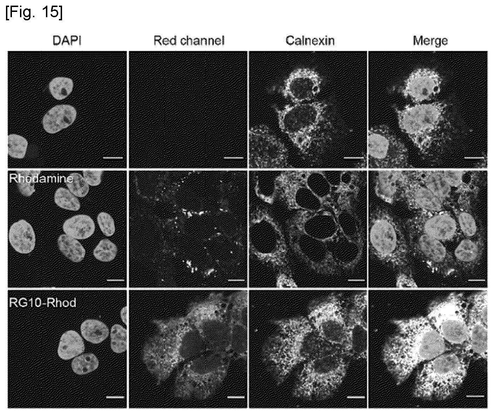

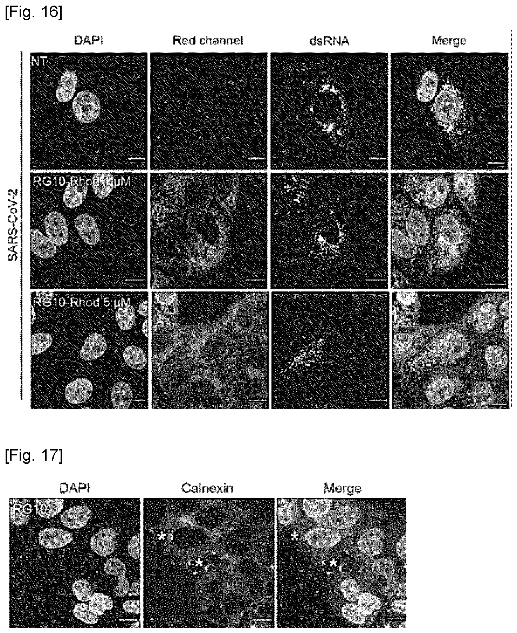

To determine where RG10 acts within the cell, the inventor generated a fluorescent version of this molecule which was coupled to a small, cell permeable, fluorescent Rhodamine dye (**Figure 13**). The RG10-rhodamine (RG10-Rhod) compound retained antiviral activity, although higher doses were required (**Figure 14**). By confocal microscopy, the inventor found that RG10-Rhod colocalizes with the endoplasmic reticulum (ER) resident protein Calnexin (**Figure 15**). In contrast, free Rhodamine clustered away from ER structures (**Figure 15**), suggesting that ER distribution of RG10-Rhod is conferred by its RG10 moiety. Using suboptimal RG10-Rhod concentrations allowing low levels of CoV2 replication, the inventor found that both RG10-Rhod and virus replication complexes localize to the ER, although in segregated regions (**Figure 16**). This suggests that RG10 does not act directly onto viral RNA replication and is rather a host-targeting agent (HTA).

### High concentration of RG10 induces sXPB-1-mediated ER stress

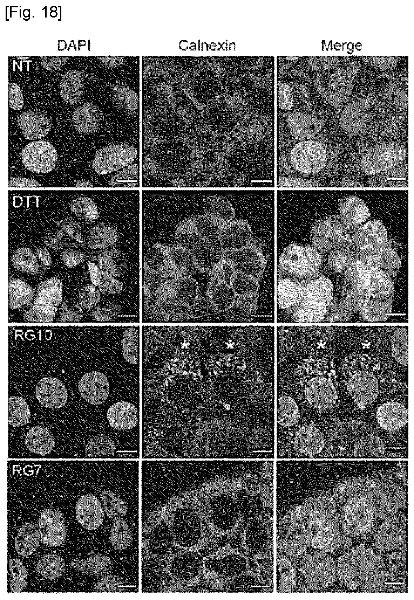

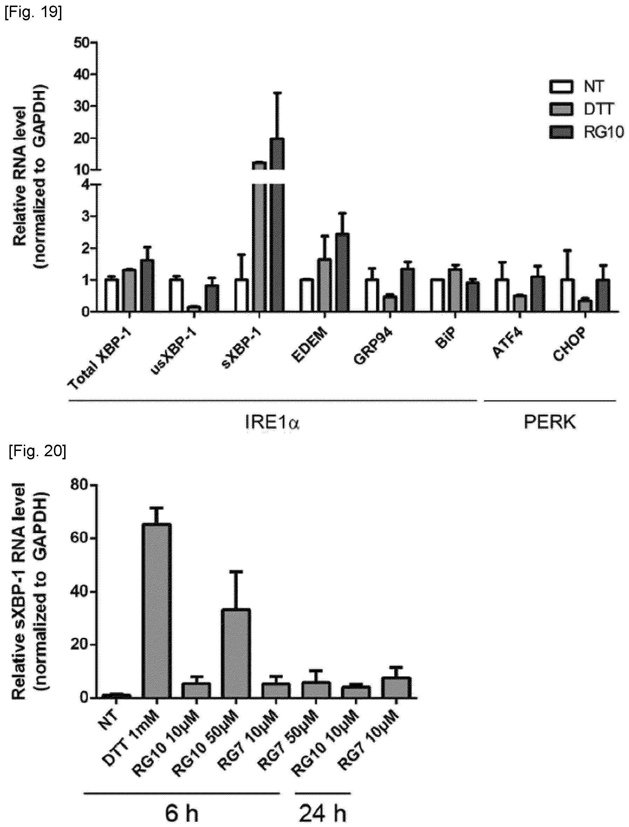

The inventor observed that RG10 perturbed ER morphology **(****Figure 17** and **Figure 18****)** and thus wondered whether RG10 could exert its antiviral activity through ER stress induction. Hence, the inventor measured the levels of RNAs of known ER stress mediators from the IRE1-α and PERK pathways and found that the spliced form of XBP-1 (sXBP-1) was enriched upon 50 µM RG10 and 1 mM DTT treatment (**Figure 19**). In contrast, 50 µM of the RG10 derivative compound RG7, which has no antiviral activity (**Figure 1**) did not significantly increase sXBP-1 mRNA levels (**Figure 20**). However, the increase of sXBP-1 RNA was lower at 10 µM and no difference was observed between RG10 and RG7 (**Figure 20**), and thus, ER stress induction cannot fully explain the antiviral activity of RG10.

### Generation of infectious CoV2 fluorescent particles

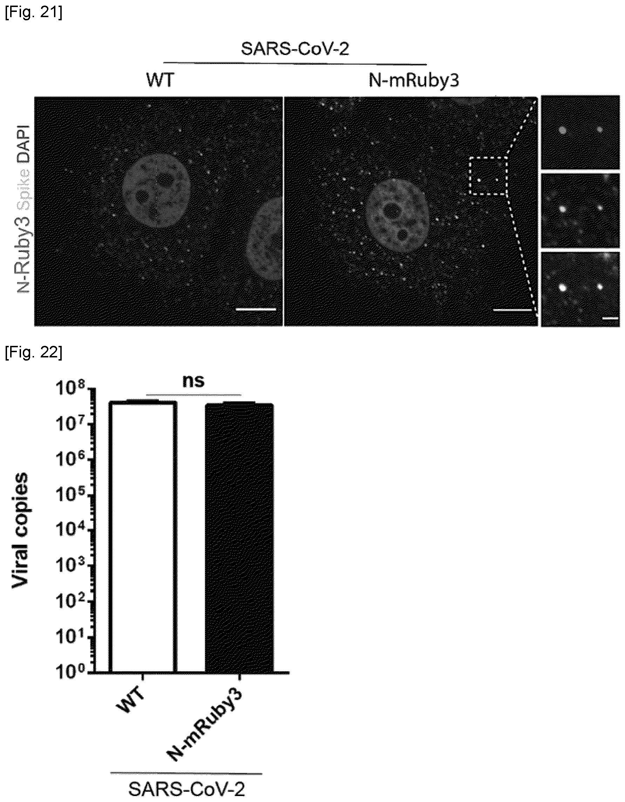

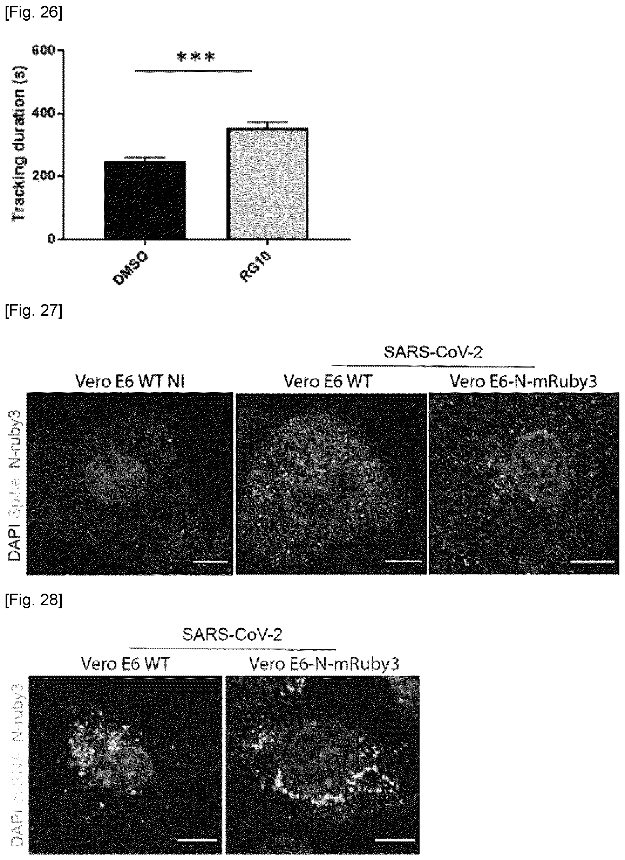

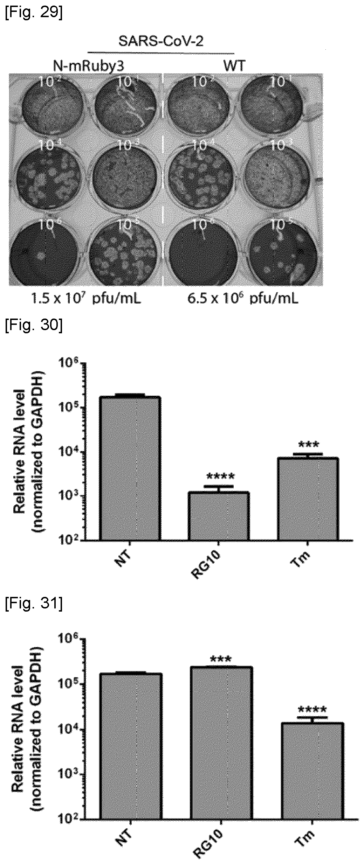

To gain further insights into the mode of action of RG10 antiviral activity, the inventor generated fluorescent infectious CoV2 using a trans-complementation approach. To this end, the inventor generated a Vero E6 cell line stably expressing the nucleocapsid (N) from CoV2 fused to the red fluorescent protein mRuby3. Wild type or Vero E6 N-mRuby3 cells were infected with wild type CoV2 virus **(****Figures 27 and 28****)** and the produced virions incorporated a subset of fluorescent N-mRuby3 proteins in trans (not encoded by the viral genome; **Figure 21****).** The N-mRuby3-containing viruses were as infectious as their non-fluorescent counterparts **(****Figure 22** and **Figure 29****),** indicating that they represent suitable tools to study CoV2 infection.

### RG10 perturbs ER-CoV2 dynamics

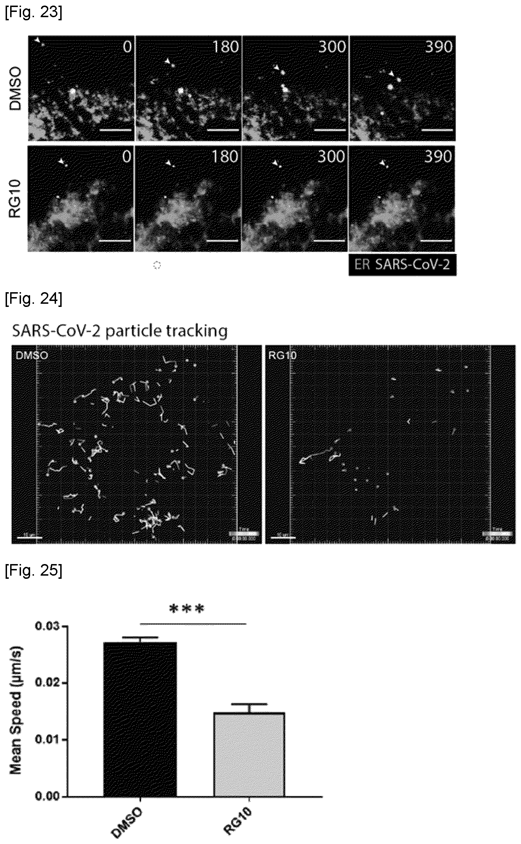

Live cell imaging of CoV2 N-mRuby3 viral particles infecting cells pre-treated with ER painter, a live fluorescent ER marker, allowed us to monitor infection dynamics in untreated and RG10-treated cells **(****Figure 23****).** Spatiotemporal tracking of viral particles showed that the virus was stalled upon RG10 treatment **(****Figure 24****).** Quantification of the mean particle speed confirmed that RG10 slowed down virus movement to the ER (**Figure 25**). Because the RG10-treated cells were more immobile, they were more reliably tracked, resulting in higher track duration measurement (**Figure 26**). These data indicate that RG10 perturbs intracellular virus dynamics and its transport toward the ER.

### RG10 exhibits coronavirus specific antiviral activity

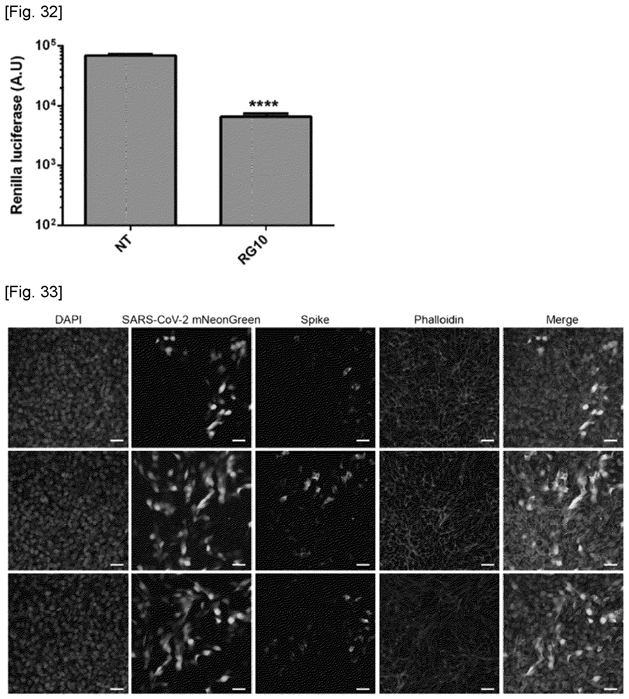

Next, the inventor aimed to determine whether RG10 had antiviral activity against another virus known to replicate at the ER. Therefore, the inventor compared in parallel the infection of Vero E6 cells by CoV2 or by Zika virus (ZIKV), another enveloped positive-strand RNA virus but from the Flaviviridae family. Interestingly, the inventor found that RG10 had no detectable effect on ZIKV, while in contrast, the ER stress inducer Tunicamycin was active against both viruses **(****Figures 30** and **31****).** To determine whether RG10 was specific to CoV2 or can act against other coronaviruses, the inventor tested its activity against the human coronavirus 229E (HCoV-229E) and observed significant antiviral activity, although potency was lower **(****Figure 32****).**

### RG10-mediated inhibition of CoV2 in human airway epithelia

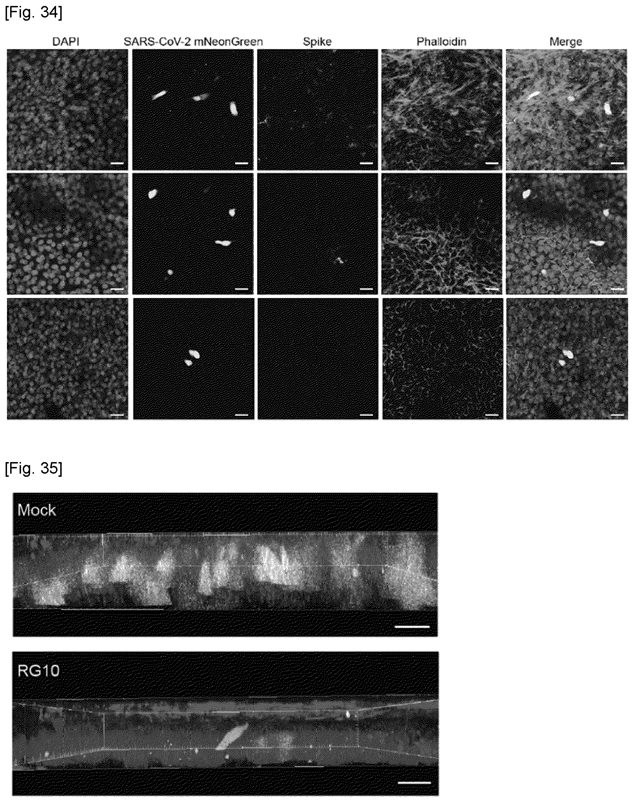

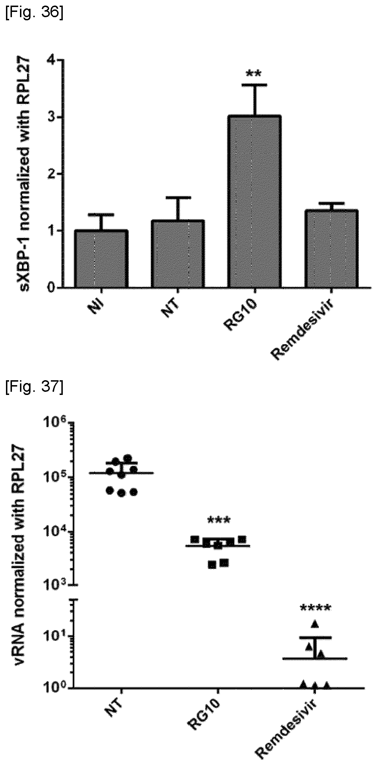

To move toward effective preclinical assays, the inventor tested the efficacy of RG10 in human airway epithelia (HAE), an ex vivo model of primary cells cultured in 2D on transwells, exhibiting expected cilia beating activity. In this model, the inventor found that RG10 decreases viral infection using a fluorescent mNeonGreen replication-competent virus, and that virus protein expression was also altered (**Figures 33** and **34**). Although HAE viability was similar, the actin network seemed to be rearranged at the apical side of RG10-treated HAE (**Figure 35**). Interestingly, sXBP-1 was upregulated upon RG10 treatment (**Figure 36**), indicating that RG10 mediates ER stress in this primary HAE model. By RT-qPCR, viral RNA was decreased by RG10, although the extent of the decrease was much lower than Remdesivir treatment (**Figure 37**).

### Identification of a more potent and stable RG10 derivative

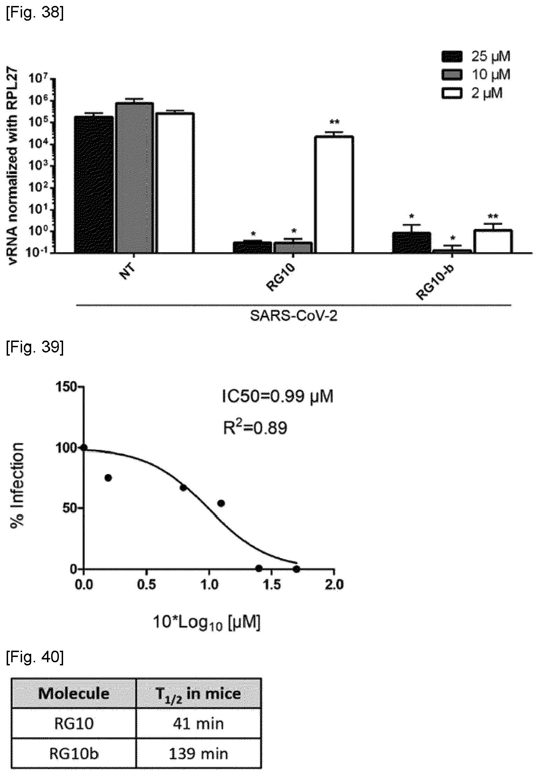

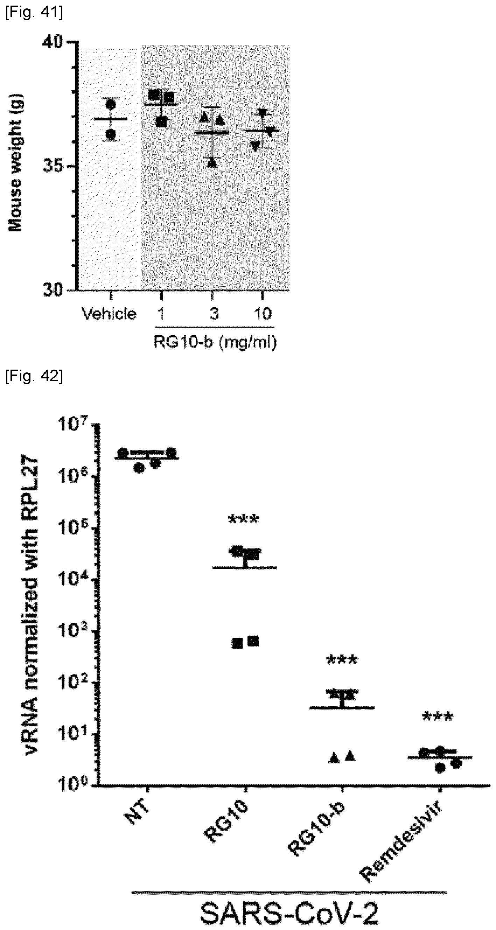

The low potency of RG10 in HAE could be attributed to its weak stability and therefore, the inventor designed a novel RG10 derivative, which the inventor called RG10b. This derivative harbors a modification shielding a molecular weakness present in RG10. RG10b was more potent than RG10 with an EC50 < 1 µM (**Figures 38** and **39**). To verify the stability of RG10 and RG10b in physiological conditions, the inventor performed pharmacokinetics analyses in mice and found that RG10b has a half-life of 2h19 in the blood, a value more than 3-fold higher than for RG10 (**Figure 40**). Innocuity assessment in mice showed that RG10b was well supported by mice at concentrations up to 10 mg/kg (higher concentrations were not tested; **Figure 41**). Treatment of HAE cells with 10 µM of the indicated compounds confirmed that RG10b was more potent than RG10, showing antiviral activity similar to Remdesivir in this ex vivo model (**Figure 42**).

### Discussion

In this work, the inventor has characterized a novel SARS-CoV-2 antiviral molecule and explored its mode of action. To this end, he generated novel tools to study the spatiotemporal dynamics of the virus at high resolution.

Numerous antiviral drugs have been considered for repurposing to treat COVID-19. Direct-acting antiviral agents (DAA) targeting the virus usually exhibit very good EC50 relative to their toxicity, but RNA viruses being prone to mutations (as highlighted by the emerging variants) suggest that this approach may not be sufficient by itself to counteract virus dissemination and may instead favor escape mutants in monotherapies. In contrast, HTAs that target cellular partners important for the virus life cycle is an approach of choice, that must be considered in parallel to DAA and vaccination. Here, the inventor choses a bottom-up approach, using a series of related proprietary molecules that had no known functions, and started to characterize it. During this study, he also generated new tools to perform live cell imaging of SARS-CoV-2 entry, offering the opportunity to learn more about the virus' life cycle, while tailoring an antiviral molecule with promising pharmacological properties.

Approved HTAs often have broad-spectrum antiviral activity due to the fact that viruses may exploit similar pathways. For instance, (hydroxy)chloroquine, ribavirin, and favipiravir are examples of broad-spectrum antivirals that are used in vitro at high concentrations to be effective. Although favipiravir shows very low potency in vitro. Hydroxychloroquine, which has an EC50 ≈ 2 µM in Vero E6 cells, does not decrease the viral loads of infected non-human primates. The heterogeneity between in vitro and in vivo potency highlights the complexity to develop HTAs. RG10 is likely an HTA as it does not localize at virus replication sites to exert its antiviral activity. The improved version of RG10, RG10b, shows EC50 below 1 µM, high stability in vivo (> 2 h), with no toxicity observed (**Figures 38** - **42**).

At this stage, the target of RG10 is unknown but the inventor identified that the molecule strongly affects virus and ER dynamics **(****Figures 21** - **26****),** suggesting that it perturbs players involved in intracellular trafficking routes. Concordantly, the inventor showed that the actin cytoskeleton was disorganized by RG10 in primary HAE cells **(****Figures 33** - **37****).** Preserved integrity of the actin cytoskeleton is required for SARS-CoV-2 infection. The FDA-approved drugs Sunitinib and BNTX inhibit SARS-CoV-2 entry and their mode of action was correlated to the disruption of actin dynamics. Moreover, SARS-CoV-2 increases filopodia number and length, while traveling along them, further indicating that a strong interplay exists between CoV2 and the actin network. RG10 has very low cytotoxicity compared to Latrunculin, jasplakinolide and cytocalasin D, three well-known direct-acting actin perturbators that inhibit CoV2 infection. It is therefore likely that RG10 subtly impacts the actin cytoskeleton in a direct or indirect manner that remains to be identified.

RG10 induces ER stress at high doses, but it was difficult to correlate this observation to the antiviral effect of RG10 because at lower doses, RG10 and RG7, two closely related molecules, similarly induced ER stress (**Figures 18** - **20**) even though RG7 showed no antiviral activity (**Figures 1** - **8**). Furthermore, RG10 was unable to inhibit ZIKV replication, which also occurs in the ER. The inventor used Tunicamycin as a potent ER stress inducer that inhibits infection by CoV2 and ZIKV, further suggesting that ER stress induction may not be sufficient for RG10 to exert an antiviral activity.
The inventor found that RG10 is targeted to the ER and impacts ER morphology. Constant reshaping of the ER structure is a requirement for maintaining its numerous functions, and ER membranes are often targeted by pathogens. For instance, influenza A virus was recently shown to impact mitochondrial morphodynamics and endoplasmic reticulum-mitochondria contact sites, and altering mitochondrial dynamics was thus proposed as a new antiviral strategy. Similarly, one can hypothesize that ER morphodynamics can represent an antiviral strategy to be further explored. In this regard, atlastins play important roles in ER dynamics and morphology and it was recently shown that atlastin-dependent misshaped ER leads to decreased ZIKV replication. To the inventor's knowledge however, the importance of atlastins during coronavirus infection has not been investigated so far. In any case, ER morphology seems to be important for CoV2 infection, and perturbation of ER dynamics by RG10 is likely to strongly contribute to its antiviral activity.

The study of CoV2 spatiotemporal dynamics, including virus entry, replication, assembly, and release requires new tools to quantitatively assess it. A comprehensive list of plasmids coding for the CoV2 viral proteins fused to fluorescent proteins has been generated, which proved very useful to evaluate the distribution and potential function of individual viral proteins. Moreover, fluorescent virus-like particles (VLP) expressing Spike from CoV2 were generated, although with modest specificity, but to date, fluorescent viral particles containing the full wild type genome of CoV2 has not been described. Using a trans-complementation approach, the inventor generated bright CoV2 particles which retain infectiveness comparable to their wildtype counterparts. Although the inventor exploited this reagent to study CoV2 arrival to ER membranes, fluorescent viral particles shall prove useful in a variety of assays aiming at a better understanding of CoV2 entry.

### Material & Methods

### Antibodies and reagents

Mouse anti-Spike and anti-GAPDH were purchased from Genetex and Rabbit anti-N from Sino Biological (Clinisciences). The mouse anti-dsRNA antibody (J2) was from Jena Bioscience and rabbit anti-Calnexin from Elabscience. NHS-Rhodamine and all secondary Alexa Fluor antibodies were purchased from Thermo Fisher Scientific. Phalloidin CF-633 was purchased from Biotium and the ER Staining Kit was from Abcam. RG10 and derivatives were synthesized by AGV Discovery.

### Cell lines and primary cells

Huh7.5.1 *(*Zhong J. et al. (2005) Proceedings of the National Academy of Sciences of the United States of America 102: 9294-9), Vero E6 (ECACC #85020206), Caco2, and HEK 293T cells were cultured in DMEM GlutaMAX (Thermo Fisher Scientific) supplemented with 10% fetal calf serum and penicillin-streptomycin (500 µg/ml; GIBCO). HAE cells (Epithelix) were cultured in Mucilair media at the basal side and maintained at air-liquid interface. Apical washes were performed each 2-3 days by temporarily adding Mucilair media for 30 minutes. All cell types were maintained at 37°C in a 5% CO₂ atmosphere.

### Plasmids

To generate a lentiviral vector containing the Nucleocapsid (N) of SARS-CoV-2 fused in C-terminus to the gene coding for the monomeric red fluorescent protein mRuby3 (Bryce T. Bajar et al, Sci Rep, 2016, 1-12), N was firstly amplified by PCR from the plasmid pLVX-EF1alpha-SARS-CoV-2-N-2xStrep-IRES-Puro (addgene#141391) using the sense primer 5' TTGCGGCCGCGCCACCATGAGCGATAACG-3' (**SEQ ID NO: 1**) (Notl site underlined) and the antisense primer 5'-CGCCTGAGTAGAATCGGCT-3' (**SEQ ID NO: 2**) (overlapped region underlined). The Ruby-3 fragment was amplified by PCR from a gblock (Integrated DNA-Technologies) using the sense primer 5'-AGCCGATTCTACTCAGGCGGGAGGTTCTGGTGGTTCTG-3' (**SEQ ID NO: 3**) (overlapped region underlined) and the antisense primer 5'-GAGGATCCTCACTTGTACAGCTCGTCCAT-3' (**SEQ ID** NO: **4**) (BamHI site underlined). The individual N and Ruby3 fragments were linked by overlapping PCR of the N-CoV2 using primers 5'- TTGCGGCCGCGCCAC-3' (**SEQ ID NO: 5**) and 5'-GAGGATCCTCACTTGTACAGCTCGT-3' (**SEQ ID NO: 6**). The resulting PCR product was ligated into the digested Notl and BamHl lentiviral plasmid pHAGE IRES puro. The resulting plasmid pHAGE N-Ruby3 IRES puro is available on Addgene (#170466). In parallel, a pHAGE N-mNeonGreen IRES puro was also generated (Addgene #170467).

### Generation of stable cell line

HEK 293T cells were transfected with pxPAX2, VSV-G and pHAGE N-Ruby3 IRES puro using JetPrime (Poluplus Transfection) and washed 6 h post-transfection. The supernatant was harvested 2 days later and cleared by centrifugation at 2000 × g for 5 min at 4°C. The lentivirus-containing supernatant was added to Vero E6 cells (ECACC #85020206) for 48 h. Selection of the positive cells was performed using 4 µg/ml puromycin and viable cells were expanded for flow cytometry cell sorting using a FACSMelody (BD Biosciences) equipped with a 561 nm excitation laser and 613/18 filter. Upon sorting, the cells were tested negative for mycoplasma. Vials of the Vero E6 N-Ruby3 stable cell line were conserved in liquid nitrogen in 10% DMSO.

### Virus production and titration

The SARS-CoV-2 strain BetaCoV/France/IDF0372/2020 was supplied by the National Reference Centre for Respiratory Viruses hosted by Institut Pasteur (Paris, France) and headed by Dr. Sylvie van der Werf. The strain was amplified through the infection of Vero E6 cells at MOI 0.0001 in DMEM GlutaMAX (Gibco) supplemented with 2% fetal bovine serum (FBS; Dominique Dutscher) and 1X penicillin-streptomycin (GIBCO). At 3 days post infection (dpi), the supernatant was harvested and cleared by centrifugation at 2000 × g for 5 min at 4°C. The cleared virus-containing supernatant was frozen in 1 ml aliquots at -80°C. For each virus production, a vial was thawed for titration by plaque assay in Vero E6 cells to estimate plaque-forming units per mL of virus (PFU/mL) as described in Gaudin & Barteneva (Gaudin & Barteneva, 2015). Viral titers ranged between 3 10⁶ and 2 10⁷ pfu/ml.

HCoV-229E-Renilla was a gift from Volker Thiel (van den Worm et al., 2012) and was amplified for 5-7 days at 33°C in Huh7.5.1 cells in 5% FCS-containing DMEM. Viral stocks were harvested when cells showed > 50% CPEs. Virus stock was titrated through TCID₅₀ in Huh7.5.1 cells used for their amplification and titer was 10⁸ TCID₅₀/mL. Infections of 50 000 cells were performed at MOI 2 for HCoV-229E-Renilla (as measured on Huh7.5.1 cells) and infection efficiency was analyzed one day later by measuring Renilla activity (Renilla Luciferase Assay System, Promega).

The ZIKV strain was obtained through BEI Resources (NIAID NR-50183, strain FLR) isolated in December 2015 from infected patient blood from Colombia. ZIKV was amplified in Vero cells for 72 h in DMEM GlutaMAX (Gibco) supplemented with 2% fetal bovine serum (FBS; Dominique Dutscher), 10 mM HEPES (Thermo Fisher Scientific) and 1X penicillin-streptomycin (Gibco). The supernatant was harvested and clarified by centrifugation at 2000 g RT for 10 min. The virus was stored at -80°C. The virus titer was obtained by using standard plaque assay on Vero cells to measure plaque-forming unit per mL of virus (PFU/mL). Viral titer was 3.8 ×10⁶ pfu/mL.

### RT-qPCR

Cells were lysed and RNA immediately extracted using the Luna Cell Ready Lysis Module according to the manufacturer's protocol (New England Biolabs). RT-qPCR on extracted RNA was performed using the Luna One-Step RT-qPCR Kit (New England Biolabs). The samples were analyzed using a Lightcycler 480 instrument (Roche) using the following thermal cycle: Reverse transcription step at 55 °C for 15 min, initial denaturation at 95 °C for 2 min. Then, 40 cycles were programmed consisting of 15 s of denaturation at 95 °C followed by 30 s of annealing/extension at 55 °. Fluorescent data collection was performed at the end of each cycle. SARS-CoV-2 viral RNA was detected using the N1 probe that targets specifically the N protein. For normalization, primers targeting Rpl27 or GAPDH were utilized.

**[Table 1]**

| ER stress Primers | Forward | Reverse |
|---|---|---|
| sXBP-1 | 5' -CTGAGTCCGAATCAGGTGCAG- 3' (**SEQ ID NO: 7**) | 5' -ATCCATGGGGAGATGTTCTGG- 3' (**SEQ ID NO: 8**) |
| usXBP-1 | 5' -CAGCACTCAGACTACGTGCA- 3' (**SEQ ID NO: 9**) | 5' -ATCCATGGGGAGATGTTCTGG- 3' (**SEQ ID NO: 10**) |
| tXBP-1 | 5' -TGGCCGGGTCTGCTGAGTCCG- 3' (**SEQ ID NO: 11**) | 5' -ATCCATGGGGAGATGTTCTGG- 3' (**SEQ ID NO: 12**) |
| ATF4 | 5' -GTTCTCCAGCGACAAGGCTA- 3' (**SEQ ID NO: 13**) | 5' -ATCCTGCTTGCTGTTGTTGG- 3' (**SEQ ID NO: 14**) |
| CHOP | 5' -AGAACCAGGAAACGGAAACAGA-3' (**SEQ ID NO: 15**) | 5' -TCTCCTTCATGCGCTGCTTT- 3' (**SEQ ID NO: 16**) |
| BiP | 5' - TGTTCAACCAATTATCAGCAAACTC - 3' (**SEQ ID NO: 17**) | 5' -TTCTGCTGTATCCTCTTCACCAGT- 3' (**SEQ ID NO: 18**) |
| GRP94 | 5' -GAAACGGATGCCTGGTGG- 3' (**SEQ ID NO: 19**) | 5' -GCCCCTTCTTCCTGGGTC- 3' (**SEQ ID NO: 20**) |
| EDEM | 5' -CAAGTGTGGGTACGCCACG- 3' (**SEQ ID NO: 21**) | 5' -AAAGAAGCTCTCCATCCGGTC- 3' (**SEQ ID NO: 22**) |

### Immunofluorescence

Cells grown on #1.5 glass coverslips were fixed with 4 % PFA for 15 min. Cells were then permeabilized with 0.1% Triton X100 and saturated with 0.2% BSA. Then, primary antibody incubation was performed for 1 h. After a series of PBS washes, secondary antibodies were added for 30 min. DAPI staining was performed for 10 min to obtain nuclear staining. Coverslips were mounted on glass slides using Mowiol mounting media. Images were acquired using a Leica SP5 confocal microscope.

### Live cell imaging

Image acquisition was performed on an AxioObserver.Z1 inverted microscope (Zeiss) mounted with a CSU-X1 spinning disk head (Yokogawa), a back-illuminated EMCCD camera (Evolve, Photometrics) and a X100, 1.4 NA oil objective (Zeiss) controlled by MetaMoprh. The microscope chamber was heated at 37°C and the automated multiposition stage maintained in 95% humidity at 37°C and 5% CO2. Images were processed using FiJi (ImageJ software version 1.51h) and quantitative analyses were performed using Bitplane Imaris x64 version 9.2. Analyses of the movies consisted on the segmentation of the single viral particles based on intensity and size. Then, tracking was performed for each individual particle and mean speed and track duration were extracted.

### Statistical analysis

Statistical analyses of the data were performed using two-tailed unequal variance Student t-tests (ns p>0.05, * p<0.05, ** p<0.01, *** p<0.001) unless mentioned otherwise. The mean and standard deviation (SD) of the mean were plotted using GraphPad Prism version 7.04. Number of independent experiments (n) is indicated in the figure legends.

### Example 3 ― Evaluation of other compounds according to the invention.

Further to the good results obtained with the compounds RG10 and RG10b (i.e. RG27), the inventor designed and synthesized other RG10 derivatives, namely RG38 of formula: 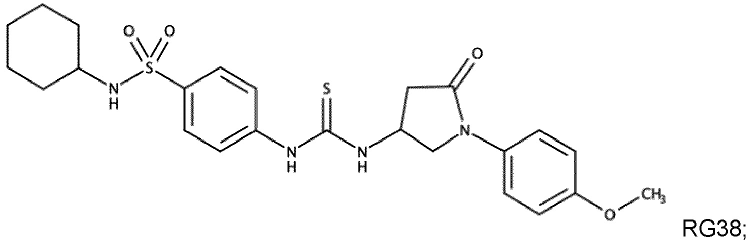 and RG41 of formula:

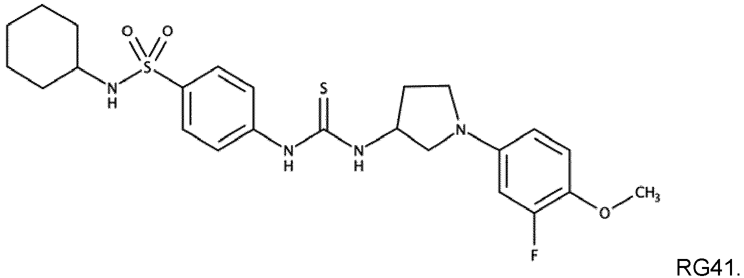

Antiviral activity was assessed as explained in Example 2. Vero E6 cells were treated with the indicated RG derivatives or Remdesivir at indicated concentrations and subsequently infected with SARS-CoV-2 at MOI 0.01 for 48 h in the presence of the compounds.

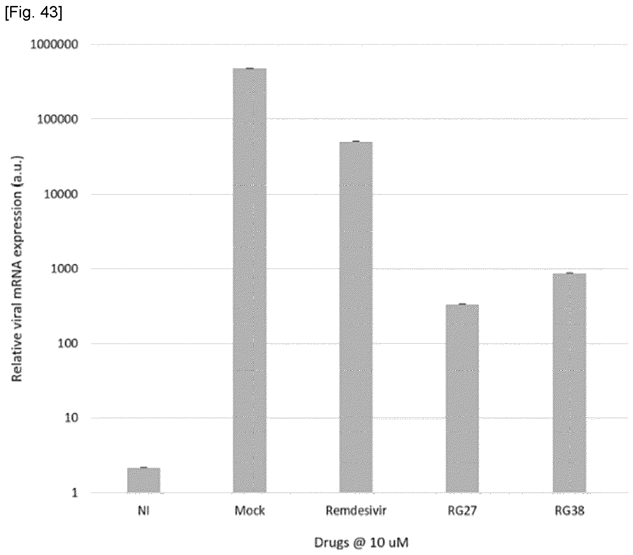

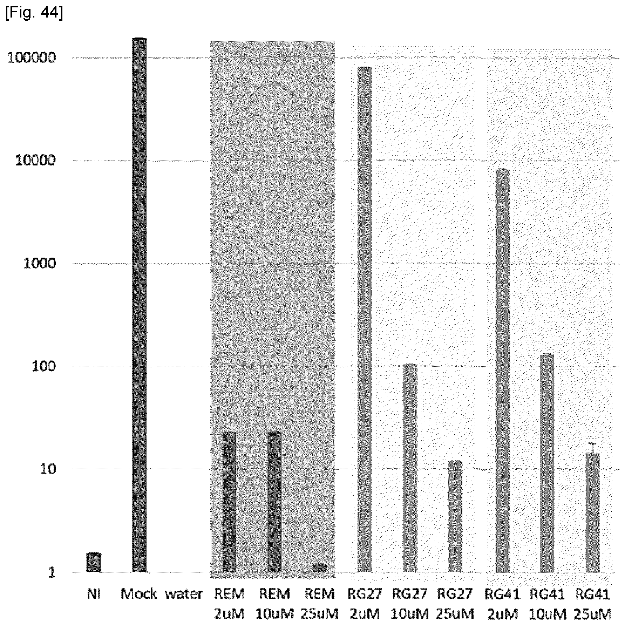

Results are shown in **Figures 43** **and** **44****.**

## Claims

1. A compound of formula I: 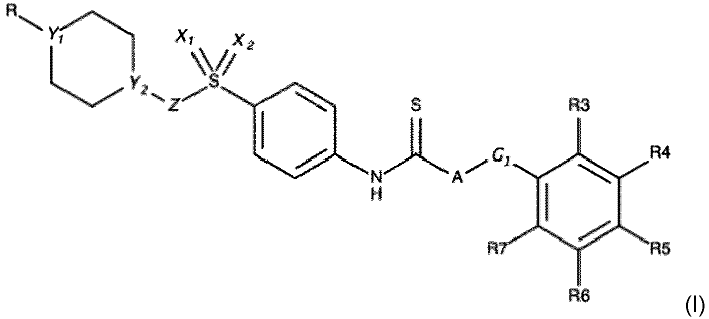 wherein
- R is H or a C1-C4 alkyl;
- A is NH or CH₂;
- Y₁ is CH or N;
- Y₂ is O, N or CH;
- Z is NH or O;
- G₁ is one of the following: 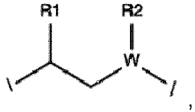 wherein W is CH, N or O, and wherein R1 and R2, are independently from each other, H, a C1-C2 alkyl, linear or branched, saturated of not, and wherein R2 is missing when W is O; 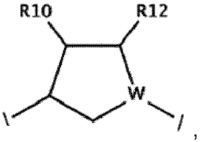 wherein W is C or N, R10 is a group chosen from an hydroxyl, an methoxy, and ethoxy, a C1-C2 alkyl, an halogen or a -CF₃ group, and R12 is H or =O or OH; 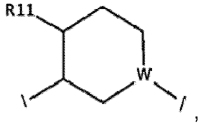 wherein W is C or N, and R11 is an amine or a C1-C2 alkyl,
- X₁ and X₂ are, independently from each other, NH, N-R9, wherein R9 is a C1-C3 alkyl, linear or branched, a -CN group or a -CF3 group;
- R3 to R7 are, independently from each other, H or a functional group from the list consisting of fluoro, chloro, bromo, nitro, cyano, amino, amino alkyl such as amino methyl and amino ethyl, methyl, hydroxylmethyl, trifluoromethyl, methoxy, trifluoromethyloxy, ethyl, trifluoroethyl, ethoxy and trifluoroethyloxy;
or a salt or a solvate thereof.

2. The compound according to claim 1, having one of the following formulas: 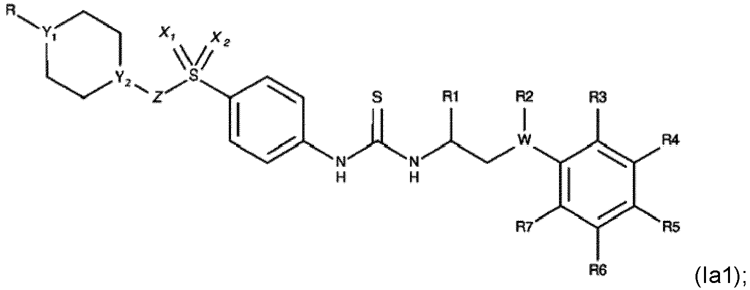 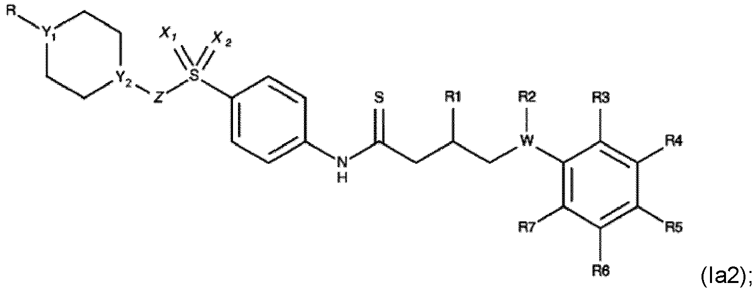 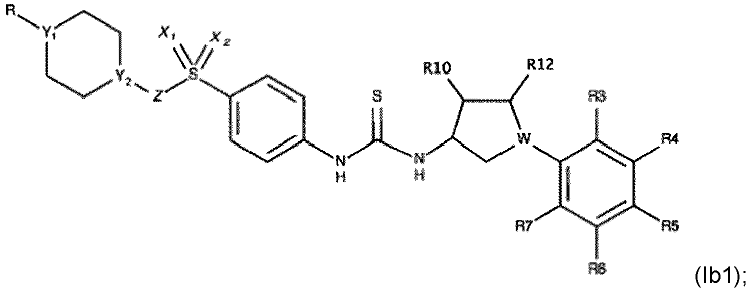 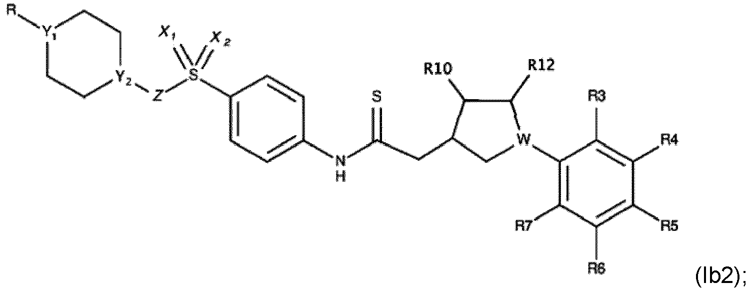 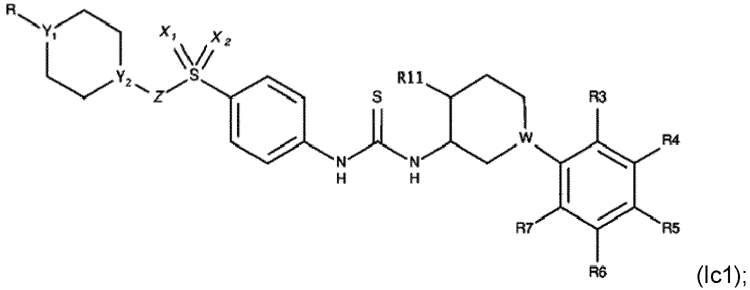 and 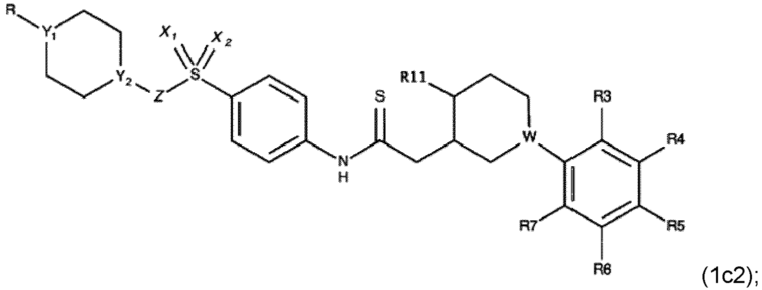 wherein Y1, Y2, W, Z, X1, X2, R and R1 to R7 are as defined in claim 1.

3. The compound according to claim 1 or 2, having one of the following formulas: 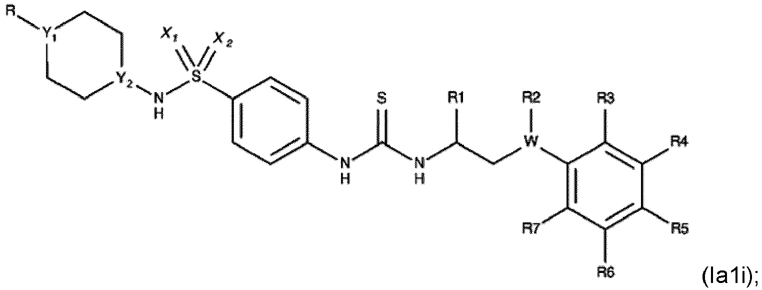 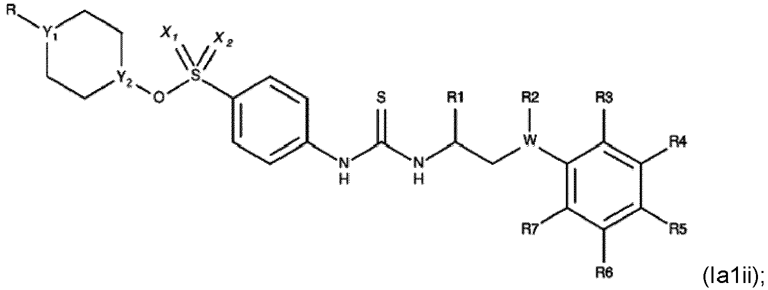 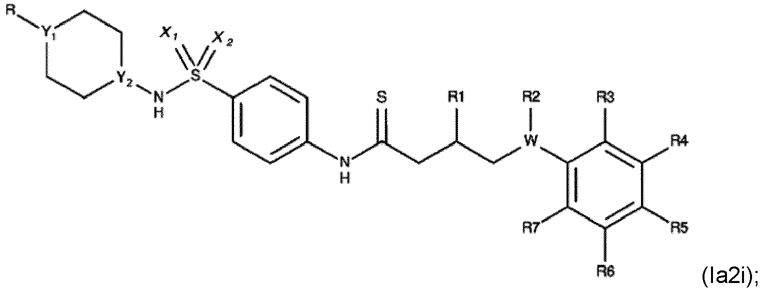 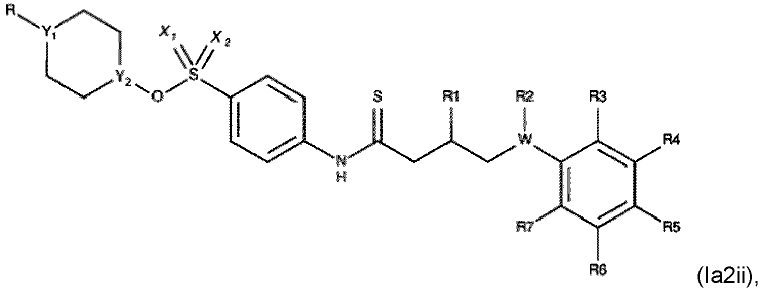 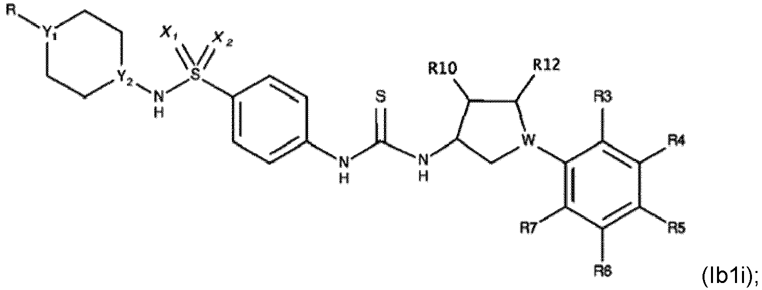 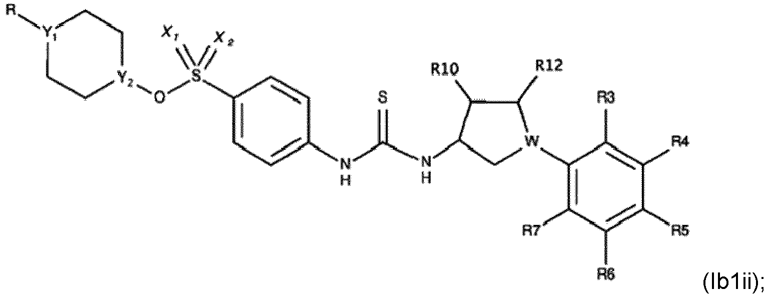 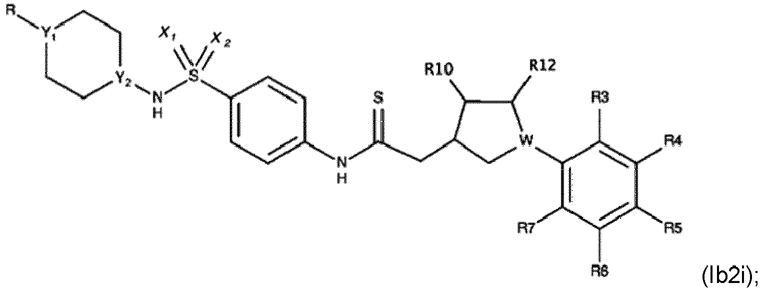 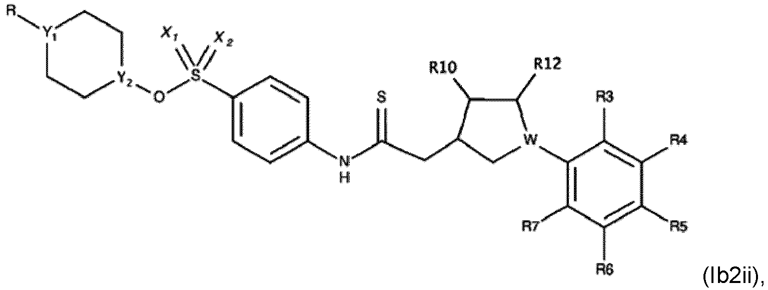 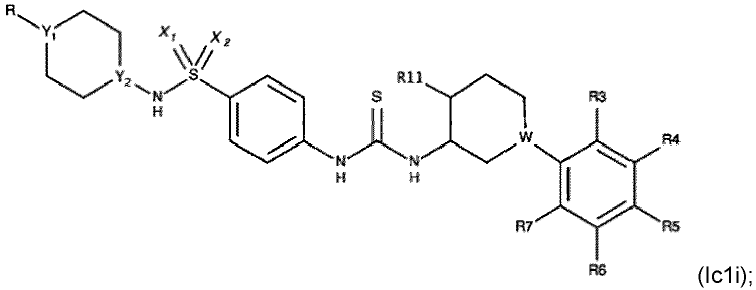 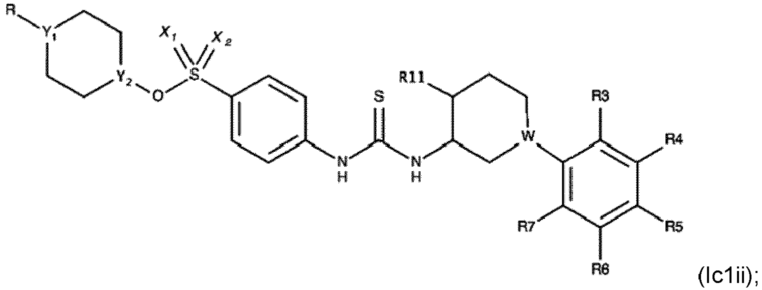 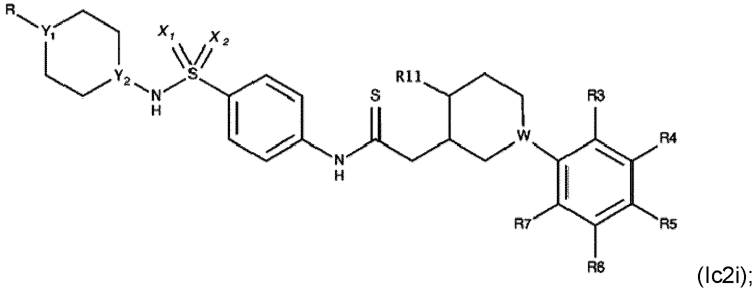 and 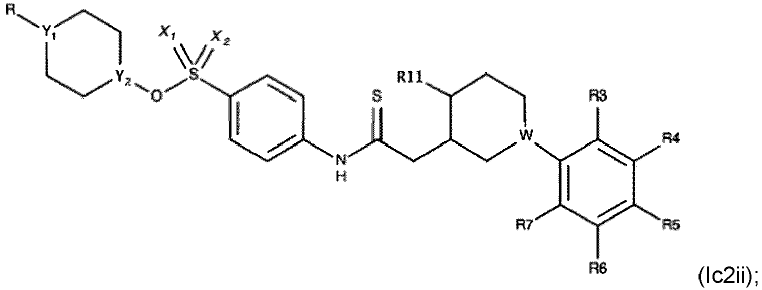 wherein Y1, Y2, W, X1, X2, R and R1 to R7 are as defined in claim 1.

4. The compound according to anyone of claims 1 to 3, having one of the following formulas: 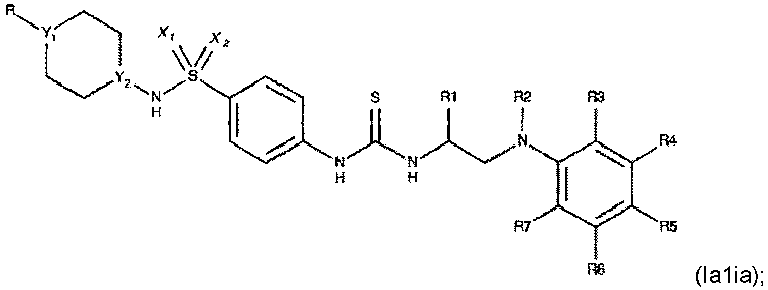 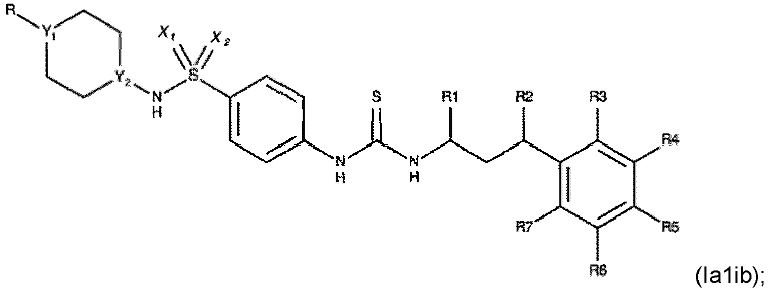 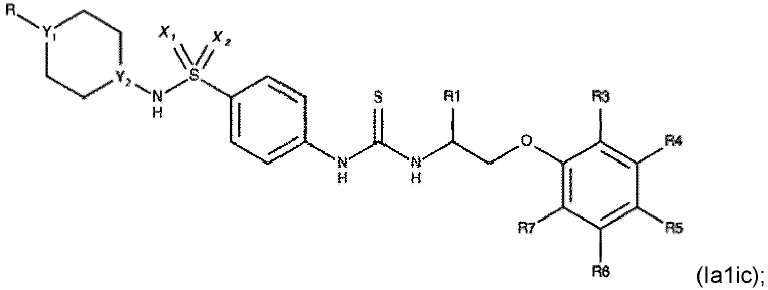 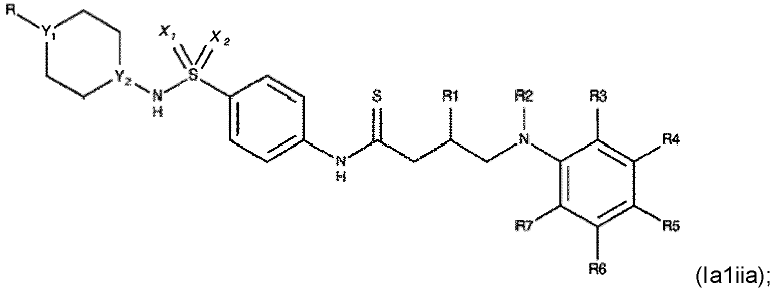 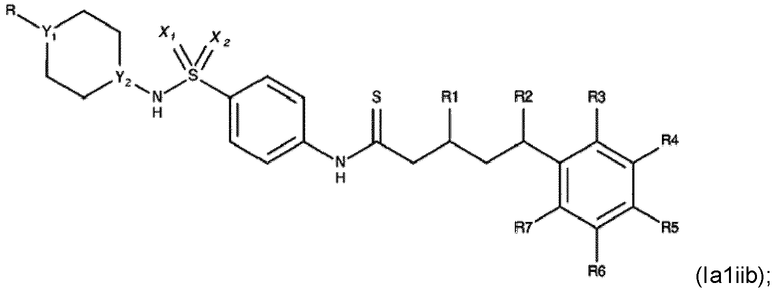 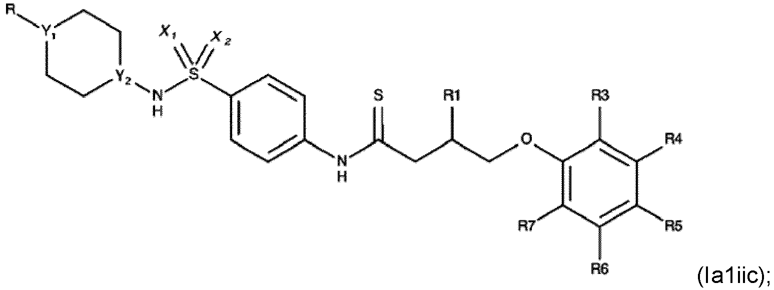 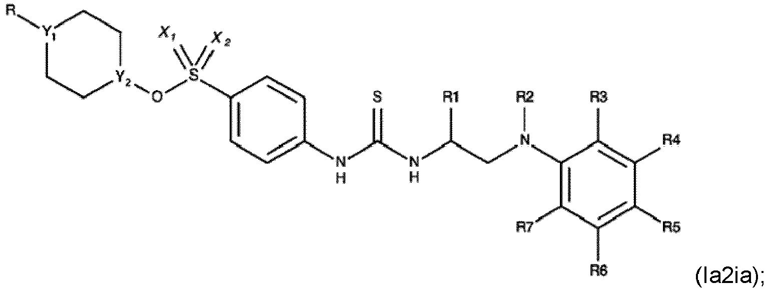 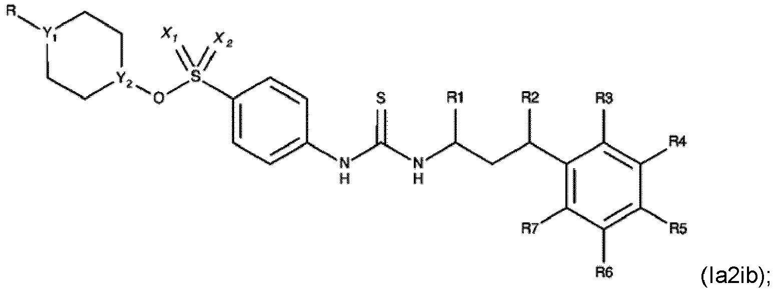 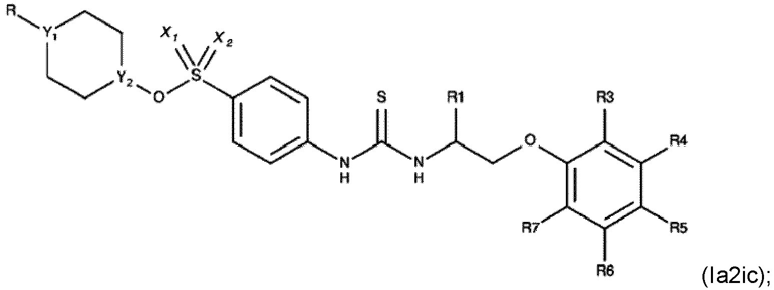 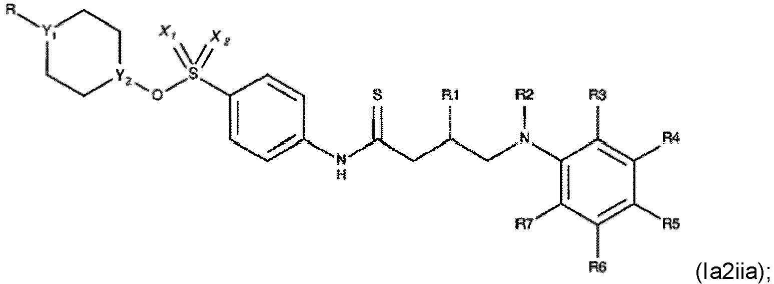 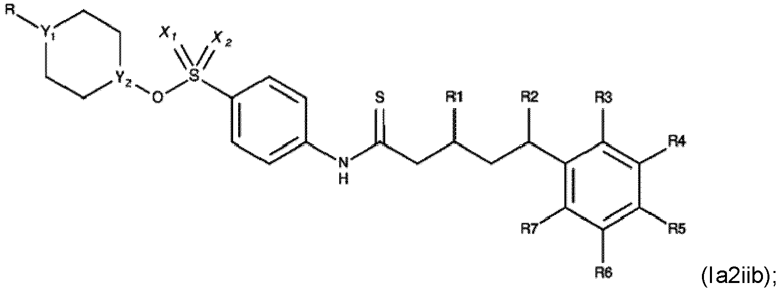 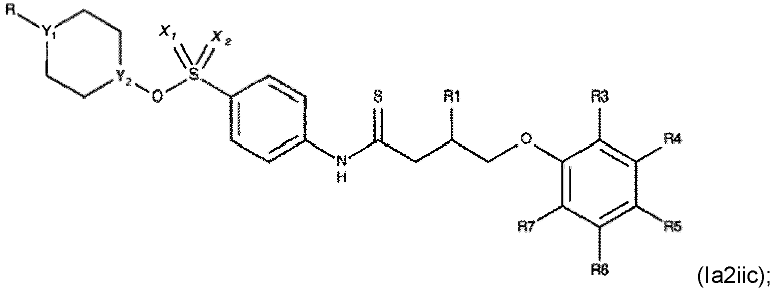 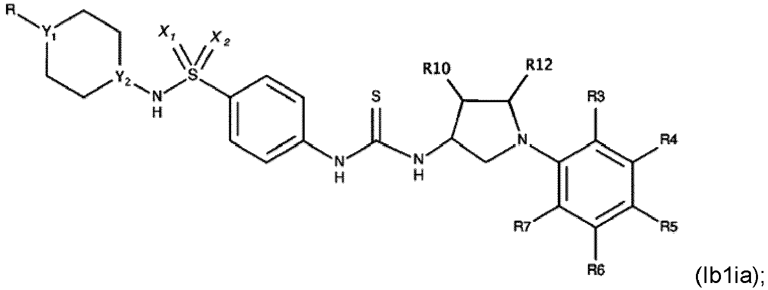 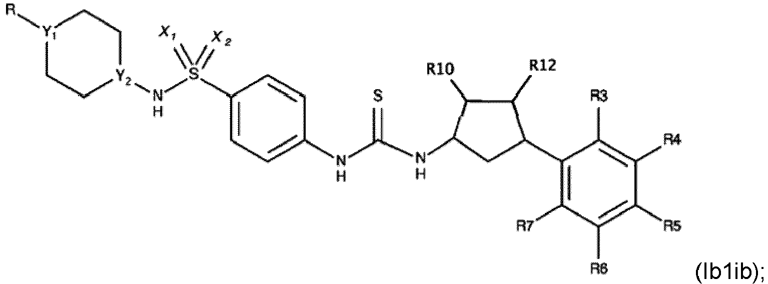 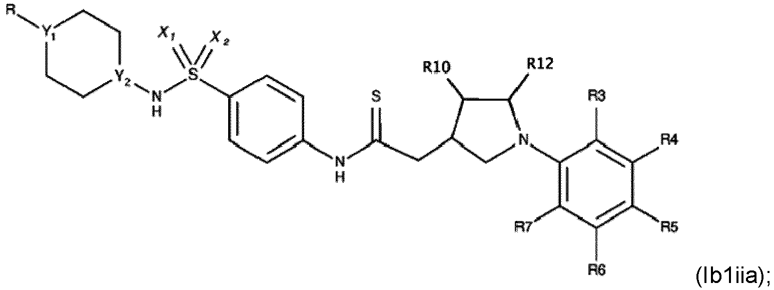 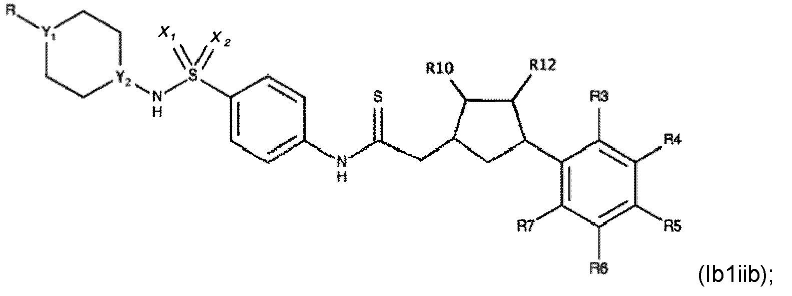 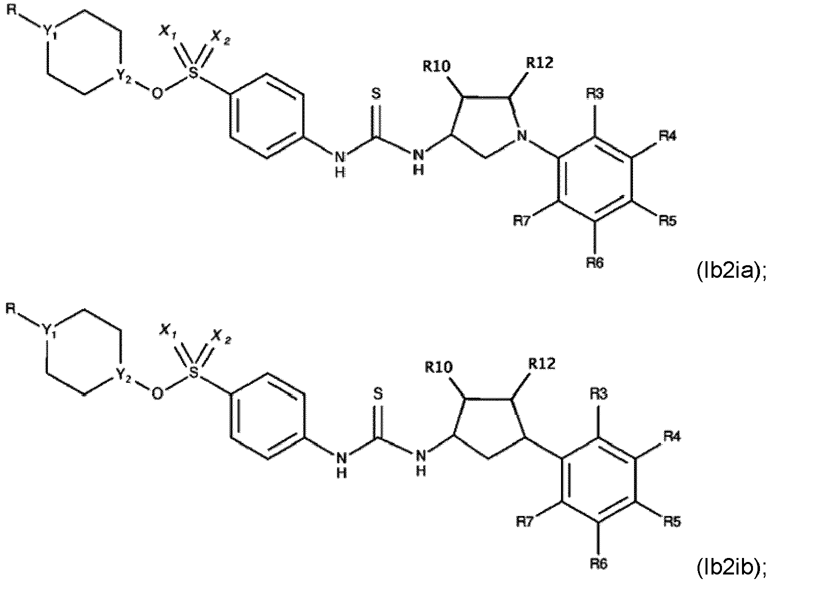 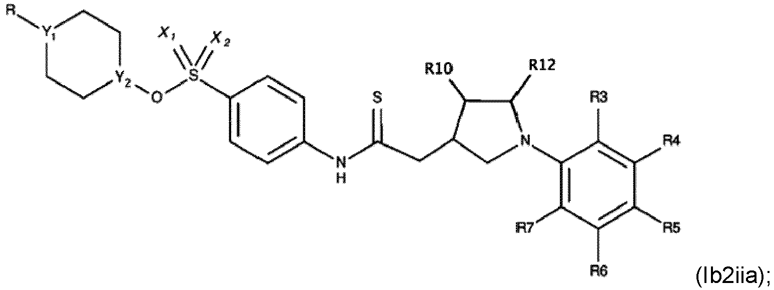 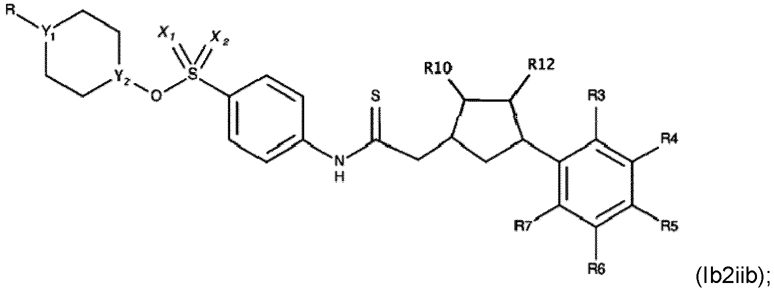 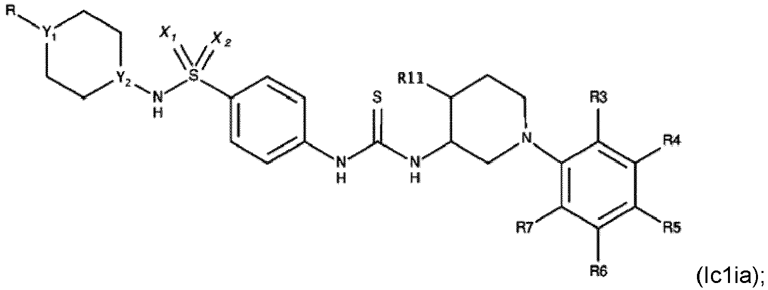 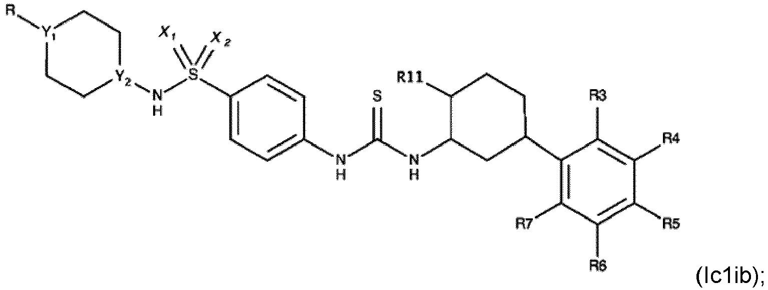 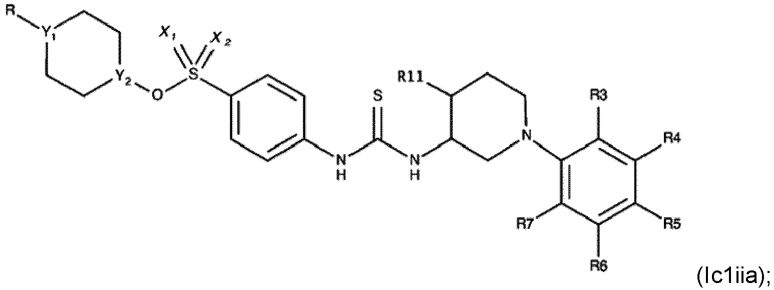 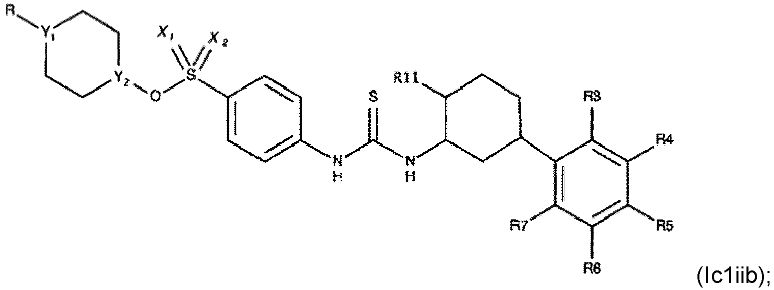 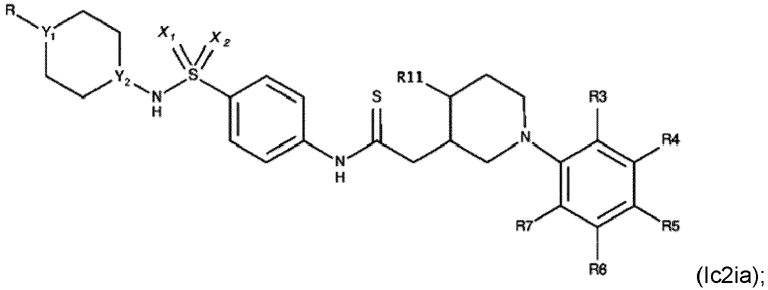 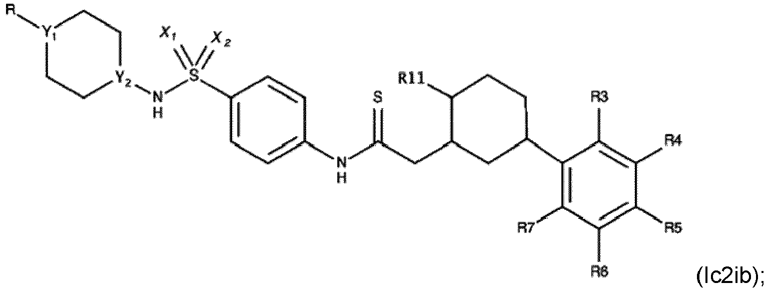 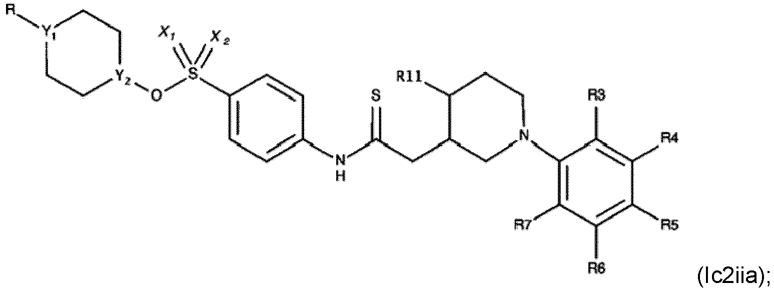 and 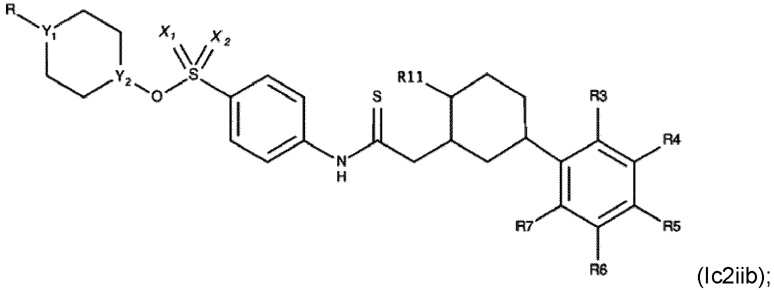 wherein Y1, Y2, X1 and X2, R and R1 to R7 are as defined in claim 1.

5. The compound according to anyone of claims 1 to 4, wherein X1 and X2 are O.

6. The compound according to anyone of claims 1 to 5, wherein R3, R4, R6 and R7 are H.

7. The compound according to anyone of claims 1 to 6, wherein R5 is -O-CH₃.

8. The compound according to anyone of claims 1 to 7, the compound having one of the following formulas: 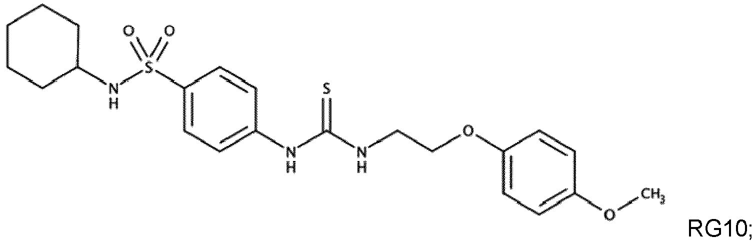 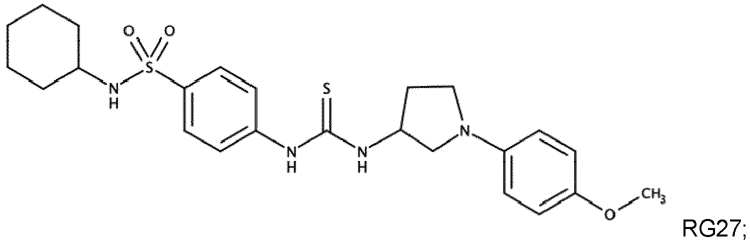 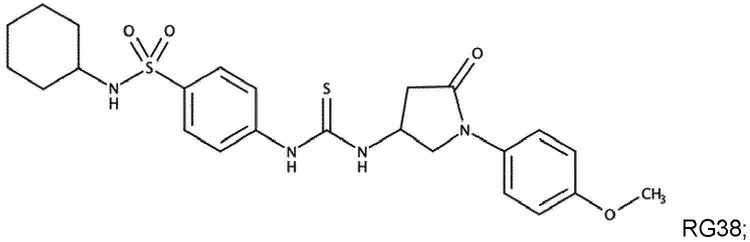 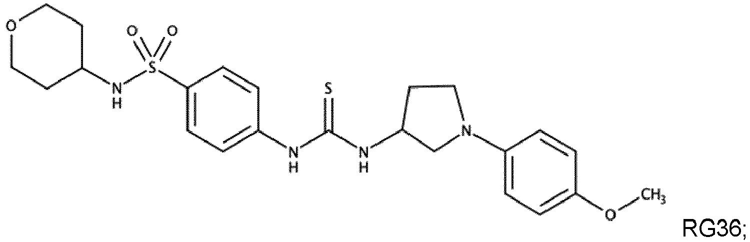 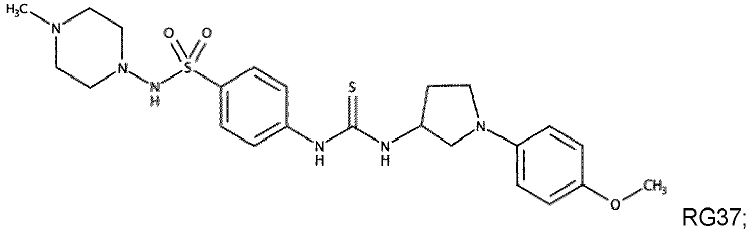 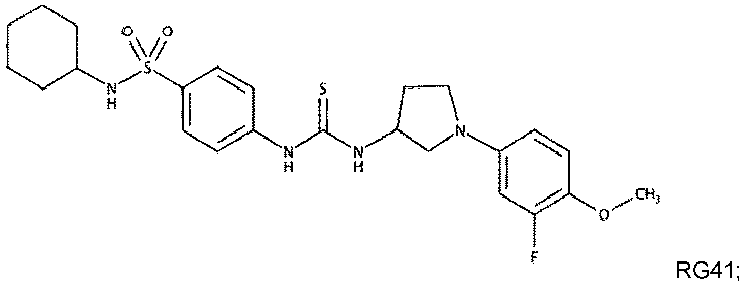 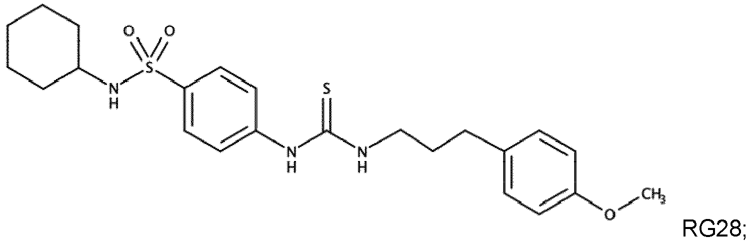 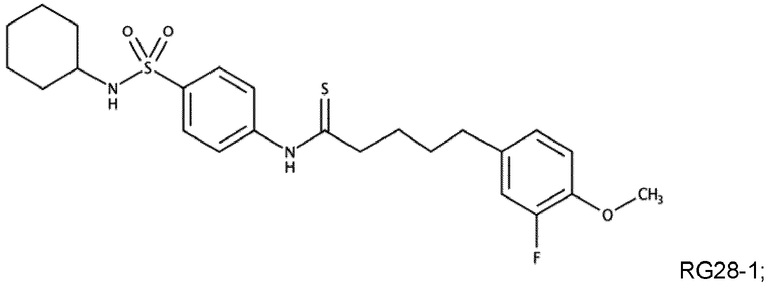 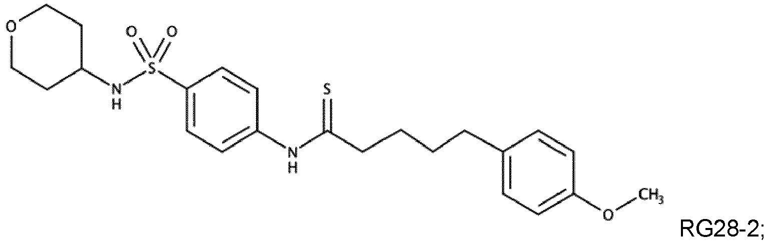 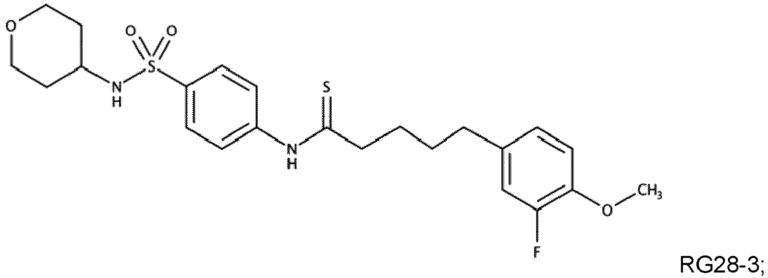 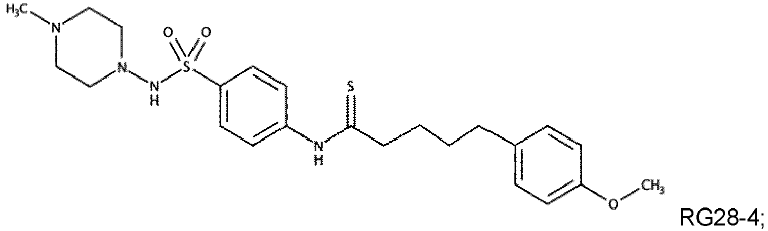 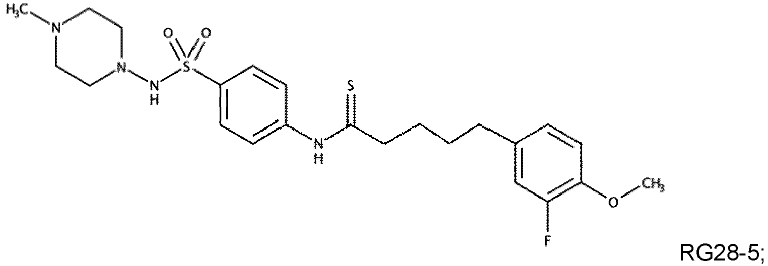 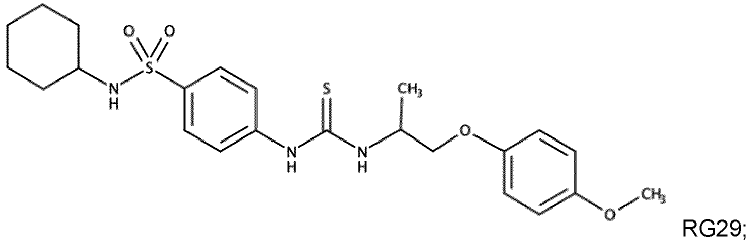 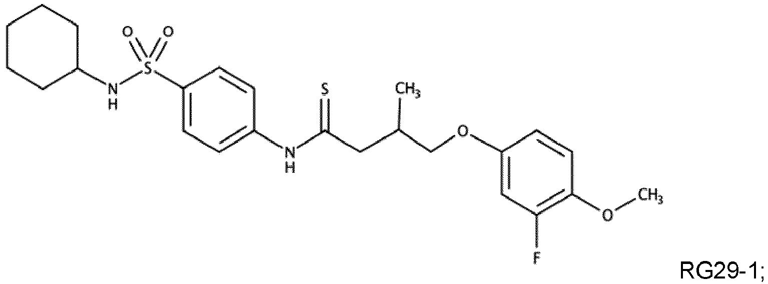 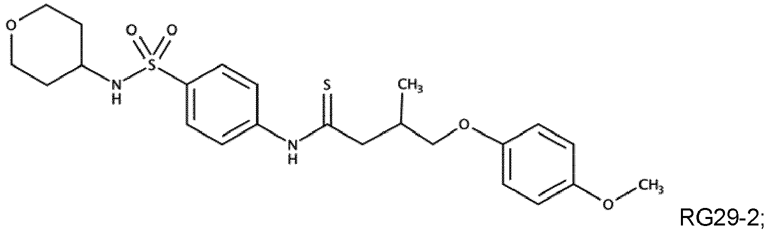 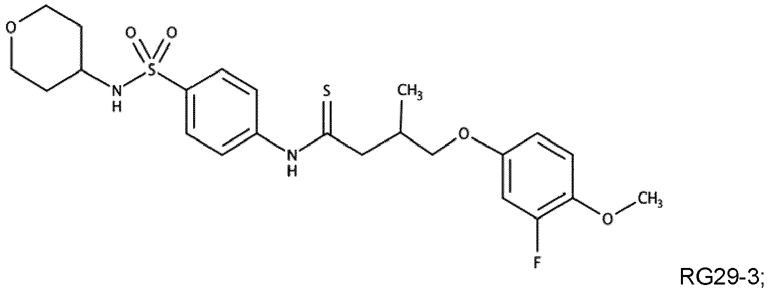 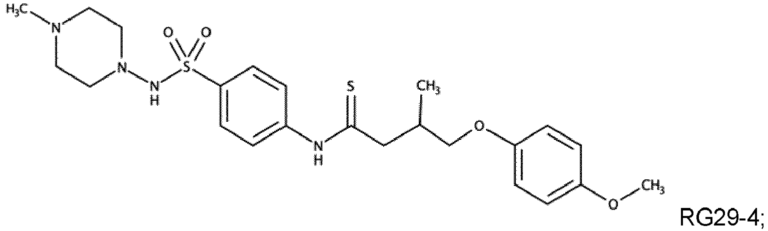 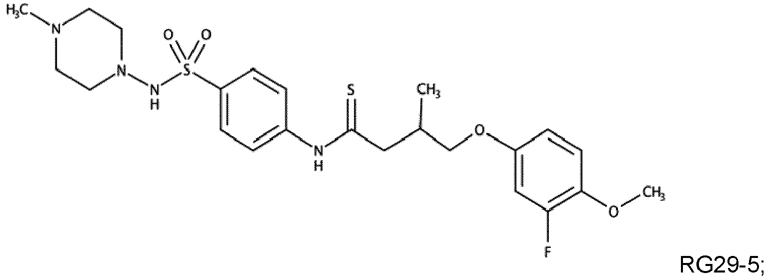 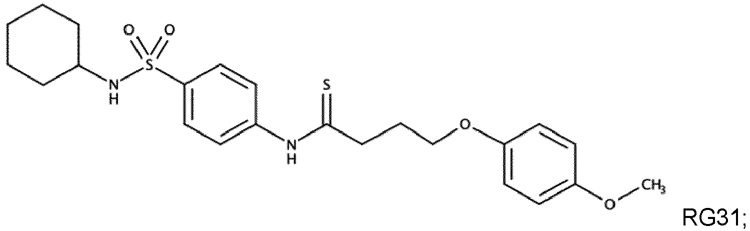 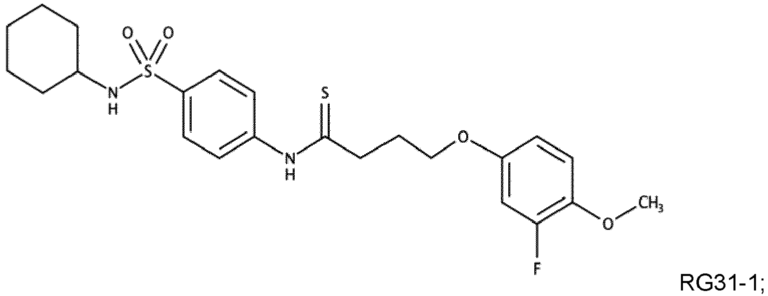 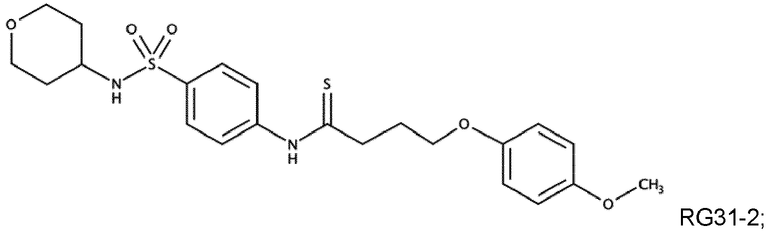 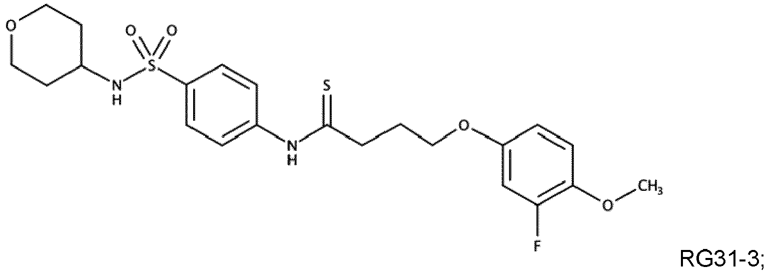 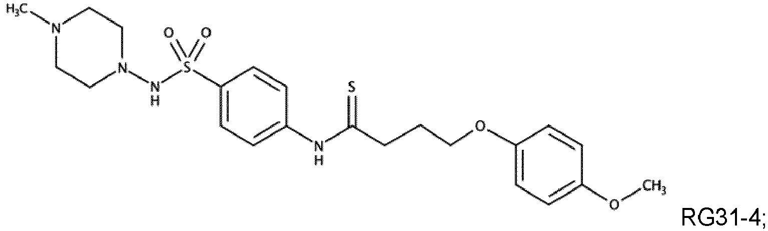 and

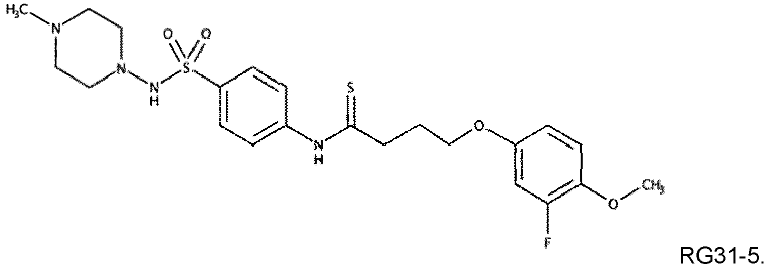

9. A pharmaceutical composition comprising, as active ingredient, the compound as defined in anyone of claims 1 to 8, in association with a pharmaceutically acceptable carrier.

10. A compound according to anyone of claims 1 to 8, for its use as a medicament.

11. A compound according to anyone of claims 1 to 8, for its use for the treatment of viral infections or a pathology linked to the viral infection.

12. The compound for its use according to claim 11, wherein the viral infection is a coronavirus infection.

13. The compound according for its use according to claim 11 or 12, wherein the viral infection is a Sars-CoV-2 infection, and the pathology liked to the viral infection is COVID-19.

14. A kit comprising
- a compound according to anyone of claims 1 to 8; and
- a vaccine against a viral infection, or an antiviral compound.

15. The kit according to claim 14, wherein the antiviral compound is one of the followings: remdesivir, azithromycin, hydroxychloroquine, chloroquine tocilizumab and sarilumab.
